(19)

**Europäisches Patentamt**
**European Patent Office**
**Office européen des brevets**

(11)　**EP 4 782 003 A1**

(12)　**EUROPEAN PATENT APPLICATION**
published in accordance with Art. 153(4) EPC

(43) Date of publication:
**29.07.2026　Bulletin 2026/31**

(21) Application number: **24868394.8**

(22) Date of filing: **08.02.2024**

(51) International Patent Classification (IPC):
*A61K 38/45* (2006.01)　　*A61P 25/16* (2006.01)
*A61K 38/17* (2006.01)　　*A61P 25/28* (2006.01)
*A61K 38/50* (2006.01)　　*A61P 25/00* (2006.01)

(52) Cooperative Patent Classification (CPC):
**A61K 38/17; A61K 38/45; A61K 38/50;**
**A61P 25/00; A61P 25/16; A61P 25/28**

(86) International application number:
**PCT/KR2024/001916**

(87) International publication number:
**WO 2025/063407 (27.03.2025 Gazette 2025/13)**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(30) Priority: **22.09.2023　KR 20230127277**

(71) Applicants:
 • **Standup Therapeutics Inc.**
　**Seoul 04418 (KR)**
 • **Yoo, Jun Sang**
　**Seoul 03675 (KR)**

(72) Inventors:
 • **SHIM, Hyun Soo**
　**Seoul 04572 (KR)**
 • **CHANG, Yujung**
　**Seoul 04616 (KR)**

 • **KIM, Chunggu**
　**Incheon 22810 (KR)**
 • **IM, Hyeonjoo**
　**Seoul 06116 (KR)**
 • **KIM, Jong-Seo**
　**Seoul 08826 (KR)**
 • **NA, Yongwoo**
　**Seoul 08830 (KR)**
 • **YOO, Jun Sang**
　**Seoul 03675 (KR)**

(74) Representative: **Vossius & Partner**
**Patentanwälte Rechtsanwälte mbB**
**Siebertstraße 3**
**81675 München (DE)**

Remarks:
The complete document including Reference
Table(s) and the Sequence Listing(s) can be
downloaded from the EPO website

(54)　**NOVEL THERAPEUTIC AGENT FOR PARKINSON'S DISEASE**

(57)　The present application relates to a novel therapeutic agent for treating Parkinson's disease and a method for treating Parkinson's disease using the same.

**EP 4 782 003 A1**

**(Cont. next page)**

【Fig. 7】

**Description**

[Technical Field]

Some embodiments of the present application relate to a composition for treating Parkinson's disease.
Some embodiments of the present application relate to a method for treating Parkinson's disease.

[Background Art]

Parkinson's disease is a brain disorder that causes unintended or uncontrollable movements, such as tremors, rigidity, and difficulties with balance and coordination. Parkinson's disease is classified as a neurodegenerative disease. Damage or degeneration of dopaminergic neurons in the substantia nigra (i.e., death or functional loss of dopaminergic neurons) is known to play a major role in the onset or progression of Parkinson's disease. Parkinson's disease is known to affect both the motor system and the non-motor system. Movement disorders characterized by classical parkinsonian motor symptoms are known to occur due to dopamine deficiency in the basal ganglia, and damage to dopaminergic neurons in the substantia nigra is known to lead to dopamine deficiency in the basal ganglia.
Meanwhile, NDST3, which is also referred to as N-deacetylase and N-sulfotransferase 3, is known to be a protein having a function of catalyzing N-deacetylation and N-sulfation of N-acetylglucosamine residues in heparan sulfate (HS) and heparin. In humans, the human NDST3 protein is encoded by the human NDST3 gene, and the human NDST3 gene is known to be located at chromosome location 4q26. However, the therapeutic effect of the NDST3 protein or a nucleic acid encoding the same on Parkinson's disease has not yet been reported.

[Detailed Description of the Invention]

[Technical Problem]

Since the identification of Parkinson's disease, there has been, and continues to be, a need in the relevant field to identify substances having therapeutic effects for Parkinson's disease. The present application provides a novel therapeutic agent for Parkinson's disease and a method for treating Parkinson's disease using the same.

[Technical Solution]

Some embodiments of the present application provide a novel therapeutic agent for treating Parkinson's disease.
Some embodiments of the present application provide a pharmaceutical composition for treating Parkinson's disease, comprising an NDST3 protein or a nucleic acid encoding the same.
Some embodiments of the present application provide a pharmaceutical composition for treating Parkinson's disease, comprising a vector comprising a nucleic acid encoding an NDST3 protein.
Some embodiments of the present application provide a method for treating Parkinson's disease in a subject, comprising administering to the subject the pharmaceutical composition for treating Parkinson's disease according to some embodiments of the present application.

[Advantageous Effects of the Invention]

The composition for treating Parkinson's disease according to the present application has a therapeutic effect on Parkinson's disease. Through the method for treating Parkinson's disease according to the present application, Parkinson's disease in a subject can be treated.

[Brief Description of the Drawings]

Fig. 1 shows a schematic diagram of a carrier plasmid having a sequence encoding a human NDST3 protein.

Fig. 2 shows a schematic diagram of a nucleic acid included in an AAV vector encoding Ndst3.

Fig. 3 shows a predicted schematic diagram of a nucleic acid included in an AAV vector encoding Ndst3 upon administration to a human.

Fig. 4 shows a schematic diagram of a nucleic acid included in an AAV vector encoding Ndst3. The nucleic acid in the schematic diagram shown in Fig. 4 comprises an NG2 promoter.

Fig. 5 shows a predicted schematic diagram of a nucleic acid included in an AAV vector encoding Ndst3 upon administration to a human. The nucleic acid in the schematic diagram shown in Fig. 5 comprises an NG2 promoter.

Fig. 6 shows in vitro results confirming neuronal markers (TUJ1 and MAP2) of a control group, a 6-OHDA-treated group, and an Ndst3-treated group by immunofluorescence staining.

Fig. 7 shows a graph quantifying the results of confirming neuronal markers (TUJ1 and MAP2) of the control group, the 6-OHDA-treated group, and the Ndst3-treated group by immunofluorescence staining (** $p < 0.01$).

Fig. 8 shows in vitro results confirming a dopaminergic neuronal marker (TH) of the control group, the 6-OHDA-treated group, and the Ndst3-treated group. In Fig. 8, A shows results confirming the expression of the neuronal marker (TH) of the control group, the 6-OHDA-treated group, and the Ndst3-treated group by western blot, and B shows a graph quantifying the results of A in Fig. 8.

Fig. 9 shows results of analyzing action potentials (AP; action potential) of the control group, the 6-OHDA-treated group, and the Ndst3-treated group.

Fig. 10 shows results of analyzing the action potential firing rate (AP firing rate) of the control group, the 6-OHDA-treated group, and the Ndst3-treated group (* $p < 0.05$, ** $p < 0.01$).

Fig. 11 shows results of analyzing the resting membrane potential of a control group, a 6-OHDA-treated group, and an Ndst3-treated group (* $p < 0.05$, ** $p < 0.01$).

Fig. 12 shows results confirming the expression of a dopaminergic neuronal marker (TH) by immunofluorescence staining after treatment with Ndst3 in a 6-OHDA-induced Parkinson's disease mouse model.

Fig. 13 shows a graph quantifying the results of confirming the expression of the dopaminergic neuronal marker (TH) related to Fig. 12 (** $p < 0.01$).

Fig. 14 shows results confirming the expression of a dopaminergic neuronal marker (TH) in the SN (substantia nigra) and the ST (striatum) by immunostaining (DAB staining) after treatment with Ndst3 in a 6-OHDA-induced Parkinson's disease mouse model.

Fig. 15 shows a graph quantifying the results of confirming the expression of the dopaminergic neuronal marker (TH) related to Fig. 14 (** $p < 0.01$).

Fig. 16 shows results confirming the expression of a dopaminergic neuronal marker (TH) in the SNL (lateral substantia nigra), the SNc (substantia nigra pars compacta), and the VTA (ventral tegmental area) by immunostaining (DAB staining) after treatment with Ndst3 in a 6-OHDA-induced Parkinson's disease mouse model.

Fig. 17 shows a graph quantifying the results of confirming the expression of the dopaminergic neuronal marker (TH) related to Fig. 16 (* $p < 0.05$, ** $p < 0.01$).

Fig. 18 shows results confirming the expression of a dopaminergic neuronal marker (DAT) in the SN (substantia nigra) by immunostaining (DAB staining) after treatment with Ndst3 in a 6-OHDA-induced Parkinson's disease mouse model.

Fig. 19 shows a graph quantifying the results of confirming the expression of the dopaminergic neuronal marker (DAT) related to Fig. 18 (** $p < 0.01$).

Fig. 20 shows results confirming the expression of Ndst3 by qRT-PCR after treatment with Ndst3 in a 6-OHDA-induced Parkinson's disease mouse model (** $p < 0.01$).

Fig. 21 shows results confirming the expression of a dopaminergic neuronal marker (TH) in the ST (striatum) and the SN (substantia nigra) by immunostaining (DAB staining) after treatment with Ndst3 in an MPTP-induced Parkinson's disease mouse model.

Fig. 22 shows a graph quantifying the results related to Fig. 21 (** $p < 0.01$).

Fig. 23 shows results confirming the expression of a dopaminergic neuronal marker (DAT) in the SN (substantia nigra) by immunostaining (DAB staining) after treatment with Ndst3 in an MPTP-induced Parkinson's disease mouse model.

Fig. 24 shows a graph quantifying the results related to Fig. 23 (** $p < 0.01$).

Fig. 25 shows results of a rotational test of mice in each group (including a 6-OHDA-treated group and an Ndst3-treated group). In Fig. 25, A shows photographs of the rotational test performed on mice in each group, and B shows a graph quantifying the results of the rotational test (* $p < 0.05$).

Fig. 26 shows results of a tail suspension test of mice in each group (including a 6-OHDA-treated group and an Ndst3-treated group). In Fig. 26, A shows photographs of the tail suspension test performed on mice in each group, and B shows a graph quantifying the results of the tail suspension test (* $p < 0.05$).

Fig. 27 shows results of a cylinder test of mice following intrathecal administration (ICM injection) of Ndst3. Specifically, Fig. 27 shows the results of the mouse cylinder test for each group (including a 6-OHDA-treated group and an Ndst3-treated group). In Fig. 27, A shows a graph quantifying the number of uses of the ipsilateral forelimb in each group, and B shows a graph quantifying the number of uses of the contralateral forelimb in each group.

[Best Mode for Carrying Out the Invention]

Some embodiments of the present application provide a pharmaceutical composition for treating Parkinson's disease, comprising a vector comprising a nucleic acid encoding an NDST3 protein.
In some embodiments, the vector may be a viral vector.
In certain embodiments, the viral vector may be selected from retrovirus, lentivirus, adenovirus, adeno-associated virus, vaccinia virus, poxvirus, HIV (human immunodeficiency virus), MLV (Murine leukemia virus), ASLV (avian sarcoma/leukosis virus), SNV (spleen necrosis virus), RSV (Rous sarcoma virus), MMTV (mouse mammary tumor virus), herpes simplex virus, episomal vector, and herpes virus.
In certain embodiments, the viral vector may be an adeno-associated virus (AAV) vector.
In certain embodiments, the AAV may be selected from AAV1, AAV2, AAV3, AAV4, AAV5, AAV6, AAV7, AAV8, AAV9, AAV10, AAV11, AAV12, AAV13, AAV-DJ, AAV-DJ/8, AAV-Rh10, AAV-retro, AAV-PHP.B, AAV-PHP.eB, and AAV-PHP.S.
In certain embodiments, the AAV may be AAV9.
In some embodiments, the vector may be a non-viral vector.
In certain embodiments, the non-viral vector may be selected from a plasmid, phage, naked DNA, a DNA-RNA lipid complex, liposome, nanoparticle, DNA-polymer complex, mRNA, artificial chromosome, and cosmid.
In some embodiments, the pharmaceutical composition may further comprise one or more pharmaceutically acceptable additional components.
In certain embodiments, each of the pharmaceutically acceptable additional components may be independently selected from a carrier, excipient, diluent, and preservative.
In some embodiments, the NDST3 protein may be a human NDST3 protein.
In some embodiments, the human NDST3 protein may be a wild-type human NDST3 protein or a variant thereof.
In certain embodiments, the human NDST3 protein may comprise the amino acid sequence of SEQ ID NO: 1 or an amino acid sequence having at least 80% and less than 100% sequence identity thereto.
In certain embodiments, the human NDST3 protein may comprise the amino acid sequence of SEQ ID NO: 1.
In certain embodiments, the human NDST3 protein may be a wild-type human NDST3 protein or a functional equivalent thereof.
In certain embodiments, the human NDST3 protein may be a variant of a wild-type human NDST3 protein. In this case, the variant of the wild-type NDST3 protein may comprise one or more conservative substitutions of amino acid residues in the wild-type human NDST3 protein having the amino acid sequence of SEQ ID NO: 1, thereby the human NDST3 protein may comprise an amino acid sequence having at least 80% and less than 100% sequence identity to the amino acid sequence of the wild-type human NDST3 protein.
In some embodiments, the nucleic acid encoding the NDST3 protein may comprise an NDST3 protein-encoding region, wherein the NDST3 protein-encoding region may comprise a nucleic acid sequence encoding the NDST3 protein.
In certain embodiments, the nucleic acid sequence encoding the NDST3 protein may be a DNA sequence, an RNA sequence, or a DNA/RNA hybrid sequence.

In certain embodiments, the nucleic acid sequence encoding the NDST3 protein may comprise a full-length sequence of an NDST3 gene, an open reading frame (ORF) sequence of the NDST3 gene, a coding sequence (CDS) of the NDST3 gene, or a cDNA sequence of the NDST3 gene.

In certain embodiments, the nucleic acid sequence encoding the NDST3 protein may comprise a full-length sequence of a wild-type human NDST3 gene, an open reading frame (ORF) sequence of the wild-type human NDST3 gene, a coding sequence (CDS) of the wild-type human NDST3 gene, or a cDNA sequence of the wild-type human NDST3 gene.

In certain embodiments, the nucleic acid sequence encoding the NDST3 protein may comprise the nucleic acid sequence of SEQ ID NO: 7 or a nucleic acid sequence having at least 80% and less than 100% sequence identity thereto.

In certain embodiments, the nucleic acid sequence encoding the NDST3 protein may comprise the nucleic acid sequence of SEQ ID NO: 7.

In certain embodiments, the nucleic acid encoding the NDST3 protein may further comprise one or more additional elements.

In certain embodiments, each of the one or more additional elements may be independently selected from a promoter, enhancer, polyadenylation signal, Kozak consensus sequence, inverted terminal repeat (ITR), long terminal repeat (LTR), terminator, origin of replication, multicloning site (MCS), internal ribosome entry site (IRES), a nuclear localization signal-encoding sequence, and 2A self-cleaving peptides.

In certain embodiments, the nucleic acid encoding the NDST3 protein may further comprise a promoter, wherein the promoter may be operably linked to the NDST3 protein-encoding region.

In certain embodiments, the promoter may be an SV40 early promoter, an LTR (mouse mammary tumor virus long terminal repeat) promoter, an Ad MLP (adenovirus major late) promoter, an HSV (herpes simplex virus) promoter, a CMV (cytomegalovirus) promoter, an RSV (Rous sarcoma virus) promoter, a U6 promoter, a CBA promoter, a PGK promoter, an NG2 promoter, an NES promoter, a GFAP promoter, a CaMKII promoter, an NSE promoter, a SYN1 promoter, or a CAG promoter.

Some embodiments of the present application provide a pharmaceutical composition for treating Parkinson's disease, comprising:

a viral vector encoding an NDST3 protein,

wherein the viral vector encoding the NDST3 protein comprises a nucleic acid encoding the NDST3 protein.

In some embodiments, the NDST3 protein may comprise the amino acid sequence of SEQ ID NO: 1 or an amino acid sequence having at least 80% and less than 100% sequence identity thereto.

In some embodiments, the nucleic acid encoding the NDST3 protein may comprise an NDST3 protein-encoding region, wherein the NDST3 protein-encoding region may comprise the nucleic acid sequence of SEQ ID NO: 7 or a nucleic acid sequence having at least 80% and less than 100% sequence identity thereto.

In certain embodiments, the nucleic acid encoding the NDST3 protein may further comprise a promoter, wherein the promoter may be operably linked to the NDST3 protein-encoding region.

In certain embodiments, the promoter may be an SV40 early promoter, an LTR (mouse mammary tumor virus long terminal repeat) promoter, an Ad MLP (adenovirus major late) promoter, an HSV (herpes simplex virus) promoter, a CMV (cytomegalovirus) promoter, an RSV (Rous sarcoma virus) promoter, a U6 promoter, a CBA promoter, a PGK promoter, an NG2 promoter, an NES promoter, a GFAP promoter, a CaMKII promoter, an NSE promoter, a SYN1 promoter, or a CAG promoter.

In some embodiments, the viral vector may be an AAV vector.

In some embodiments, the viral vector may be an AAV9 vector.

Some embodiments of the present application provide a method for treating Parkinson's disease in a subject, comprising: administering to the subject the pharmaceutical composition for treating Parkinson's disease according to some embodiments of the present application.

In some embodiments, the subject may be a subject having Parkinson's disease.

In some embodiments, the pharmaceutical composition may be administered to the subject via a parenteral route.

In some embodiments, the pharmaceutical composition may be administered intravenously, subcutaneously, intramuscularly, intradermally, intraperitoneally, intra-arterially, mucosally, spinally, intrathecally, nasally, or intracerebrally.

[Modes for Carrying Out the Invention]

Hereinafter, the invention disclosed in the present application will be described in further detail through embodiments and examples. The invention disclosed in the present application may be implemented in various forms and is not limited to the specific embodiments described herein.

Those skilled in the art to which the invention disclosed in the present application pertains will be able to conceive of various modifications and other embodiments based on the disclosure of the present application. Accordingly, the invention disclosed in the present specification should not be construed as being limited to the specific embodiments or examples described herein, and modifications and other embodiments thereof should also be understood to fall within the scope of the invention disclosed in the present application.

## Definition of Terms

Unless otherwise defined, all technical and scientific terms used herein have the meanings commonly understood by those skilled in the art to which the present application pertains. All publications, patents, and other references cited herein are incorporated by reference in their entirety.

As used herein, the term "about" refers to a value that may vary by approximately 30, 25, 20, 15, 10, 9, 8, 7, 6, 5, 4, 3, 2, or 1% with respect to a referenced amount, level, value, number, frequency, percentage, dimension, size, quantity, weight, or length.

As used herein, the term "treating" refers to alleviating, ameliorating, and/or inhibiting the progression of a disorder, disease, and/or symptom. The term "treating," as used in the present application, encompasses alleviation and amelioration of a disorder, disease, and/or symptom. For example, treatment may refer to a partial or complete recovery of a disorder, disease, and/or symptomatic condition to a normal state. For example, the treatment may refer to a change in condition in which symptoms are improved or beneficially altered by the composition of the present application, or any action that causes the symptoms to be improved or altered. In some cases, treatment may be interpreted as including aspects of prevention.

As used herein, the terms "protein," "peptide," and "polypeptide" are used interchangeably and encompass naturally occurring proteins as well as non-naturally occurring proteins. Non-naturally occurring proteins may include, for example, recombinant proteins or synthetic proteins. For example, a protein, peptide, or polypeptide may be naturally occurring, recombinant, synthetic, or any combination thereof. For example, the protein, peptide, or polypeptide may be naturally occurring. For example, the protein, peptide, or polypeptide may be recombinant or synthetic, or a combination of recombinant or synthetic forms with naturally occurring forms.

As used herein, the term "nucleic acid" encompasses naturally occurring nucleic acids as well as non-naturally occurring nucleic acids. Non-naturally occurring nucleic acids may include, for example, recombinant nucleic acids or synthetic nucleic acids. For example, the nucleic acid may be naturally occurring, recombinant, synthetic, or any combination thereof. For example, the nucleic acid may be naturally occurring. For example, the nucleic acid may be recombinant or synthetic, or a combination of recombinant or synthetic forms with naturally occurring forms.

As used herein, the term "amino acid" may refer to both an amino acid that is not bound to another amino acid and an amino acid residue bound to other amino acids within a protein, polypeptide, or peptide, and may be appropriately interpreted depending on the content or context of the paragraph in which the term "amino acid" is used. The term "amino acid," as used herein, may include both natural amino acids and unnatural amino acids. As used herein, a "natural amino acid" collectively refers to the twenty amino acids synthesized in the human body through transcription and translation processes of genes. Specifically, the natural amino acids include alanine (Alanine; Ala, A), arginine (Arginine; Arg, R), asparagine (Asparagine; Asn, N), aspartic acid (Aspartic acid; Asp, D), cysteine (Cysteine; Cys, C), glutamic acid (Glutamic acid; Glu, E), glutamine (Glutamine; Gln, Q), glycine (Glycine; Gly, G), histidine (Histidine; His, H), isoleucine (Isoleucine; Ile, I), leucine (Leucine; Leu, L), lysine (Lysine; Lys, K), methionine (Methionine; Met, M), phenylalanine (Phenylalanine; Phe, F), proline (Proline; Pro, P), serine (Serine; Ser, S), threonine (Threonine; Thr, T), tryptophan (Tryptophan; Trp, W), tyrosine (Tyrosine; Tyr, Y), and valine (Valine; Val, V). As used herein, an "unnatural amino acid" refers to an amino acid that is not synthesized in the human body through transcription and translation of genes, but is synthesized through processes other than transcription and translation, is artificially synthesized, or is synthesized in organisms other than humans. As described above, the term "amino acid" as used herein may refer to both an amino acid that is not bound to another amino acid and an amino acid residue bound to other amino acids within a protein or peptide. As used herein, the term "amino acid residue" refers to a structure derived from an amino acid that is included in a compound, peptide, and/or protein and is covalently linked to other portions of the compound, peptide, and/or protein. For example, when alanine, arginine, and glutamic acid are linked via amide bonds to form a peptide having an ARE sequence, the peptide comprises three amino acid residues, wherein A may refer to an alanine residue, R may refer to an arginine residue, and E may refer to a glutamic acid residue. Furthermore, as described above, in the peptide having the ARE sequence, the peptide may comprise three amino acids, and A may also refer to alanine, R to arginine, and E to glutamic acid. As another example, when aspartic acid, phenylalanine, and lysine are linked via amide bonds to form a peptide having a DFK sequence, the peptide comprises three amino acid residues, wherein D may refer to an aspartic acid residue, F may refer to a phenylalanine residue, and K may refer to a lysine residue. Furthermore, as described above, in the peptide having the DFK sequence, the peptide may comprise three amino acids, and D may also refer to aspartic acid, F to phenylalanine, and K to lysine.

Unless otherwise specified, when an amino acid sequence is described in the present specification, the amino acid sequence is written from the N-terminus to the C-terminus using either the one-letter amino acid code or the three-letter amino acid code. For example, when expressed as RNVP, it refers to a peptide in which arginine, asparagine, valine, and proline are sequentially linked from the N-terminus to the C-terminus. As another example, when expressed as Thr-Leu-Lys, it refers to a peptide in which threonine, leucine, and lysine are sequentially linked from the N-terminus to the C-terminus. In the case of amino acids that cannot be represented using the one-letter code, they may be represented using other characters and further explained as necessary.

Unless otherwise specified, nucleic acid sequences disclosed in the present specification (for example, DNA sequences, RNA sequences, or DNA/RNA hybrid sequences) should be understood as being written in the 5' to 3' direction. In the case of DNA, which is a type of nucleic acid, it is well known that two strands (for example, a first strand and a second strand) may form a double helix structure (for example, in human genomic DNA). Accordingly, when a certain nucleic acid sequence (e.g., a DNA sequence) is disclosed, it is desirable that, unless otherwise specified, a sequence complementary to the disclosed nucleic acid sequence is also considered to be disclosed in the present specification. Furthermore, unless otherwise specified, when a certain DNA sequence is disclosed, an RNA sequence corresponding to the DNA sequence (for example, a sequence in which all T residues of the DNA sequence are replaced with U residues) and an RNA sequence transcribed from the DNA sequence are also considered to be disclosed in the present specification.

As used herein, the term "sequence identity" refers to a term used in relation to the degree of similarity between two or more sequences. For example, the term "sequence identity" may be used together with a term referring to a reference sequence and a term indicating a ratio (e.g., a percentage). For example, the term "sequence identity" may be used to describe a sequence that is similar or substantially identical to a reference nucleic acid sequence. When described as "a sequence having at least 90% sequence identity to sequence A," the reference sequence is sequence A. For example, the percentage of sequence identity may be calculated by aligning the reference sequence and the sequence to be measured for sequence identity. The percentage of sequence identity may be calculated by including mismatches, deletions, and insertions of one or more nucleotides. The method for calculating and/or determining the percentage of sequence identity is not particularly limited and may be calculated and/or determined using any suitable method or algorithm available to those skilled in the art.

As used herein, when an expression such as "A comprises B" is used, the expression "A comprises B" should be interpreted as not excluding the possibility that A may further comprise additional elements in addition to B. Furthermore, the expression "A comprises B" may be alternatively expressed as "A is B," "A consists of B," or "A is represented by B."

## Parkinson's Disease

This section relating to Parkinson's disease is provided to facilitate understanding of Parkinson's disease, and the scope of the present application is not limited by the description of Parkinson's disease set forth in this section. Parkinson's disease is a brain disorder that causes unintended or uncontrollable movements, such as tremors, rigidity, and difficulties with balance and coordination. Parkinson's disease is classified as a neurodegenerative disease. Damage or degeneration of dopaminergic neurons in the substantia nigra (i.e., death or functional loss of dopaminergic neurons) is known to play a major role in the onset or progression of Parkinson's disease. Parkinson's disease is known to affect both the motor system and the non-motor system. Movement disorders characterized by classical parkinsonian motor symptoms are known to occur due to dopamine deficiency in the basal ganglia, and damage to dopaminergic neurons in the substantia nigra is known to lead to dopamine deficiency in the basal ganglia. Symptoms of Parkinson's disease are known to include motor symptoms and non-motor symptoms, and the symptoms may vary to some extent depending on the individual patient. Examples of motor symptoms include tremor, slowness of movement (bradykinesia), rigidity, and postural instability such as problems with gait and balance. Examples of non-motor symptoms include depression, anxiety, apathy, hallucinations, constipation, orthostatic hypotension, sleep disorders, loss of sense of smell, and various cognitive impairments.

Parkinson's disease is generally classified into three stages: an early stage, a mid stage, and an advanced stage, or alternatively into five stages: stage 1, stage 2, stage 3, stage 4, and stage 5.

Such information relating to Parkinson's disease is well known in the art. Details regarding Parkinson's disease are described in the following references: Greenamyre, J. T., & Hastings, T. G. (2004). Parkinson's-divergent causes, convergent mechanisms. Science, 304(5674), 1120-1122; Ziemssen, T., & Reichmann, H. (2007). Non-motor dysfunction in Parkinson's disease. Parkinsonism & Related Disorders, 13(6), 323-332; and Bose, A., & Beal, M. F. (2016).

Mitochondrial dysfunction in Parkinson's disease. Journal of Neurochemistry, 139, 216-231. The entire contents of each of these references are incorporated herein by reference.

The need for the development of novel therapeutic agents for treating Parkinson's disease has continuously existed in the relevant field since the identification of Parkinson's disease and still exists today.

**NDST3**

This section relating to NDST3 is provided to facilitate understanding of NDST3, and the scope of the present application is not limited by the description of NDST3 provided in this section. NDST3, also referred to as N-deacetylase and N-sulfotransferase 3, is a protein known to catalyze N-deacetylation and N-sulfation of N-acetylglucosamine residues in heparan sulfate (HS) and heparin. In humans, the human NDST3 protein is encoded by the human NDST3 gene, which is known to be located at the chromosomal location 4q26. Information regarding the human NDST3 gene encoding the NDST3 protein, which is located on chromosome 4, can be accessed through the National Center for Biotechnology Information (NCBI), and the Gene ID of the human NDST3 gene is 9348. The amino acid sequence of the known wild-type human NDST3 protein and the NCBI accession information are disclosed in Table 1 below.

## Table 1. Amino Acid Sequence Information of the Wild-Type Human NDST3 Protein

| Name | Amino Acid Sequence (SEQ ID No) | Accession |
|------|--------------------------------|-----------|
| Wild-type human NDST3 protein | MSFIMKLHRHFQRTVYLLATFCWVSIIISAVYLYSGYKQENELSETASEVDCGDLQHL PYQLMEVKAMKLFDASRTDYTVLVFVESQYSSLGQDIIMILESSRFQYHIEIAPGKGD LFVLIDXNRGKYILIIYEVILKYINMDSWNRSLLDKYCVEYGVGVIGFHKTSEKSVQS FQLKGFPFSIYGNLAVKDCCINPHSPLIRVTKSSKLERGSLFGTDWTVFQINHSAYQP VIFAKVKTPENLSPSISKGAPYATIIHDLGLHDGIQRVLFGXXLNFWLHKLIFIDAIS FLSGKRLTLSLDRYILVDIDDIFVGKEGTRXYTNDVKALLDTQNLLRAQITNFTFXLG FSGKFYHTGTEEEDEGDDCLLGSVDEFWWFMDXWSHMQFHLFHNESSLVEQMILXKKF ALEHGIFTDWGYAVAFHHSGVYFVHVQLYEAWKKVWNIKITSTEEYFMLKPARYKRGF IHKVLWVLPRQTCGLFTHTIFYKEYPGGFKELDKSIQGGELFFTVVLNFISIFMTHLS NYGNDRLGLYTFVNLANFVKSWTNLRLQTLFTVQLAHKYFELFTDQKDPLWQNFCDDK RHRDIWSKEKTCDRLFKFLVIGFQKTGTTALYLFLVMHFSILSNSFSFKTFEEVQFFY RXNVYHRGIDWYMDFFFVFSWVTTDFLFEKSAXYFHSEEAFKRAASLVTKAKIITILID PSDRAYSWYQHQRSHEDFAALKFSFYEVISAGFRAFSELRALQKRCLVFGWYASHIER WLVYFFFQLLIIDGQXLRTDFATVMDEVQKFLGVLFHYNYSEALTFDSHKGFWCQLL EEGKTKCLGFSKGRRYFFMDSDSRTFLSSYYTRDHNVELSKLLHKLGQFLFSWLRQELQ KVR (SEQ ID NO: 1) | NP_004775.1 |

It is known that the wild-type human NDST3 protein has isoforms, and the amino acid sequences and NCBI accession numbers thereof are disclosed in Table 2 below.

Table 2. Amino Acid Sequence Information of Isoforms of the Wild-Type Human

NDST3 Protein

| Name | Amino Acid Sequence (SEQ ID No.) | Accession |
|---|---|---|
| Human NDST3 protein isoform X1 | MSFIMKLHRHPQRTVILLATFCMVSIIISAYYLYSGYKQENELSETASEVDCGDLQHL PYQLMEVKAMKLFDASRTDPTVLVFVESQYSSLGQDIIMILESSRFQYHIEIAPGKGD LPVLIDKMKGKYILIIYENILKYINMDSWNRSLLDKYCVEYGVGVIGFHKTSEKSYQS FQLKGFPFSIYGNLAVKDCCINPHSPLIRVTKSSKLEKGSLPGTDWTVFQINHSAYQP VIFAKVKTPENLSPSISKGAFYATIIHDLGLHDGIQRVLFGXNLNFWLHKLIFIDAIS FLSGKRLTLSLDRYILVDIDDIFVGKEGTRMXTNDVKALLDTQNLLRAQITNFTFNLG FSGKFYHTGTEEEDEGDDCLLGSVDEFWWFTHMWSHMQPHLFHNESSLVEQMILNKKF ALEHGIPTDMGYAVAPHHSGVYPVHVQLYEAWKKVWNIKITSTEEYPHLKPARYRRGF IHKNIMVLPRQTCGLFTHTIFYKEYPGGPKELDKSIQGGELFFTVVLNPISIFMTHLS MYGNDRLGLYTFVNLANFVKSWTNLRLQTLPPVQLAHKYFELFTDQKDPLWQNTCDDK RHRDIWSKEKTCDRLPKFLVIGPQKTGTTALYLFLVMHPSILSNSPSPKTFEEVQFFM RNNYHRGIDWYMDFFPVPSNVTTDFLFEKSANYFHSEEAPKRAASLVPKAKIITILID PSDRAYSWYQHQRSHEDPAALKFSFYEVISAGPRAPSELRALQKRCLVPGWYASHIER WLVYFPTPQLLIDGQQLRTDPATVMDEVQKFLGVLPHYNYSEALTFDSHKGFWCQLL EEGKTKCLGKSKGRKYPPMDSDDISVKLLSRSQRGTLKAAAQTGSASAILAETGAAES KIALREMLRLHRPCRTHLFQSFQKLPKGS (SEQ ID NO: 02) | XP_0168643 29.1 |
| Human NDST3 protein isoform X3 | MSFIMKLHRHPQRTVILLATFCMVSIIISAYYLYSGYKQENELSETASEVDCGDLQHL PYQLMEVKAMKLFDASRTDPTVLVFVESQYSSLGQDIIMILESSRFQYHIEIAPGKGD LPVLIDKMKGKYILIIYENILKYINMDSWNRSLLDKYCVEYGVGVIGFHKTSEKSYQS FQLKGFPFSIYGNLAVKDCCINPHSPLIRVTKSSKLEKGSLPGTDWTVFQINHSAYQP VIFAKVKTPENLSPSISKGAFYATIIHDLGLHDGIQRVLFGXNLNFWLHKLIFIDAIS FLSGKRLTLSLDRYILVDIDDIFVGKEGTRMXTNDVKALLDTQNLLRAQITNFTFNLG FSGKFYHTGTEEEDEGDDCLLGSVDEFWWFPHMWSHMQPHLFHNESSLVEQMILNKKF ALEHGIPTDMGYAVAPHHSGVYPVHVQLYEAWKKVWNIKITSTEEYPHLKPARYRRGF IHKNIMVLPRQTCGLFTHTIFYKEYPGGPKELDKSIQGGELFFTVVLNPFIIDCKLNR QS (SEQ ID NO: 03) | XP_011530 717.1 |
| Human NDST3 protein isoform X4 | MWSHMQPHLFHNESSLVEQMILNKKFALEHGIPTDMGYAVAPHHSGVYPVHVQLYEAW KKVWNIKITSTEEYPHLKPARYRRGFIHKNIMVLPRQTCGLFTHTIFYKEYPGGPKEL DKSIQGGELFFTVVLNPISIFMTHLSMYGNDRLGLYTFVNLANFVKSWTNLRLQTLPP VQLAHKYFELFTDQKDPLWQNTCDDKRHRDIWSKEKTCDRLPKFLVIGPQKTGTTALY LFLVMHPSILSNSPSPKTFEEVQFFMRNNYHRGIDWYMDFFPVPSNVTTDFLFEKSAN YFHSEEAPKRAASLVPKAKIITILIDPSDRAYSWYQHQRSHEDPAALKFSFYEVISAG PRAPSELRALQKRCLVPGWYASHIERWLVYFPTPQLLIDGQQLRTDPATVMDEVQKF LGVLPHYNYSEALTFDSHKGFWCQLLEEGKTKCLGKSKGRKYPPMDSDDISVKLLSRS QRGTLKAAAQTGSASAILAETGAAESKIALREMLRLHRPCRTHLFQSFQKLPKGS (SEQ ID NO: 04) | XP_0168643 33.1 |
| Human NDST3 protein isoform X5 | MSFIMKLHRHPQRTVILLATFCMVSIIISAYYLYSGYKQENELSETASEVDCGDLQIL PYQLMEVKAMKLFDASRTDPTVLVFVESQYSSLGQDIIMILESSRFQYHIEIAPGKGD LPVLIDKMKGKYILIIYENILKYINMDSWNRSLLDKYCVEYGVGVIGFHKTSEKSYQS FQLKGFPFSIYGNLAVKDCCINPHSPLIRVTKSSKLEKGSLPGTDWTVFQINHSAYQP | XP_047272 371.1 |

| | | |
|---|---|---|
| | YIFAKVKTPENLSPSISKGAFYATIIHDLGLHDGIQRVLFGNNLNFWLHKLIFIDAIS FLSGKRLTLSLDRYTLVDIDDIFVGKEGTRMNTNDVKALLDTQNLLRAQITNFTFNLG FSGKPYHTGTEEEDEGDDCLLGSVDEPWWFTHWWSHAQPHLFHNESSLVEQMILNKKF ALEHGIPTDMGYAVAPHHSGVYPVHVQLYEAWKKVWNIKITSTEEYPHLKPARYRRGF IHKNIWVLPRQTCGLFTHTIFYKEYPGGPKELDKSIQGGELFFTVVLNPKMR (SEQ ID NO: 05) | |
| Human NDST3 protein isoform X6 | MGYAVAPHHSGVYPVHVQLYEAWKKVWNIKITSTEEYPHLKPARYRRGFIHKNIWVLP RQTCGLFTHTIFYKEYPGGPKELDKSIQGGELFFTVVLNPISIFWTHLSNYGNDRLGL YTFVNLANFVKSWTNLRLQTLPPVQLAHKYFELFPDQKDPLWQNPCDDKRHRDIWSKE KTCDRLPKFLVIGPQKTGTTALYLFLVMHPSILSNSPSPKTFEEVQFFNKNNYHRGID WYMDFFPVPSWVTTDFLFEKSAWYFHSEEAPKRAASLVPKAKIITILIDPSDRAYSWY QHQRSHEDPAALKFSFYEVISAGPRAPSELRALQKRCLVPGWYASHTERWLVYFPTFQ LLIIDGQQLRTDPATVMDEVQKFLGVLPHYNYSEALTFDSHKGFWCQLLEEGKTRCLG KSKGRKYPMDSDDISVKLLSRSQRGTLKAAAQTGSASAILAETGAAESKIALRENLR LHRPCRTHLFQSFQKLPKGS (SEQ ID NO: 06) | XP_016864 332.1 |

Meanwhile, as described above, the therapeutic effect of NDST3 (NDST3 protein or a nucleic acid encoding the same) on Parkinson's disease had not been known prior to the filing date of the present application.

In some embodiments of the present application, the therapeutic agent for Parkinson's disease provided by the present application is NDST3. In some embodiments, the novel therapeutic agent for Parkinson's disease provided by the present application is an NDST3 protein or a nucleic acid encoding the NDST3 protein. In some embodiments, the present application provides a composition for treating Parkinson's disease comprising NDST3 (or an NDST3 protein or a nucleic acid encoding the same).

Hereinafter, the composition for treating Parkinson's disease will be described in detail.

## Composition for Treating Parkinson's Disease

### Overview of Composition for Treating Parkinson's Disease

Some embodiments of the present application provide a composition for treating Parkinson's disease (or a composition for the treatment of Parkinson's disease). The composition for treating Parkinson's disease according to the present application may be used to treat Parkinson's disease in a subject. For example, the composition for treating Parkinson's disease according to the present application may be used to treat Parkinson's disease in a subject. For example, the composition of the present application may be used to treat a human having Parkinson's disease or diagnosed with Parkinson's disease. For example, the composition of the present application may be used to treat symptoms of Parkinson's disease in a subject.

The designation referring to the composition for treating Parkinson's disease according to the present application is not particularly limited. For example, the composition for treating Parkinson's disease may be referred to by other names, such as a composition, a composition comprising NDST3, a composition comprising an NDST3 protein or a nucleic acid encoding the same, or a composition comprising a therapeutic agent for Parkinson's disease.

In some embodiments, the composition for treating Parkinson's disease according to the present application comprises an NDST3 protein or a nucleic acid encoding the NDST3 protein.

In some embodiments, the composition for treating Parkinson's disease may comprise, as an active substance (active ingredient or active pharmaceutical ingredient), an NDST3 protein or a nucleic acid encoding the NDST3 protein. In some embodiments, the composition for treating Parkinson's disease may comprise an NDST3 protein as the active substance. In some embodiments, the composition for treating Parkinson's disease may comprise a nucleic acid encoding the NDST3 protein as the active substance. In some embodiments, the composition for treating Parkinson's disease may comprise, as the active substance, a vector comprising a nucleic acid encoding the NDST3 protein or a vector encoding the NDST3 protein. In some embodiments, the composition for treating Parkinson's disease may comprise a therapeutically effective amount of the active substance. As used herein, the term "effective amount" may refer to an amount of a biologically active agent sufficient to elicit a desired biological response.

Hereinafter, the NDST3 protein or a nucleic acid encoding the same will be described in detail.

### NDST3 Protein or Nucleic Acid Encoding the Same

In some embodiments, the composition for treating Parkinson's disease according to the present application comprises an NDST3 protein or a nucleic acid encoding the same. In some embodiments, the composition for treating Parkinson's disease may comprise an NDST3 protein or a nucleic acid encoding the same as an active substance. In some embodiments, the composition for treating Parkinson's disease may comprise an effective amount (or a pharmaceutically effective amount or a therapeutically effective amount) of the NDST3 protein or an effective amount (or a pharmaceutically effective amount or a therapeutically effective amount) of a nucleic acid encoding the NDST3 protein.

In some embodiments, the composition for treating Parkinson's disease comprises an NDST3 protein. For example, the NDST3 protein may be included in a carrier for protein delivery (e.g., a liposome, nanoparticle, or delivery protein) or may be linked or attached to such a carrier. In some embodiments, the composition for treating Parkinson's disease comprises a nucleic acid encoding the NDST3 protein. For example, the nucleic acid encoding the NDST3 protein may be packaged into a vector (e.g., a plasmid, a viral vector such as an AAV vector, or a cosmid). That is, the composition for treating Parkinson's disease may comprise a vector comprising a nucleic acid encoding the NDST3 protein.

The NDST3 protein and the nucleic acid encoding the NDST3 protein will be described in detail below.

**NDST3 Protein**

In some embodiments, the composition for treating Parkinson's disease according to the present application may comprise a nucleic acid encoding an NDST3 protein. In some embodiments, the composition for treating Parkinson's disease according to the present application may comprise an NDST3 protein. In some embodiments, the composition for treating Parkinson's disease may comprise the NDST3 protein as an active substance.

In some embodiments, the NDST3 protein may be naturally occurring, recombinant, synthetic, or a combination thereof. In some embodiments, the NDST3 protein may be naturally occurring. In some embodiments, the NDST3 protein may be a recombinant NDST3 protein. In some embodiments, the NDST3 protein may be synthetic.

In some embodiments, the NDST3 protein may be a human NDST3 protein (e.g., derived from a human). In some embodiments, the NDST3 protein may be a wild-type human NDST3 protein or a variant thereof. In some embodiments, the NDST3 protein may be selected from a canine NDST3 protein (e.g., derived from dog), an equine NDST3 protein (e.g., derived from horse), a feline NDST3 protein (e.g., derived from cat), a camel NDST3 protein (e.g., derived from camel), a mouse NDST3 protein (e.g., derived from mouse), a rat NDST3 protein (e.g., derived from rat), a porcine NDST3 protein (e.g., derived from pig), a rabbit NDST3 protein (e.g., derived from rabbit), an ovine NDST3 protein (e.g., derived from sheep), a monkey NDST3 protein (e.g., derived from monkey), a chimpanzee NDST3 protein (e.g., derived from chimpanzee), and a bovine NDST3 protein (e.g., derived from cattle). In some embodiments, the NDST3 protein may be selected from a wild-type canine NDST3 protein or a variant thereof, a wild-type equine NDST3 protein or a variant thereof, a wild-type feline NDST3 protein or a variant thereof, a wild-type camel NDST3 protein or a variant thereof, a wild-type mouse NDST3 protein or a variant thereof, a wild-type rat NDST3 protein or a variant thereof, a wild-type porcine NDST3 protein or a variant thereof, a wild-type rabbit NDST3 protein or a variant thereof, a wild-type ovine NDST3 protein or a variant thereof, a wild-type monkey NDST3 protein or a variant thereof, a wild-type chimpanzee NDST3 protein or a variant thereof, and a wild-type bovine NDST3 protein or a variant thereof. In some embodiments, the NDST3 protein may be derived from a human.

In some embodiments, the NDST3 protein may include modifications commonly applied in the relevant art. In some embodiments, the NDST3 protein may further comprise modifications and/or additional elements. Here, the modification may include, for example, addition of polyethylene glycol (e.g., PEGylation), addition of a linker (e.g., a peptide linker), functionalization, modification for conjugation, addition of a hydrophilic moiety, addition of a hydrophobic moiety, addition of a PEG moiety (e.g., PEGylation), addition of an amide group (e.g., C-terminal amidation), addition of a carbohydrate group, addition of a hydroxyl group, addition of a phosphate group, addition of a prenyl group (e.g., prenylation), and/or addition of a farnesyl group (e.g., farnesylation). In some embodiments, the modification applied to the NDST3 protein may be a modification commonly applied to proteins in the relevant art. In some embodiments, the modification applied to the NDST3 protein may be at a level that does not impair the intrinsic function of the NDST3 protein.

As described above, the NDST3 protein may be a human NDST3 protein. In some embodiments, the NDST3 protein may have or comprise the amino acid sequence of SEQ ID NO: 1 or an amino acid sequence having at least 80% sequence identity thereto. In some embodiments, the NDST3 protein may have the amino acid sequence of SEQ ID NO: 1 or an amino acid sequence having at least 80% and less than 100% sequence identity thereto. Here, the amino acid sequence of SEQ ID NO: 1 may be the amino acid sequence of the wild-type human NDST3 protein.

Hereinafter, aspects of the NDST3 protein will be described in detail.

In some embodiments, the NDST3 protein may comprise the amino acid sequence of SEQ ID NO: 1 or an amino acid sequence having at least 80% and less than 100% sequence identity thereto. In some embodiments, the NDST3 protein may be a protein having the amino acid sequence of SEQ ID NO: 1 or a protein having an amino acid sequence having at least 80% and less than 100% (e.g., 99.9% or less) sequence identity to the amino acid sequence of SEQ ID NO: 1. In some embodiments, the NDST3 protein may comprise the amino acid sequence of SEQ ID NO: 1 or an amino acid sequence having at least 80% and less than 100%, at least 82% and less than 100%, at least 84% and less than 100%, at least 86% and less than 100%, at least 88% and less than 100%, at least 90% and less than 100%, at least 92% and less than 100%, at least 94% and less than 100%, at least 95% and less than 100%, at least 96% and less than 100%, at least 97% and less than 100%, at least 97.5% and less than 100%, at least 98% and less than 100%, at least 98.5% and less than 100%, at least 99% and less than 100%, or at least 99.5% and less than 100% sequence identity to the amino acid sequence of SEQ ID NO: 1. In certain embodiments, the NDST3 protein may comprise the amino acid sequence of SEQ ID NO: 1 or an amino acid sequence having at least 90% and less than 100%, at least 92% and less than 100%, at least 94% and less than 100%, at least 95% and less than 100%, at least 96% and less than 100%, at least 97% and less than 100%, at least 97.5% and less than 100%, at least 98% and less than 100%, at least 98.5% and less than 100%, at least 99% and less than 100%, or at least 99.5% and less than 100% sequence identity to the amino acid sequence of SEQ ID NO: 1. In certain embodiments, the NDST3 protein may comprise the amino acid sequence of SEQ ID NO: 1 or an amino acid sequence having at least 95% and less than 100%, at least 96% and less than 100%, at least 97% and less than 100%, at least 97.5% and less than 100%, at least 98% and less than 100%, at least 98.5% and less than 100%, at least 99% and less than 100%, or at least 99.5% and less than 100% sequence identity to the amino acid sequence of SEQ ID NO: 1. In some embodiments, the NDST3 protein may comprise the amino acid sequence of SEQ ID NO: 1 or an amino acid sequence having about 80%, 81%, 82%, 83%, 84%, 85%, 86%, 87%, 88%, 89%, 90%, 91%, 92%, 93%, 94%, 95%, 96%, 97%, 97.2%, 97.4%, 97.6%, 97.8%, 98%, 98.2%, 98.4%, 98.6%, 98.8%, 99%, 99.1%, 99.2%, 99.3%, 99.4%, 99.5%, 99.6%, 99.7%, 99.8%, or 99.9% sequence identity to the amino acid sequence of SEQ ID NO: 1. In certain embodiments, the NDST3 protein may comprise the amino acid sequence of SEQ ID NO: 1 or an amino acid sequence having about 90%, 91%, 92%, 93%, 94%, 95%, 96%, 97%, 97.2%, 97.4%, 97.6%, 97.8%, 98%, 98.2%, 98.4%, 98.6%, 98.8%, 99%, 99.1%, 99.2%, 99.3%, 99.4%, 99.5%, 99.6%, 99.7%, 99.8%, or 99.9% sequence identity to the amino acid sequence of SEQ ID NO: 1. In certain embodiments, the NDST3 protein may comprise the amino acid sequence of SEQ ID NO: 1 or an amino acid sequence having about 95%, 96%, 97%, 97.2%, 97.4%, 97.6%, 97.8%, 98%, 98.2%, 98.4%, 98.6%, 98.8%, 99%, 99.1%, 99.2%, 99.3%, 99.4%, 99.5%, 99.6%, 99.7%, 99.8%, or 99.9% sequence identity to the amino acid sequence of SEQ ID NO: 1. In certain embodiments, the NDST3 protein may comprise the amino acid sequence of SEQ ID NO: 1.

In some embodiments, the NDST3 protein may be a human NDST3 protein. In some embodiments, the human NDST3 protein may be a wild-type human NDST3 protein or a variant of the human NDST3 protein (e.g., a variant of the wild-type human NDST3 protein).

In some embodiments, the wild-type human NDST3 protein may comprise the amino acid sequence of SEQ ID NO: 1.

In some embodiments, a variant of the human NDST3 protein may be generated or derived by insertion of one or more amino acid residues into the wild-type human NDST3 protein, deletion of one or more amino acid residues of the wild-type human NDST3 protein, and/or substitution of one or more amino acid residues of the wild-type human NDST3 protein (i.e., substitution with another amino acid residue). In some embodiments, there may be a difference of at least one amino acid residue between the amino acid sequence of the variant of the human NDST3 protein and the amino acid sequence of the wild-type human NDST3 protein. In some embodiments, there may be a difference of 1 to 200, 1 to 100, 1 to 50, 1 to 30, 1 to 20, or 1 to 10 amino acid residues between the amino acid sequence of the variant of the human NDST3 protein and the amino acid sequence of the wild-type human NDST3 protein. In some embodiments, the variant of the human NDST3 protein and the wild-type human NDST3 protein may differ by about 1, 2, 3, 4, 5, 6, 7, 8, 9, 10, 11, 12, 13, 14, 15, 16, 17, 18, 19, 20, 22, 24, 26, 28, 30, 32, 34, 36, 38, 40, 42, 44, 46, 48, 50, 55, 60, 65, 70, 75, 80, 85, 90, 95, or 100 amino acid residues. The difference in amino acid residues may include differences resulting from insertion, deletion, and substitution. For example, the variant of the human NDST3 protein may comprise substitutions (or mutations) of about 1, 2, 3, 4, 5, 6, 7, 8, 9, 10, 11, 12, 13, 14, 15, 16, 17, 18, 19, 20, 22, 24, 26, 28, 30, 32, 34, 36, 38, 40, 42, 44, 46, 48, 50, 55, 60, 65, 70, 75, 80, 85, 90, 95, or 100 amino acid residues of the wild-type human NDST3 protein with other amino acid residues. In this case, each substitution of an amino acid residue with another amino acid residue may occur independently.

For example, a variant of the human NDST3 protein may comprise an amino acid sequence having at least 80% and less than 100% sequence identity to the amino acid sequence of the wild-type human NDST3 protein (e.g., the amino acid sequence of SEQ ID NO: 1). For example, the variant of the human NDST3 protein may comprise an amino acid sequence having at least 90% and less than 100% sequence identity to the amino acid sequence of the wild-type human NDST3 protein. For example, the variant of the human NDST3 protein may comprise an amino acid sequence having at least 95% and less than 100% sequence identity to the amino acid sequence of the wild-type human NDST3 protein.

In some embodiments, one or more differences in amino acid sequences between the wild-type human NDST3 protein and the variant of the human NDST3 protein may result from conservative substitution of amino acids. For example, the variant of the human NDST3 protein may comprise one or more substitutions from the amino acid sequence of the wild-type human NDST3 protein, wherein the one or more substitutions may be conservative substitutions of amino acids. For example, the NDST3 protein may comprise an amino acid sequence having at least 80% and less than 100% sequence identity to the amino acid sequence of SEQ ID NO: 1, wherein all or some of the differences in the amino acid sequence having at least 80% and less than 100% sequence identity to the amino acid sequence of SEQ ID NO: 1 may be due to conservative substitutions of amino acids.

A conservative substitution of an amino acid refers to the replacement of a specific amino acid in a protein with another amino acid having similar properties. Examples of conservative substitutions are disclosed in Table 3, and useful conservative substitutions among them are disclosed in the "Preferred substitutions" column of Table 3. Variants of the NDST3 protein (e.g., variants of the human NDST3 protein) comprising conservative substitutions are included within the scope of the NDST3 protein disclosed in the present application. In some embodiments, a variant of the NDST3 protein comprising conservative substitutions may not substantially alter the biological activity of the wild-type NDST3 protein (e.g., wild-type human NDST3 protein), and such variants having no substantial change in biological activity are included within the scope of the NDST3 protein disclosed in the present application.

## Table 3. Examples of Conservative Substitutions

| Original residue | Exemplary Substitutions | Preferred Substitutions |
|---|---|---|
| Ala (A) | Val, Leu, Ile | Val |
| Arg (R) | Lys, Gln, Asn | Lys |
| Asn (N) | Gln, His, Lys, Arg | Gln |
| Asp (D) | Glu | Glu |
| Cys (C) | Ser | Ser |
| Gln (Q) | Asn | Asn |
| Glu (E) | Asp | Asp |
| Gly (G) | Pro, Ala | Ala |
| His (H) | Asn, Gln, Lys, Arg | Arg |
| Ile (I) | Leu, Val, Met, Ala, Phe, Norleucine | Leu |
| Leu (L) | Norleucine, Ile, Val, Met, Ala, Phe | Ile |
| Lys (K) | Arg, Gln, Asn | Arg |
| Met (M) | Leu, Phe, Ile | Leu |
| Phe (F) | Leu, Val, Ile, Ala, Tyr | Leu |
| Pro (P) | Ala | Ala |
| Ser (S) | Thr | Thr |
| Thr (T) | Ser | Ser |
| Trp (W) | Tyr, Phe | Tyr |
| Tyr (Y) | Trp, Phe, Thr, Ser | Phe |
| Val (V) | Ile, Leu, Met, Phe, Ala, Norleucine | Leu |

(see, e.g., Reference [US 8,980,821 B2, Application No. US 13/088233], the entire contents of which are incorporated herein by reference)

For example, when a variant of the human NDST3 protein comprises a substitution of DXXE relative to the wild-type human NDST3 protein (wherein DXXE indicates that the D residue at position XX is substituted with E, and XX represents a specific number), the difference between the variant of the human NDST3 protein and the wild-type human NDST3 protein may be due to a conservative substitution.

In some embodiments, a variant of the human NDST3 protein may comprise substitution of 1 to 100 amino acid residues of the wild-type human NDST3 protein with other amino acid residues, wherein the substitution of the 1 to 100 amino acid residues with other amino acid residues may be conservative substitutions. In some embodiments, the variant of the human NDST3 protein may comprise 1 to 100, 1 to 50, 1 to 30, 1 to 20, or 1 to 10 conservative substitutions of amino acid residues. For example, the variant of the human NDST3 protein may comprise 1, 2, 3, 4, 5, 6, 7, 8, 9, or 10 conservative substitutions. In some embodiments, the NDST3 protein (e.g., a variant of the human NDST3 protein) may further comprise one or more non-conservative substitutions in addition to the conservative substitutions. In some embodiments, the NDST3 protein (e.g., a variant of the human NDST3 protein) may comprise one or more unnatural amino acids. The unnatural amino acids may be introduced, for example, for conjugation of the NDST3 protein with other elements.

In some embodiments, a variant of the human NDST3 protein may be a functional equivalent of the wild-type human NDST3 protein. As used herein, the term "functional equivalent" refers to a second molecule or conceptual element that is functionally equivalent to a first molecule or conceptual element but is not necessarily structurally equivalent. For example, a functional equivalent of the NDST3 protein may refer to a protein that has substantially the same or similar function (e.g., biological activity) as the NDST3 protein but does not necessarily have the same amino acid sequence. In some embodiments, a variant of the NDST3 protein (e.g., a variant of the human NDST3 protein) may be a functional equivalent of a wild-type NDST3 protein (e.g., a wild-type human NDST3 protein). For example, a protein having an amino acid sequence having at least 80% and less than 100% sequence identity to the amino acid sequence of the wild-type human NDST3 protein may be a functional equivalent of the wild-type human NDST3 protein. In some embodiments, the functional equivalent may be generated by conservative substitution of one or more amino acids within a reference protein (e.g., a wild-type human NDST3 protein), but is not particularly limited thereto.

In some embodiments, the NDST3 protein of the present application may comprise substitution with one or more amino acid residues containing substituents; fusion or conjugation with another compound (e.g., fusion with another compound or protein to increase the stability and/or solubility of the NDST3 protein); and/or fusion or conjugation with other proteins such as serum albumin, an IgG Fc protein, an antibody, a leader sequence, a secretion sequence, a sequence facilitating purification, or a nuclear localization sequence. For example, the present application provides an antibody-NDST3 protein conjugate in which an antibody and the NDST3 protein are covalently linked (e.g., via a linker), or an albumin-NDST3 protein in which albumin (e.g., human serum albumin) and the NDST3 protein are covalently linked (e.g., via a linker). Such modifications, fusions, or conjugations may be recognized as being within the scope of those skilled in the art in view of the teachings of the present application.

The NDST3 protein described above (e.g., a wild-type human NDST3 protein or a variant thereof) or a modified form of the NDST3 protein may be produced by protein production methods known in the art (e.g., recombinant protein production methods). Methods for protein expression and purification are well known. For example, those skilled in the art may refer to Green, Michael R., and Joseph Sambrook, Molecular Cloning: A Laboratory Manual, 4th ed. (2012), for methods of protein production, synthesis, or expression, the entire contents of which are incorporated herein by reference.

**Nucleic Acid Encoding NDST3 Protein**

Overview of Nucleic Acid Encoding NDST3 Protein

In some embodiments, the composition for treating Parkinson's disease may comprise a nucleic acid encoding an NDST3 protein. In some embodiments, the composition for treating Parkinson's disease may comprise, as an active substance, a nucleic acid encoding an NDST3 protein. In some embodiments, the nucleic acid encoding the NDST3 protein may be referred to as a nucleic acid for expression of the NDST3 protein. In some embodiments, the nucleic acid encoding the NDST3 protein may be referred to as a nucleic acid comprising a region encoding the NDST3 protein. In some embodiments, the nucleic acid encoding the NDST3 protein may be referred to by various names including, but not limited to, a nucleic acid, a nucleic acid construct, a nucleic acid for expression of NDST3 (protein), an expression nucleic acid, an expression construct, or a nucleic acid for delivery of NDST3 (protein).

For example, the nucleic acid encoding the NDST3 protein may be used to express the NDST3 protein at a specific site (e.g., a cell or the nucleus of a cell). For example, the nucleic acid encoding the NDST3 protein may be used to express the NDST3 protein at a desired site (e.g., a desired cell or a host cell), but is not limited thereto. In some embodiments, the desired cell (or target cell) may be a central nervous system cell. In some embodiments, the nucleic acid encoding the NDST3 protein may be used to express the NDST3 protein in a cell (e.g., a central nervous system cell of a subject), but is not limited thereto. In some embodiments, the central nervous system cell may be a central nervous system neuron, an astrocyte, or a glial cell. In some embodiments, the cell may be a neuron, astrocyte, or glial cell that is damaged or has lost its function. For example, the central nervous system cell may be a cell of the substantia nigra. For example, the neuron may be selected from one or more of a dopaminergic neuron (or a dopaminergic neuron that is damaged or has lost its function), a cholinergic neuron, an adrenergic neuron, a GABAergic neuron, a glutamatergic neuron, a serotonergic neuron, a purinergic neuron, and a histaminergic neuron, but is not limited thereto.

In some embodiments, the nucleic acid encoding the NDST3 protein may have a nucleic acid sequence encoding the NDST3 protein. In some embodiments, the nucleic acid encoding the NDST3 protein may comprise a region encoding the NDST3 protein (e.g., a coding region). In this case, the region encoding the NDST3 protein may have a nucleic acid sequence encoding the NDST3 protein. In some embodiments, the nucleic acid encoding the NDST3 protein may comprise a nucleic acid sequence encoding the NDST3 protein.

The NDST3 protein is described in detail in the preceding description, including the section titled "NDST3 Protein" of the present application. For example, the NDST3 protein may be a human NDST3 protein. For example, the NDST3 protein may be a wild-type human NDST3 protein or a variant thereof. For example, the NDST3 protein may have the amino acid sequence of SEQ ID NO: 1 or an amino acid sequence having 80% or more and less than 100% sequence identity thereto.

In some embodiments, the nucleic acid encoding the NDST3 protein may be a DNA molecule, an RNA molecule, or a DNA/RNA hybrid molecule, or a portion of a DNA molecule (e.g., a partial region), a portion of an RNA molecule (e.g., a partial region), or a portion of a DNA/RNA hybrid molecule (e.g., a partial region). In some embodiments, the nucleic acid encoding the NDST3 protein may be in the form of a single strand (e.g., single-stranded DNA) or a double strand (e.g., double-stranded DNA). In some embodiments, the nucleic acid encoding the NDST3 protein may be a nucleic acid construct or a portion of a nucleic acid construct. For example, the nucleic acid encoding the NDST3 protein may be a DNA construct, an RNA construct, or a DNA/RNA hybrid construct, or a portion of a DNA construct (e.g., a partial region), a portion of an RNA construct (e.g., a partial region), or a portion of a DNA/RNA hybrid construct (e.g., a partial region).

In some embodiments, the nucleic acid encoding the NDST3 protein may be a recombinant nucleic acid.

In some embodiments, the length of the nucleic acid encoding the NDST3 protein may be about 500 nt (or 500 bases), 1,000 nt, 2,000 nt, 3,000 nt, 4,000 nt, 5,000 nt, 6,000 nt, 7,000 nt, 8,000 nt, 9,000 nt, 10,000 nt, 20,000 nt, 30,000 nt, 40,000 nt, 50,000 nt, 60,000 nt, 70,000 nt, 80,000 nt, 90,000 nt, 100,000 nt, 500,000 nt, 1,000,000 nt, or 2,000,000 nt, or may fall within a range defined by two values selected from the foregoing values, but is not limited thereto. For example, the length of the nucleic acid encoding the NDST3 protein may be 1,000 nt to 10,000 nt, 2,000 nt to 6,000 nt, or 2,000 nt to 5,000 nt.

Nucleic Acid Sequence Encoding the NDST3 Protein

As described above, the nucleic acid encoding the NDST3 protein may comprise a nucleic acid sequence encoding the NDST3 protein. In some embodiments, the nucleic acid encoding the NDST3 protein may comprise a coding region (e.g., an NDST3 protein coding region), and the coding region may have a nucleic acid sequence encoding the NDST3 protein.

In some embodiments, the nucleic acid sequence encoding the NDST3 protein may encompass nucleic acid sequences of a wild-type or a variant. In some embodiments, information on the nucleic acid sequence encoding a wild-type NDST3 protein may be obtained from a known database.

In some embodiments, the nucleic acid sequence encoding the NDST3 protein may comprise the full-length sequence of the NDST3 gene (e.g., the full-length sequence of the human NDST3 gene). In some embodiments, the nucleic acid sequence encoding the NDST3 protein may be the full-length sequence of the NDST3 gene comprising introns and exons. In some embodiments, the sequence encoding the NDST3 protein may comprise the full-length sequence or a portion thereof of the NDST3 gene selected from a human NDST3 gene, a canine NDST3 gene, an equine NDST3 gene, a feline NDST3 gene, a camel NDST3 gene, a mouse NDST3 gene, a rat NDST3 gene, a porcine NDST3 gene, a rabbit NDST3 gene, a sheep NDST3 gene, a monkey NDST3 gene, a chimpanzee NDST3 gene, or a bovine NDST3 gene.

In some embodiments, the nucleic acid sequence encoding the NDST3 protein may comprise one or more introns and one or more exons of the NDST3 gene (e.g., the human NDST3 gene). In some embodiments, the nucleic acid sequence encoding the NDST3 protein may comprise one or more exons of the NDST3 gene but may not comprise introns of the NDST3 gene. In some embodiments, the nucleic acid sequence encoding the NDST3 protein may comprise only exons of the NDST3 gene. For example, the human NDST3 gene is known to have 14 exons. In some embodiments, the nucleic acid sequence encoding the NDST3 protein may comprise all exons of the human NDST3 gene. In some embodiments, the nucleic acid sequence encoding the NDST3 protein may comprise one or more selected from exon 1, exon 2, exon 3, exon 4, exon 5, exon 6, exon 7, exon 8, exon 9, exon 10, exon 11, exon 12, exon 13, and exon 14 of the human NDST3 gene.

In some embodiments, the nucleic acid sequence encoding the NDST3 protein may comprise a cDNA sequence of the NDST3 gene (or NDST3) or a sequence complementary thereto. In some embodiments, the sequence encoding the NDST3 protein may comprise a cDNA sequence of a human NDST3 gene, a canine NDST3 gene, an equine NDST3 gene, a feline NDST3 gene, a camel NDST3 gene, a mouse NDST3 gene, a rat NDST3 gene, a porcine NDST3 gene, a rabbit NDST3 gene, a sheep NDST3 gene, a monkey NDST3 gene, a chimpanzee NDST3 gene, or a bovine NDST3 gene, or a sequence complementary thereto. In some embodiments, the nucleic acid sequence encoding the NDST3 protein may comprise the cDNA sequence of the human NDST3 gene.

In some embodiments, the nucleic acid sequence encoding the NDST3 protein may comprise a CDS (coding sequence) of the NDST3 gene (or NDST3) or a sequence complementary thereto. In some embodiments, the sequence encoding the NDST3 protein may comprise a CDS of a human NDST3 gene, a canine NDST3 gene, an equine NDST3 gene, a feline NDST3 gene, a camel NDST3 gene, a mouse NDST3 gene, a rat NDST3 gene, a porcine NDST3 gene, a rabbit NDST3 gene, a sheep NDST3 gene, a monkey NDST3 gene, a chimpanzee NDST3 gene, or a bovine NDST3 gene, or a sequence complementary thereto. In some embodiments, the nucleic acid sequence encoding the NDST3 protein may comprise the CDS of the human NDST3 gene.

In some embodiments, the nucleic acid sequence encoding the NDST3 protein may comprise an ORF (open reading frame) sequence of the NDST3 gene. In some embodiments, the sequence encoding the NDST3 protein may comprise an ORF sequence of a human NDST3 gene, a canine NDST3 gene, an equine NDST3 gene, a feline NDST3 gene, a camel NDST3 gene, a mouse NDST3 gene, a rat NDST3 gene, a porcine NDST3 gene, a rabbit NDST3 gene, a sheep NDST3 gene, a monkey NDST3 gene, a chimpanzee NDST3 gene, or a bovine NDST3 gene. In some embodiments, the nucleic acid sequence encoding the NDST3 protein may comprise the ORF sequence of the human NDST3 gene.

In some embodiments, the nucleic acid sequence encoding the NDST3 protein may comprise the nucleic acid sequence of SEQ ID NO: 7, a nucleic acid sequence having 70% or more and less than 100% (e.g., 99.9% or less) sequence identity thereto, or a sequence complementary thereto.

[SEQ ID NO: 7]

atgagttttatcatgaagcttcacagacactttcaaagaacagtcattctgcttgccactttttgt
atggtgagcattattatttctgcttactacctgtacagtggctacaaacaggaaaatgaactctct
gagacggcttcagaagttgactgtggcgacctccaacacctaccatatcaactaatggaagtgaaa
gcaatgaagcttttgatgcctcaaggacagaccccacagtcctagtatttgtagagagccagtac
tcatctcttggtcaagacatcattatgattctagaatcaagtagattccagtatcacattgaaatt
gcccctggaaagggagatctcccagtgcttatagacaaaatgaaaggcaaatacattctcattatt
tatgagaatattttaaagtatataaatatggattcctggaatcgaagccttctagataaatactgt
gtagaatatggtgtgggtgtcattggattccacaaaactagtgagaagagtgtacagagctttcag
ttaaaaggtttccctttttccatatatggaaatcttgcagtaaaagattgttgtattaatcctcat
tctccattgattcgtgtgaccaaatcttccaagcttgaaaaaggttctttacctggaactgactgg
acagtttttcagattaatcattcagcctatcaaccagtaatatttgccaaagtaaagaccccagaa
aaccttctccttccatctctaaaggtgctttttatgccactattatacatgacctggggcttcat
gatggaattcaaaggggttcttttttggcaacaacttgaacttttggctgcacaagctcatcttcata
gatgccatctccttcttatcagggaagaggctgacattgtccttggacaggtacattcttgtggat
attgatgatatatttgtgggaaaagagggaacaagaatgaacaccaatgatgtaaaggccctgctt
gatactcagaatcttttgcgtgcacaaatcacaaattttacattcaacctgggattttcagggaaa
ttttaccatacaggaactgaagaggaagatgaaggagatgactgtctgttggggtctgtggatgag
ttctggtggtttcctcacatgtggagccatatgcagccccacctcttccacaatgagtcatctttg
gtggagcagatgattctcaacaaaaaattgccttagagcacggcattccaacggacatgggctac
gctgtggcccctcaccattcgggcgtctaccctgtacatgttcagctttatgaggcctggaagaag
gtctggaatattaaaataccagcactgaagaatatccacatctgaagccagctagataccggagg
ggttttatccacaaaaacatcatggttctcccaagacaaacctgtgggcttttcactcacaccatt
ttctacaagaatatccaggggggtcctaaagagctggataagagtatccaaggaggagaacttttc
ttcactgtcgtcctcaaccctatcagcattttcatgacccatttgtccaactatgggaatgaccga
ctgggattatatacatttgttaatctggccaactttgtgaagagctggaccaacctgcgacttcag
actctgcctccagtacaactggcccacaagtattttgagctgtttcctgatcagaaagaccctctc
tggcagaatccttgcgatgacaaacgccacagagacatttggtctaaagaaaaaacttgtgatcgc
ttaccaaaattcttggtaataggaccccagaaaactggtaccactgcttttgtatttgttcctggtt
atgcatccttccatccttagtaactcccccagcccaaaaaacctttgaggaggtacagttctttaat
agaaataactaccacaggggggattgattggtatatggatttcttcccagtcccatctaatgtcact
accgacttttttgtttgagaagagtgccaattacttccactcagaggaagcccctaaaagagctgct
tctctggttcccaaagccaagattatcaccattctcattgacccttcagaccgagcatactcctgg
taccagcatcagcgatcacatgaagaccctgcagctctgaagtttagcttctacgaagtgatctca

gcagggccccgtgcaccctcggagctcagagccttgcagaagagatgtttggtcccggggtggtat
gccagccacatcgagagatggcttgtttatttccccccatttcagttgctaattattgatgggcaa
caactaagaactgatcctgctacagtgatggatgaagtacagaagttctaggagtcttgcctcat
tataattactcagaagctttaacgtttgattctcataaaggtttctggtgtcagttactggaagaa
ggtaaaacaaaatgccttggaaagagcaaaggaagaaaataccctccaatggattctgatagcagg
acatttctgtcaagctactatcgagatcacaacgtggaactctcaaagctgctgcacaaactgggt
cagcctctgccatcctggctgagacaggagctgcagaaagtaaga<u>tag</u>

The sequence of SEQ ID NO: 7 may be the CDS sequence of the human NDST3 gene. The sequence of SEQ ID NO: 7 encodes the wild-type human NDST3 protein. In SEQ ID NO: 7, the underlined notation of the terminal tag indicates that the tag is a stop codon.

In some embodiments, the nucleic acid sequence encoding the NDST3 protein (e.g., a nucleic acid sequence encoding the human NDST3 protein) may comprise the nucleic acid sequence of SEQ ID NO: 7 or a nucleic acid sequence having 70% or more and less than 100% sequence identity thereto (e.g., 72% or more and less than 100%, 74% or more and less than 100%, 76% or more and less than 100%, 78% or more and less than 100%, 80% or more and less than 100%, 82% or more and less than 100%, 84% or more and less than 100%, 86% or more and less than 100%, 88% or more and less than 100%, 90% or more and less than 100%, 92% or more and less than 100%, 94% or more and less than 100%, 95% or more and less than 100%, 96% or more and less than 100%, 97% or more and less than 100%, 97.5% or more and less than 100%, 98% or more and less than 100%, 98.5% or more and less than 100%, 99% or more and less than 100%, or 99.5% or more and less than 100%). In certain embodiments, the nucleic acid sequence encoding the human NDST3 protein may comprise the nucleic acid sequence of SEQ ID NO: 7 or a nucleic acid sequence having 90% or more and less than 100% sequence identity thereto, 92% or more and less than 100% sequence identity thereto, 94% or more and less than 100% sequence identity thereto, 95% or more and less than 100% sequence identity thereto, 96% or more and less than 100% sequence identity thereto, 97% or more and less than 100% sequence identity thereto, 97.5% or more and less than 100% sequence identity thereto, 98% or more and less than 100% sequence identity thereto, 98.5% or more and less than 100% sequence identity thereto, 99% or more and less than 100% sequence identity thereto, or 99.5% or more and less than 100% sequence identity thereto. In certain embodiments, the nucleic acid sequence encoding the human NDST3 protein may comprise the nucleic acid sequence of SEQ ID NO: 7 or a nucleic acid sequence having 95% or more and less than 100% sequence identity thereto, 96% or more and less than 100% sequence identity thereto, 97% or more and less than 100% sequence identity thereto, 97.5% or more and less than 100% sequence identity thereto, 98% or more and less than 100% sequence identity thereto, 98.5% or more and less than 100% sequence identity thereto, 99% or more and less than 100% sequence identity thereto, or 99.5% or more and less than 100% sequence identity thereto. In some embodiments, the nucleic acid sequence encoding the human NDST3 protein may comprise the nucleic acid sequence of SEQ ID NO: 7 or a nucleic acid sequence having about 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 97.2%, 97.4%, 97.6%, 97.8%, 98%, 98.2%, 98.4%, 98.6%, 98.8%, 99%, 99.1%, 99.2%, 99.3%, 99.4%, 99.5%, 99.6%, 99.7%, 99.8%, or 99.9% sequence identity thereto. In certain embodiments, the nucleic acid sequence encoding the human NDST3 protein may comprise the nucleic acid sequence of SEQ ID NO: 7 or a nucleic acid sequence having about 90%, 91%, 92%, 93%, 94%, 95%, 96%, 97%, 97.2%, 97.4%, 97.6%, 97.8%, 98%, 98.2%, 98.4%, 98.6%, 98.8%, 99%, 99.1%, 99.2%, 99.3%, 99.4%, 99.5%, 99.6%, 99.7%, 99.8%, or 99.9% sequence identity thereto. In certain embodiments, the nucleic acid sequence encoding the human NDST3 protein may comprise the nucleic acid sequence of SEQ ID NO: 7 or a nucleic acid sequence having about 95%, 96%, 97%, 97.2%, 97.4%, 97.6%, 97.8%, 98%, 98.2%, 98.4%, 98.6%, 98.8%, 99%, 99.1%, 99.2%, 99.3%, 99.4%, 99.5%, 99.6%, 99.7%, 99.8%, or 99.9% sequence identity thereto. In certain embodiments, the nucleic acid sequence encoding the human NDST3 protein may be the nucleic acid sequence of SEQ ID NO: 7.

In some embodiments, the difference between the nucleic acid sequence of SEQ ID NO: 7 and a nucleic acid sequence having 70% or more and less than 100% sequence identity to the nucleic acid sequence of SEQ ID NO: 7, for example all differences or one or more differences, may be due to codon degeneracy.

In some embodiments, the length of the nucleic acid sequence encoding the NDST3 protein may be about 500 nt or 500 bases, 1,000 nt, 2,000 nt, 3,000 nt, 4,000 nt, 5,000 nt, 6,000 nt, 7,000 nt, 8,000 nt, 9,000 nt, 10,000 nt, 20,000 nt, 30,000 nt, 40,000 nt, 50,000 nt, 60,000 nt, 70,000 nt, 80,000 nt, 90,000 nt, 100,000 nt, 500,000 nt, or 1,000,000 nt, or may fall within a range defined by two values selected from the foregoing values, but is not limited thereto. For example, the length of the nucleic acid sequence encoding the NDST3 protein may be 500 nt to 10,000 nt, 1,000 nt to 6,000 nt, or 2,000 nt to 3,000 nt.

Optional Additional Elements Included in the Nucleic Acid Encoding the NDST3 Protein

As described above, the nucleic acid encoding the NDST3 protein may comprise a nucleic acid sequence encoding the NDST3 protein. For example, the nucleic acid encoding the NDST3 protein may comprise a coding region, and the coding region may have a nucleic acid sequence encoding the NDST3 protein.

In some embodiments, the nucleic acid encoding the NDST3 protein may further comprise one or more additional elements in addition to the coding region. In some embodiments, the additional elements may independently be selected from a promoter, an enhancer, a polyadenylation signal, a Kozak consensus sequence, an inverted terminal repeat (ITR), a long terminal repeat (LTR), a terminator, an origin of replication, a multicloning site (MCS), an internal ribosome entry site (IRES), and a 2A self-cleaving peptide. In some embodiments, the additional element may be a control element or a control sequence. As used herein, the term control element may refer to an element, region, or DNA sequence operably linked to a coding region, for example a nucleic acid sequence encoding the NDST3 protein, that enables expression of the coding region, for example expression of the NDST3 protein, in a desired organism or host organism. Examples of control elements in prokaryotic cells may include a promoter, an operator sequence, and a ribosome binding site. Examples of control elements in eukaryotic cells may include a promoter, a polyadenylation sequence, and an enhancer.

In some embodiments, the nucleic acid encoding the NDST3 protein may further comprise a promoter. In some embodiments, the promoter may be an SV40 early promoter, an LTR promoter from mouse mammary tumor virus long terminal repeat, an Ad MLP promoter from adenovirus major late, an HSV promoter from herpes simplex virus, a CMV promoter from cytomegalovirus, an RSV promoter from Rous sarcoma virus, a U6 promoter, a CBA promoter, a PGK promoter, an NG2 promoter, an NES promoter, a GFAP promoter, a CaMKII promoter, an NSE promoter, a SYN1 promoter, or a CAG promoter. In some embodiments, the promoter may be a promoter derived from human, mouse, canine, equine, feline, rat, porcine, rabbit, sheep, monkey, chimpanzee, bovine, or virus, but is not limited thereto.

In some embodiments, one or more additional elements may be operably linked to the coding region. For example, one or more of a promoter and an enhancer may be operably linked to the coding region. For example, in the nucleic acid encoding the NDST3 protein, the promoter may be operably linked to the coding region, for example a nucleic acid sequence encoding the NDST3 protein. An element or nucleic acid operably linked may be connected to the coding region directly or through a linker, for example an oligonucleotide linker. The promoter and/or enhancer may be operably linked to the coding region and may influence transcription of the coding region. In some embodiments, the promoter may be linked to the 5' end or the 3' end of the coding region, for example a nucleic acid sequence encoding the NDST3 protein. Preferably, the promoter may be linked to the 5' end of the coding region, and the promoter may be operably linked to the coding region.

Methods for designing and/or producing nucleic acids, for example nucleic acids having a nucleic acid sequence encoding the NDST3 protein, are well known to those skilled in the art. For example, those skilled in the art may refer to Green, Michael R., and Joseph Sambrook, Molecular Cloning: A Laboratory Manual, 4th ed. (2012), for the design and/or production of nucleic acids, for example recombinant nucleic acids, the entire contents of which are incorporated herein by reference.

The NDST3 protein described above or the nucleic acid encoding the same may be included in or attached to a vehicle for delivery to, or use in, a desired site, organ, tissue, or organism, for example a cell. For example, the NDST3 protein or the nucleic acid encoding the same may be attached to or included in a vehicle and provided for delivery to, or use in, a desired site, organ, tissue, or cell. For example, the nucleic acid encoding the NDST3 protein may be provided in the form of a vector, or provided as included in a vector, for delivery to, or use in, a desired site, organ, tissue, or cell. The vehicle and vector are described in detail below.

**Vehicle**

In some embodiments, the NDST3 protein or the nucleic acid encoding the same may be attached to or included in a vehicle for delivery, for example a drug delivery vehicle or drug carrier.

In some embodiments, the NDST3 protein or the nucleic acid encoding the same may be attached to or included in a vehicle for delivery to, or use in, a desired site, organ, tissue, or cell. As used herein, attachment includes both covalent attachment through a covalent bond and non-covalent attachment through a non-covalent bond. As used herein, the term vehicle may refer to an element used for transport and/or delivery of an element or substance intended for delivery or use. For example, the vehicle may be referred to as cargo.

In some embodiments, the composition for treating Parkinson's disease may comprise a vehicle comprising the NDST3 protein or a nucleic acid encoding the NDST3 protein. In some embodiments, the composition for treating Parkinson's disease may comprise, as an active substance, a vehicle comprising the NDST3 protein or a nucleic acid encoding the NDST3 protein.

In some embodiments, the vehicle may be a nanoparticle, for example a silica nanoparticle, a metal nanoparticle such as a gold nanoparticle or a silver nanoparticle, or a protein nanoparticle such as an albumin nanoparticle, a liposome, a lipid nanoparticle, a carbon nanotube, chitosan, a protein such as an antibody or albumin, a peptide, DNA, a DNA-RNA lipid complex, a polymer, a DNA-polymer complex, an artificial chromosome, a cosmid, or a vector such as a viral vector, but is not limited thereto. For example, the NDST3 protein or the nucleic acid encoding the same may be attached to or included in the vehicle. In some embodiments, the vehicle may be a vehicle for targeted drug delivery. That is, the vehicle may be a vehicle for active targeting. In some embodiments, the vehicle may be a vehicle for passive targeting. Vehicles for delivery of proteins or peptides are described in detail in Jain, A., Jain, A., Gulbake, A., Shilpi, S., Hurkat, P., and Jain, S. K., "Peptide and protein delivery using new drug delivery systems," Critical Reviews in Therapeutic Drug Carrier Systems, 30(4) (2013), the entire contents of which are incorporated herein by reference.

Hereinafter, vectors used for transport, loading, and/or delivery of the nucleic acid encoding the NDST3 protein will be described in detail.

**Vector**

Overview of Vector

As used herein, the term vector refers to a vehicle, particle, or element used for transport, loading, carrying, and/or delivery of a nucleic acid, for example an exogenous nucleic acid or exogenous gene. The vector may comprise a nucleic acid, and the nucleic acid comprised in the vector may be a recombinant nucleic acid. For example, the vector may comprise exogenous DNA and recombinant DNA. The vector may be used for transport or delivery of the nucleic acid comprised therein to a desired site, organ, tissue, or cell. The vector may be designed, for example, for expression of the exogenous nucleic acid comprised in the vector in another cell. In such a case, the exogenous nucleic acid introduced into the other cell through the vector, for example an exogenous gene, may be transcribed through an endogenous promoter of the other cell or through an exogenous promoter introduced together with the exogenous nucleic acid.

In some embodiments, the nucleic acid encoding the NDST3 protein may be included in a vector. In some embodiments, the nucleic acid encoding the NDST3 protein may be provided in the form of a vector. In some embodiments, the nucleic acid encoding the NDST3 protein may be packaged in a vector.

In some embodiments, the present application provides a vector comprising a nucleic acid encoding the NDST3 protein. The vector comprising the nucleic acid encoding the NDST3 protein may be used for treatment of Parkinson's disease.

In some embodiments, the composition for treating Parkinson's disease of the present application may comprise a vector comprising a nucleic acid encoding the NDST3 protein. In some embodiments, the composition for treating Parkinson's disease of the present application may comprise, as an active substance, a vector comprising a nucleic acid encoding the NDST3 protein. In this case, the vector comprising the nucleic acid encoding the NDST3 protein may be referred to as a vector encoding the NDST3 protein. The vector comprising the nucleic acid encoding the NDST3 protein may also be referred to as an NDST3 protein or gene delivery vector, an NDST3 protein or gene expression vector, an expression vector, a vector for treatment of Parkinson's disease, or by other names, and is not otherwise limited.

Vectors are generally classified into viral vectors and non-viral vectors. The viral vectors are described in detail below.

Viral Vector

A viral vector is a tool commonly and usefully employed in the field of molecular biology for transport or delivery of exogenous nucleic acids, for example exogenous genes. Viral vectors are described in detail in Bulcha, J. T., Wang, Y., Ma, H., Tai, P. W., and Gao, G., "Viral vector platforms within the gene therapy landscape," Signal Transduction and Targeted Therapy, 6(1), 53 (2021); and Lundstrom, K., "Viral vectors in gene therapy," Diseases, 6(2), 42 (2018), the entire contents of each of which are incorporated herein by reference.

In some embodiments, the vector may be a viral vector. In some embodiments, the nucleic acid encoding the NDST3 protein may be included in a viral vector. In some embodiments, a viral vector comprising a nucleic acid encoding the NDST3 protein, for example a viral vector encoding the NDST3 protein, may be provided. In some embodiments, a composition comprising a viral vector comprising a nucleic acid encoding the NDST3 protein, for example a composition for treatment of Parkinson's disease, may be provided. The viral vector may be in a recombinant form, but is not limited thereto.

In some embodiments, the viral vector may be derived from one or more of a retrovirus, lentivirus, adenovirus, adeno-associated virus, vaccinia virus, poxvirus, HIV (human immunodeficiency virus), MLV (murine leukemia virus), ASLV (avian sarcoma or leukosis virus), SNV (spleen necrosis virus), RSV (Rous sarcoma virus), MMTV (mouse mammary tumor virus), herpes simplex virus, episomal virus, or herpes virus.

In some embodiments, the viral vector may be selected from one or more of a retroviral vector, a lentiviral vector, an adenoviral vector, an adeno-associated viral vector, a vaccinia viral vector, a poxviral vector, an HIV (human immunodeficiency virus) vector, an MLV (murine leukemia virus) vector, an ASLV (avian sarcoma or leukosis virus) vector, an SNV (spleen necrosis virus) vector, an RSV (Rous sarcoma virus) vector, an MMTV (mouse mammary tumor virus) vector, a herpes simplex virus vector, an episomal vector, a herpes virus vector, and a hybrid vector such as an adeno or AAV hybrid vector.

In some embodiments, the nucleic acid encoding the NDST3 protein, or the NDST3 protein coding region, may be integrated into or inserted into the genome of a cell. In some embodiments, the nucleic acid encoding the NDST3 protein may be integrated into or inserted into a desired location of the cellular genome, for example a target site. For example, the nucleic acid encoding the NDST3 protein may be integrated into or inserted into the AAVS1 gene located on human chromosome 19.

In some embodiments, the vector or vector system may be designed to insert or integrate the nucleic acid encoding the NDST3 protein into a specific site, for example into the AAVS1 gene. Such a vector or vector system may be referred to as a vector or vector system for insertion into AAVS1.

In some embodiments, insertion into AAVS1 may use Rep, a replicase or integrase protein. For example, in addition to a vector encoding the NDST3 protein, a vector encoding Rep, for example an AAV vector encoding Rep, may be used. In another example, Rep may be used in addition to the vector encoding the NDST3 protein. In another example, a vector comprising a nucleic acid encoding Rep may be used in addition to the nucleic acid encoding the NDST3 protein. In such a case, the nucleic acid encoding Rep may be included in a single nucleic acid construct together with the nucleic acid sequence encoding the NDST3 protein, or may be included in a nucleic acid construct separate from the nucleic acid encoding the NDST3 protein. Rep used for insertion or integration into the AAVS1 gene may be Rep78 and/or Rep68, but is not limited thereto. Rep78 and Rep68 are known to induce insertion or integration into AAVS1 together with inverted terminal repeats. In some embodiments, a vector for insertion into AAVS1 may be an AAV vector or a hybrid vector such as an adeno or AAV hybrid vector, but is not limited thereto.

In some embodiments, the viral vector may be an adeno-associated virus AAV vector. The AAV vector is described in detail below.

AAV Vector

In some embodiments, the viral vector may be an adeno-associated virus AAV vector. The use or application of AAV vectors is well known in the art, and a detailed description of AAV vectors may be found in Asokan, A., Schaffer, D. V., and Samulski, R. J., "The AAV vector toolkit: poised at the clinical crossroads," Molecular Therapy, 20(4), 699-708 (2012); and Li, C., and Samulski, R. J., "Engineering adeno-associated virus vectors for gene therapy," Nature Reviews Genetics, 21(4), 255-272 (2020), the entire contents of which are incorporated herein by reference. Examples of well-known serotypes of AAV vectors include AAV1, AAV2, AAV3, AAV4, AAV5, AAV6, AAV7, AAV8, AAV9, AAV10, AAV11, and AAV12.

In some embodiments, the AAV vector, for example a recombinant AAV vector, may be natural in origin, engineered, chimeric, or a variant.

In some embodiments, the AAV vector may be one selected from AAV1, AAV2, AAV3, AAV4, AAV5, AAV6, AAV7, AAV8, AAV9, AAV10, AAV11, AAV12, AAV13, AAV-DJ, AAV-DJ/8, AAV-Rh10, AAV-retro, AAV-PHP.B, AAV-PHP.eB, and AAV-PHP.S vectors.

In some embodiments, the AAV vector may be an AAV9 vector.

In some embodiments, an AAV vector comprising a nucleic acid encoding the NDST3 protein may be provided. For example, the nucleic acid encoding the NDST3 protein may be referred to as a transgene cassette, but is not limited thereto. For example, the AAV vector comprising the nucleic acid encoding the NDST3 protein may be referred to as an AAV cassette, but is not limited thereto.

In some embodiments, the AAV vector, for example an AAV9 vector, may comprise a capsid and a nucleic acid encoding the NDST3 protein. Structurally, the capsid may surround and package the nucleic acid encoding the NDST3 protein.

The nucleic acid encoding the NDST3 protein is described in detail in the preceding description, including the section titled "Nucleic Acid Encoding NDST3 Protein" of the present application. In some embodiments, the nucleic acid encoding the NDST3 protein may comprise a coding region having a nucleic acid sequence encoding the NDST3 protein. In some embodiments, the nucleic acid encoding the NDST3 protein may further comprise one or more additional elements. In some embodiments, one of the one or more additional elements may be a promoter. In some embodiments, the nucleic acid encoding the NDST3 protein may comprise a coding region and a promoter. In such a case, the promoter may be operably linked to the coding region. The promoter may be operably linked to the 5' end or the 3' end of the coding region. In some embodiments, the nucleic acid encoding the NDST3 protein may further comprise one or more inverted terminal repeats, for example one or two inverted terminal repeats. For example, the nucleic acid encoding the NDST3 protein may comprise a coding region having a nucleic acid sequence encoding the NDST3 protein. For example, the nucleic acid encoding the NDST3 protein may comprise a coding region and a promoter. For example, the nucleic acid encoding the NDST3 protein may comprise a coding region, a promoter, and one or more inverted terminal repeats. For example, the nucleic acid encoding the NDST3 protein may comprise a coding region and one or more inverted terminal repeats.

For example, a viral vector comprising a nucleic acid encoding the NDST3 protein may be produced by methods well known in the art. For example, a researcher may purchase materials or kits for producing viral vectors from a supplier of viral vectors and may produce a viral vector comprising a nucleic acid encoding the NDST3 protein according to the manufacturer's protocol.

Hereinafter, examples of methods for producing an AAV vector are described, but the scope of the present application is not limited thereto.

For example, an AAV vector may be produced by methods known in the art. Methods for producing an AAV vector may generally be performed using two or three or more plasmids.

By way of example, a method for producing an AAV vector may comprise introducing into a host cell a first plasmid comprising a sequence intended for delivery or expression, for example a nucleic acid sequence encoding the NDST3 protein, wherein the first plasmid may be referred to as a transfer plasmid and may further comprise an inverted terminal repeat and a promoter in addition to the sequence intended for delivery or expression; a second plasmid comprising a nucleic acid sequence encoding a replication protein and/or a capsid protein, wherein the second plasmid may be referred to as a Rep-Cap plasmid; and a third plasmid comprising a sequence encoding a helper gene, wherein the third plasmid may be referred to as a helper plasmid; and extracting AAV particles, for example an AAV vector, from the host cell.

In this case, the AAV particle may be an AAV2 particle or an AAV9 particle.

Here, the host cell into which the three types of plasmids or two types of plasmids, for example the transfer plasmid and the Rep-Cap plasmid, are introduced may be, for example, HEK293, HEK293T, Huh-7, HeLa, HepG2, Hep1A, SV40, CHO, COS, MeWo, NIH3T3, A549, PERC6, HT1180, a 293 AAV cell, a monocyte, or a dendritic cell.

A researcher may introduce the above-described three types or two types of plasmids into a host cell and then culture the host cell by an appropriate method, for example according to a protocol provided by the manufacturer of the host cell, in order to obtain an AAV vector from the host cell. After culture, an AAV vector comprising the desired sequence may be obtained from the host cell.

Introduction of the plasmids into the host cell may be performed by appropriately selecting a method known in the art.

For example, electroporation, gene gun, sonoporation, magnetofection, microinjection, transient cell compression or squeezing, cationic liposome methods, lithium acetate-DMSO methods, lipid-mediated transfection, calcium phosphate precipitation, lipofection, polyethyleneimine-mediated transfection, and DEAE-dextran-mediated transfection may be used, but are not limited thereto.

In this case, the order in which the plasmids are introduced into the host cell is not particularly limited.

The AAV particle may be prepared in the form of an exosome or microparticle, but is not limited thereto. Methods for extracting AAV particles may be performed using methods known in the art.

For example, methods for extracting AAV particles may include centrifugation, precipitation, immunoprecipitation, affinity chromatography, filtration, use of magnetic beads coated with a specific antibody or aptamer, freeze-thaw methods, sonication, or use of a commercial kit.

The AAV particles may optionally be concentrated by methods known in the art. For example, the concentration may be performed using immunomagnetic capture, organic flocculation, polyethylene glycol precipitation, or similar techniques, but is not limited thereto.

Optionally, quality testing may further be performed. For example, the quality testing may include titer measurement, sterility testing for bacteria and fungi, and mycoplasma detection testing, but is not limited thereto.

The produced AAV vector comprises a capsid and AAV vector nucleic acid, wherein the AAV vector nucleic acid is known to be located within the capsid. The AAV vector nucleic acid comprises a sequence of interest. The AAV vector nucleic acid optionally further comprises a promoter and/or ITR.

Methods for producing a viral vector (for example, an AAV vector) for delivery or administration of a designed sequence are well known in the art. For example, a researcher may purchase host cells for producing a viral vector such as an AAV vector from a host cell supplier and produce or obtain an AAV vector comprising the designed sequence according to the supplier's protocol. In another example, a researcher may purchase a kit for producing an AAV vector from a kit supplier and produce or obtain an AAV vector comprising the designed sequence according to the supplier's protocol. In yet another example, a researcher may design a sequence to be delivered or administered to a subject and produce or obtain a viral vector (for example, an AAV vector) comprising the designed sequence by referring to the literature [Green, Michael R., and Joseph Sambrook. "Molecular Cloning: A Laboratory Manual," 4th ed. (2012)], the entire contents of which are incorporated herein by reference.

Non-viral Vector

In some embodiments, the vector may be a non-viral vector. In some embodiments, the nucleic acid encoding the NDST3 protein may be included in or packaged within a non-viral vector.

In some embodiments, the non-viral vector may be selected from the group consisting of a plasmid, phage, naked DNA, a DNA-RNA lipid complex, a liposome, a nanoparticle, a DNA-polymer complex, mRNA, an artificial chromosome, and a cosmid.

**Additional Elements That May Be Included in the Composition**

In some embodiments, the composition (for example, a composition for treating Parkinson's disease) may further comprise one or more additional elements in addition to the active ingredient.

In some embodiments, the additional element may be a pharmaceutically acceptable additional element. As used herein, the term "pharmaceutically acceptable" may refer to materials, compositions, and/or dosage forms that are suitable for use in contact with the tissues of humans and animals without excessive toxicity, irritation, allergic response, or other problems or complications, commensurate with a reasonable benefit/risk ratio, within the scope of sound medical judgment.

In some embodiments, the additional element may be physiologically acceptable and related to, or act on, stabilization, solubilization, absorption, or delivery efficiency of the active ingredient of the present application. For example, the additional element may be a carrier, excipient, diluent, preservative, or the like, but is not limited thereto. For example, each additional element (carrier, excipient, or diluent) may be independently selected from lactose, dextrose, sucrose, sorbitol, mannitol, xylitol, erythritol, maltitol, starch, acacia gum, alginate, gelatin, calcium phosphate, calcium silicate, cellulose, methyl cellulose, distilled water, physiological saline, glycerol, ethanol, and HSA (human serum albumin). For example, the preservative may be benzoic acid, sodium benzoate, sorbic acid, para-hydroxybenzoic acid, or chlorobutanol, but is not limited thereto.

The composition may further comprise a filler, bulking agent, binder, and/or wetting agent for formulation, but is not limited thereto.

**Use of the Composition for Treating Parkinson's Disease**

Pharmaceutical Composition

In some embodiments, the composition for treating Parkinson's disease of the present application may be used to treat Parkinson's disease in a subject having Parkinson's disease (for example, a human). In some embodiments, the composition for treating Parkinson's disease may be used to treat symptoms of Parkinson's disease in a subject. In some embodiments, the composition for treating Parkinson's disease may be used to treat an individual in need of treatment for Parkinson's disease. In some embodiments, the Parkinson's disease may be Parkinson's disease at an early stage, an intermediate stage, or an advanced stage. In some embodiments, the Parkinson's disease may be Parkinson's disease at stage 1, stage 2, stage 3, stage 4, or stage 5.

In some embodiments, the subject having Parkinson's disease may be a subject who has been medically or clinically diagnosed as having Parkinson's disease. In some embodiments, the subject may be a subject suspected of having Parkinson's disease.

In some embodiments, the subject for treatment of Parkinson's disease using the composition of the present application may be a human. In some embodiments, the subject for treatment of Parkinson's disease using the composition of the present application may be an animal other than a human, for example a non-human vertebrate. For example, the subject may be a dog, horse, cat, camel, mouse, rat, pig, rabbit, sheep, monkey, chimpanzee, or cow. Preferably, the subject for treatment of Parkinson's disease using the composition of the present application may be a human.

The composition for treating Parkinson's disease may be used for pharmaceutical purposes.

In some embodiments, the composition for treating Parkinson's disease of the present application may be a pharmaceutical composition for treating Parkinson's disease. For example, the pharmaceutical composition for treating Parkinson's disease may comprise, as an active ingredient, the active material of the composition for treating Parkinson's disease described in the preceding descriptions, for example NDST3 protein or a nucleic acid encoding the same, a vehicle comprising NDST3 protein or a nucleic acid encoding the same, or a vector comprising a nucleic acid encoding NDST3 protein. In some embodiments, the pharmaceutical composition for treating Parkinson's disease may comprise the active material in a therapeutically effective amount.

In some embodiments, the pharmaceutical composition may be formulated for oral or parenteral administration. For example, when formulated for oral administration, it may be prepared in the form of a solid preparation, liquid preparation, suspension, granule, capsule, or semi-solid form. For example, when formulated for parenteral administration, it may be prepared in the form of an injection, sustained-release preparation, or aerosol. Preferably, it may be formulated as an injection.

In the case of an injectable formulation, methods known in the art may be additionally used as necessary to prolong the effect of the drug or to delay absorption of the drug. For example, an injectable depot form may be prepared by encapsulating the active ingredient in a biodegradable polymer. In another example, an injectable depot form may be prepared by entrapping the active ingredient in a liposome or a microemulsion.

Kit

In some embodiments, the present application provides a kit comprising a composition for treating Parkinson's disease. A kit comprising the composition for treating Parkinson's disease may be referred to as a kit for treating Parkinson's disease.

The kit for treating Parkinson's disease may comprise instructions for use according to methods known in the art. Generally, the instructions for use include directions for administration of a pharmaceutical composition for treatment, diagnosis, and/or prevention of a disease, for example Parkinson's disease. In some embodiments, the instructions for use may include one or more of information regarding dosage, dosing schedule, and route of administration for the intended treatment. For example, the instructions for use may include information regarding dosage, dosing schedule, and route of administration for treatment of Parkinson's disease using the composition for treating Parkinson's disease of the present application.

In some embodiments, the kit for treating Parkinson's disease may comprise one or more containers. Each container may independently be a vial or a bottle, but is not limited thereto, and may be appropriately selected depending on the intended use or the components contained therein. In some embodiments, the container may contain the composition for treating Parkinson's disease. The type of container may be appropriately selected depending on the form of the composition for treating Parkinson's disease.

## Method for Treating Parkinson's Disease

In some embodiments, the present application provides a method for treating Parkinson's disease in a subject. In some embodiments, the present application provides a method for treating symptoms of Parkinson's disease in a subject. In some embodiments, the method for treating Parkinson's disease or symptoms of Parkinson's disease may comprise administering NDST3 protein or a nucleic acid encoding the same to the subject. For example, a therapeutically effective amount of NDST3 protein or a nucleic acid encoding the same may be administered to the subject. In some embodiments, the method for treating Parkinson's disease may comprise administering to a subject the composition for treating Parkinson's disease of the present application or a pharmaceutical composition thereof. For example, a therapeutically effective amount of the composition for treating Parkinson's disease may be administered to the subject. The composition for treating Parkinson's disease is described in detail in the preceding descriptions including the section "Composition for Treating Parkinson's Disease" of the present application. For example, the composition for treating Parkinson's disease may comprise NDST3 protein or a nucleic acid encoding the same. For example, the composition for treating Parkinson's disease may comprise a vector encoding NDST3 protein, for example an AAV vector, but is not limited thereto. In the treatment method of the present application, the composition for treating Parkinson's disease administered to the subject may be referred to as a therapeutic agent for Parkinson's disease, but is not limited thereto.

In some embodiments, the subject to which the treatment method of the present application is applied may be an individual in need of treatment or prevention of Parkinson's disease. In some embodiments, the subject may be a subject having Parkinson's disease. In some embodiments, the subject may be a subject diagnosed with Parkinson's disease. In some embodiments, the Parkinson's disease may be Parkinson's disease at an early stage, an intermediate stage, or an advanced stage. In some embodiments, the Parkinson's disease may be Parkinson's disease at stage 1, stage 2, stage 3, stage 4, or stage 5.

In some embodiments, the subject may be a human. That is, the subject may be a human having Parkinson's disease or diagnosed with Parkinson's disease. For example, the subject may be a human having Parkinson's disease at an early stage, an intermediate stage, or an advanced stage, or diagnosed with Parkinson's disease at an early stage, an intermediate stage, or an advanced stage. In some embodiments, the Parkinson's disease may be Parkinson's disease at stage 1, stage 2, stage 3, stage 4, or stage 5, and the subject may be a human having Parkinson's disease at stage 1, stage 2, stage 3, stage 4, or stage 5, or diagnosed with Parkinson's disease at stage 1, stage 2, stage 3, stage 4, or stage 5. In some embodiments, the subject may be an animal other than a human, for example a non-human vertebrate. For example, the subject may be a dog, horse, cat, camel, mouse, rat, pig, rabbit, sheep, monkey, chimpanzee, or cow.

The therapeutic agent for Parkinson's disease of the present application, for example the composition for treating Parkinson's disease, may be administered to a subject through various routes depending on clinical needs.

In some embodiments, the composition for treating Parkinson's disease may be administered through an oral or parenteral route. For example, the composition for treating Parkinson's disease may be injected into the subject through a parenteral route.

In some embodiments, the composition for treating Parkinson's disease may be administered orally, intravenously, subcutaneously, intramuscularly, intradermally, intraperitoneally, intra-arterially, through a mucosal route, spinally, intrathecally, intranasally, into the central nervous system for example the brain, or into the cerebrospinal fluid.

In some embodiments, the composition for treating Parkinson's disease may be administered into a ventricle of the brain. For example, the composition for treating Parkinson's disease may be administered to a subject by intracerebroventricular injection.

In some embodiments, the composition for treating Parkinson's disease may be administered to the substantia nigra. For example, the composition for treating Parkinson's disease may be administered to a subject by intra-nigral injection.

In some embodiments, the composition for treating Parkinson's disease may be administered to a subject by intrathecal injection. For example, the composition for treating Parkinson's disease may be injected into the cisterna magna or into the cerebrospinal fluid through intrathecal administration. For example, the composition for treating Parkinson's disease may be administered to a subject by intra-cisterna magna injection, injection of substances directly into cerebrospinal fluid, or injection through lumbar puncture.

In some embodiments, the composition for treating Parkinson's disease may be administered to a subject by intraparenchymal injection into the brain.

In some embodiments, the composition for treating Parkinson's disease may be administered intrathecally. In some embodiments, the composition for treating Parkinson's disease may be administered to the brain, for example to the substantia nigra of the brain.

The dosage of the composition for treating Parkinson's disease of the present application may be determined in consideration of one or more selected from the type of disease, site of administration, body weight, age, and degree of disease progression, but is not limited thereto. Furthermore, the dosage may be appropriately determined within the scope of sound medical judgment, in accordance with a reasonable benefit-risk ratio, and within a range that does not cause excessive toxicity, irritation, allergic reactions, or other problems.

By way of example, when the composition for treating Parkinson's disease comprises an AAV vector, the composition for treating Parkinson's disease may be administered at the following dosage.

For example, an AAV vector may be administered in an amount selected from about $1\times10^5$ GC (genome copy)/kg, $1\times10^6$ GC/kg, $1\times10^7$ GC/kg, $1\times10^8$ GC/kg, $1\times10^9$ GC/kg, $1\times10^{10}$ GC/kg, $1\times10^{11}$ GC/kg, $1\times10^{12}$ GC/kg, $1\times10^{13}$ GC/kg, $1\times10^{14}$ GC/kg, $1\times10^{15}$ GC/kg, $1\times10^{16}$ GC/kg, $1\times10^{17}$ GC/kg, $1\times10^{18}$ GC/kg, $1\times10^{19}$ GC/kg, and $1\times10^{20}$ GC/kg, or within a range defined by two values selected from the foregoing values. For example, an AAV vector may be administered in an amount selected from about $1\times10^5$ GC (genome copy)/head, $1\times10^6$ GC/head, $1\times10^7$ GC/head, $1\times10^8$ GC/head, $1\times10^9$ GC/head, $1\times10^{10}$ GC/head, $1\times10^{11}$ GC/head, $1\times10^{12}$ GC/head, $1\times10^{13}$ GC/head, $1\times10^{14}$ GC/head, $1\times10^{15}$ GC/head, $1\times10^{16}$ GC/head, $1\times10^{17}$ GC/head, $1\times10^{18}$ GC/head, $1\times10^{19}$ GC/head, and $1\times10^{20}$ GC/head, or within a range defined by two values selected from the foregoing values.

In some embodiments, the volume of the composition for treating Parkinson's disease to be administered may be from 1 μl to 10 ml, but is not limited thereto. For example, the volume of the composition for treating Parkinson's disease to be administered may be selected from 1 μl, 2 μl, 3 μl, 4 μl, 5 μl, 6 μl, 7 μl, 8 μl, 9 μl, 10 μl, 20 μl, 30 μl, 40 μl, 50 μl, 60 μl, 70 μl, 80 μl, 90 μl, 100 μl, 200 μl, 300 μl, 400 μl, 500 μl, 600 μl, 700 μl, 800 μl, 900 μl, 1 ml, 5 ml, and 10 ml, or may be within a range defined by two values selected from the foregoing values.

In some embodiments, the composition for treating Parkinson's disease may be administered once or multiple times. In some embodiments, in the case of multiple administrations, the dose for each administration may be determined according to the dosage described above. The dose may be the same or different at each administration, and is not limited thereto. In some embodiments, in the case of multiple administrations, the administrations may be performed at predetermined regular time intervals. In some embodiments, the interval between administrations may not be constant.

For example, the dosage may be determined based on data obtained from cell studies, animal studies, and/or human clinical trials. In this case, the dosage range for humans may be determined based on the dosage obtained from animal studies or preclinical studies using methods known in the art. For example, the following equation may be referred to in the process of determining the dosage for humans.

$$\text{HED (Human Equivalent Dose)} = \text{animal NOAEL} \times (W_{animal}/W_{human})^{(1-b)}$$

Here, NOAEL (No Observed Adverse Effect Level) refers to a no-observed-adverse-effect dose. For example, b may be 0.67. In order to establish an appropriate dosage, a researcher may refer to the literature [Nair, A. B., & Jacob, S. (2016). A simple practice guide for dose conversion between animals and human. Journal of Basic and Clinical Pharmacy, 7(2), 27], but is not limited thereto.

In some embodiments, the composition for treating Parkinson's disease may be administered together with another element or another substance. For example, a substance administered together with the composition for treating Parkinson's disease may be a substance known as a therapeutic agent for neurological diseases such as Parkinson's disease, and such a substance known as a therapeutic agent for neurological diseases may produce a synergistic effect with the composition for treating Parkinson's disease of the present application. For example, a substance that assists the composition for treating Parkinson's disease of the present application and enhances the therapeutic effect on Parkinson's disease may be co-administered. For example, a substance for alleviating toxicity of the composition for treating Parkinson's disease may be co-administered. For example, the composition for treating Parkinson's disease may be used together with levodopa, carbidopa, and/or pramipexole, but is not limited thereto.

The above-described other element or other substance may be administered simultaneously with the composition for treating Parkinson's disease, on the same day, or at a different time. When administered at a different time, the other substance may be administered once or multiple times at regular or irregular time intervals relative to administration of the composition for treating Parkinson's disease, but is not limited thereto.

The administration concentration, administration dose, and administration schedule used in the above-described method for treating Parkinson's disease may be appropriately determined as needed. For example, a researcher, physician, or veterinarian may appropriately determine or prescribe these factors as necessary. For example, a physician or veterinarian may gradually increase or decrease the dosage until the desired therapeutic effect is achieved. In addition, a physician or veterinarian may determine the administration dose and duration of administration depending on the condition of the subject or patient.

In some embodiments, the method for treating Parkinson's disease may further comprise confirming the prognosis of the subject. For example, the treatment progress or therapeutic effect of Parkinson's disease may be evaluated using clinical or medical methods known in the art, but is not limited thereto. For example, the treatment progress of Parkinson's disease may be evaluated using F-18-DOPA PET scanning, dopamine transporter scanning, or other methods known in the art. In some embodiments, the method for treating Parkinson's disease may further comprise a step of supportive treatment. The supportive treatment may include physical therapy, rehabilitation therapy, administration of anticonvulsants and/or antidepressants, but is not limited thereto.

### Exemplary Embodiments

Hereinafter, exemplary embodiments of the invention provided according to some embodiments of the present application are described. The invention provided by the present application is not limited to the examples described below.

### Exemplary Embodiments of a Composition for Treating Parkinson's Disease Comprising NDST3 Protein or a Nucleic Acid Encoding the Same

A01. A pharmaceutical composition for treating Parkinson's disease comprising NDST3 protein or a nucleic acid encoding the same.

A02. The pharmaceutical composition of A01, wherein the NDST3 protein is a human NDST3 protein.

A03. The pharmaceutical composition of A02, wherein the NDST3 protein is a wild-type human NDST3 protein or a variant thereof.

A04. The pharmaceutical composition of A02, wherein the human NDST3 protein has the amino acid sequence of SEQ ID NO: 1 or an amino acid sequence having 80% or more and less than 100% sequence identity thereto.

A05. The pharmaceutical composition of A02, wherein the human NDST3 protein has the amino acid sequence of SEQ ID NO: 1.

A06. The pharmaceutical composition of A02, wherein the human NDST3 protein is a wild-type human NDST3 protein or a functional equivalent thereof.

A07. The pharmaceutical composition of A02, wherein the NDST3 protein is a variant of a wild-type human NDST3 protein, wherein the variant of the wild-type human NDST3 protein comprises one or more conservative substitutions of amino acid residues in the wild-type human NDST3 protein having the amino acid sequence of SEQ ID NO: 1, thereby the human NDST3 protein has an amino acid sequence having 80% or more and less than 100% sequence identity to the amino acid sequence of the wild-type human NDST3 protein.

A08. The pharmaceutical composition of A01, wherein the nucleic acid encoding the NDST3 protein comprises an NDST3 protein-encoding region, wherein the NDST3 protein-encoding region has a nucleic acid sequence encoding the NDST3 protein.

A09. The pharmaceutical composition of A08, wherein the nucleic acid sequence encoding the NDST3 protein is a DNA sequence, an RNA sequence, or a DNA/RNA hybrid sequence.

A10. The pharmaceutical composition of any one of A08 to A09, wherein the nucleic acid sequence encoding the NDST3 protein has a full-length sequence of an NDST3 gene, an ORF sequence of the NDST3 gene, a CDS of the NDST3 gene, or a cDNA sequence of the NDST3 gene.

A11. The pharmaceutical composition of any one of A08 to A10, wherein the nucleic acid sequence encoding the NDST3 protein has a full-length sequence of a wild-type human NDST3 gene, an ORF sequence of the wild-type human NDST3 gene, a CDS of the wild-type human NDST3 gene, or a cDNA sequence of the wild-type human NDST3 gene.

A12. The pharmaceutical composition of any one of A08 to A10, wherein the nucleic acid sequence encoding the NDST3 protein is the nucleic acid sequence of SEQ ID NO: 7 or a nucleic acid sequence having 80% or more and less than 100% sequence identity thereto.

A13. The pharmaceutical composition of any one of A08 to A10, wherein the nucleic acid sequence encoding the NDST3 protein is the nucleic acid sequence of SEQ ID NO: 7.

A14. The pharmaceutical composition of any one of A08 to A13, wherein the nucleic acid encoding the NDST3 protein further comprises one or more additional elements.

A15. The pharmaceutical composition of A14, wherein each of the one or more additional elements is independently selected from a promoter, enhancer, polyadenylation signal, Kozak consensus sequence, ITR (inverted terminal repeat), LTR (long terminal repeat), terminator, origin of replication, multicloning site (MCS), internal ribosome entry site (IRES), a nuclear localization signal-encoding sequence, and 2A self-cleaving peptides.

A16. The pharmaceutical composition of any one of A14 to A15, wherein the nucleic acid encoding the NDST3 protein further comprises a promoter, and the promoter is operably linked to the NDST3 protein-encoding region.

A17. The pharmaceutical composition of A16, wherein the promoter is an SV40 early promoter, LTR (mouse mammary tumor virus long terminal repeat) promoter, Ad MLP (adenovirus major late) promoter, HSV (herpes simplex virus) promoter, CMV (cytomegalovirus) promoter, RSV (Rous sarcoma virus) promoter, U6 promoter, CBA promoter, PGK promoter, NG2 promoter, NES promoter, GFAP promoter, CaMKII promoter, NSE promoter, SYN1 promoter, or CAG promoter.

A18. The pharmaceutical composition of any one of A01 to A17, wherein the NDST3 protein or the nucleic acid encoding the same is attached to or comprised in a vehicle.

A19. The pharmaceutical composition of A18, wherein the vehicle is a nanoparticle, liposome, lipid nanoparticle, carbon nanotube, chitosan, protein, peptide, DNA, DNA-RNA lipid complex, polymer, DNA-polymer complex, artificial chromosome, cosmid, or vector.

A20. The pharmaceutical composition of any one of A01 to A17, wherein the nucleic acid encoding the NDST3 protein is comprised in a vector.

A21. The pharmaceutical composition of A20, wherein the vector is a viral vector.

A22. The pharmaceutical composition of A21, wherein the viral vector is selected from retrovirus, lentivirus, adenovirus, adeno-associated virus, vaccinia virus, poxvirus, HIV (human immunodeficiency virus), MLV (Murine leukemia virus), ASLV (avian sarcoma/leukosis virus), SNV (spleen necrosis virus), RSV (Rous sarcoma virus), MMTV (mouse mammary tumor virus), herpes simplex virus, episomal virus, and herpes virus.

A23. The pharmaceutical composition of any one of A21 to A22, wherein the viral vector is an adeno-associated virus (AAV) vector.

A24. The pharmaceutical composition of A23, wherein the AAV is selected from AAV1, AAV2, AAV3, AAV4, AAV5, AAV6, AAV7, AAV8, AAV9, AAV10, AAV11, AAV12, AAV13, AAV-DJ, AAV-DJ/8, AAV-Rh10, AAV-retro, AAV-PHP.B, AAV-PHP.eB, and AAV-PHP.S.

A25. The pharmaceutical composition of any one of A23 to A24, wherein the AAV is AAV9.

A26. The pharmaceutical composition of A20, wherein the vector is a non-viral vector.

A27. The pharmaceutical composition of A26, wherein the non-viral vector is selected from plasmid, phage, naked DNA, DNA-RNA lipid complex, liposome, nanoparticle, DNA-polymer complex, mRNA, artificial chromosome, and cosmid.

A28. The pharmaceutical composition of any one of A01 to A27, wherein the pharmaceutical composition further comprises one or more pharmaceutically acceptable additional elements.

A29. The pharmaceutical composition of A28, wherein the pharmaceutically acceptable additional element is independently selected from a carrier, excipient, diluent, and preservative.


**Exemplary Embodiments of a Composition for Treating Parkinson's Disease Comprising a Vector Encoding NDST3 Protein**

B01. A pharmaceutical composition for treating Parkinson's disease comprising a vector comprising a nucleic acid encoding NDST3 protein.

B02. The pharmaceutical composition of B01, wherein the vector is a viral vector.

B03. The pharmaceutical composition of B02, wherein the viral vector is selected from retrovirus, lentivirus, adenovirus, adeno-associated virus, vaccinia virus, poxvirus, HIV (human immunodeficiency virus), MLV (Murine leukemia virus), ASLV (avian sarcoma/leukosis virus), SNV (spleen necrosis virus), RSV (Rous sarcoma virus), MMTV (mouse mammary tumor virus), herpes simplex virus, episomal virus, and herpes virus.

B04. The pharmaceutical composition of any one of B02 to B03, wherein the viral vector is an adeno-associated virus (AAV) vector.

B05. The pharmaceutical composition of B04, wherein the AAV is selected from AAV1, AAV2, AAV3, AAV4, AAV5, AAV6, AAV7, AAV8, AAV9, AAV10, AAV11, AAV12, AAV13, AAV-DJ, AAV-DJ/8, AAV-Rh10, AAV-retro, AAV-PHP.B, AAV-PHP.eB, and AAV-PHP.S.

B06. The pharmaceutical composition of any one of B04 to B05, wherein the AAV is AAV9.

B07. The pharmaceutical composition of B01, wherein the vector is a non-viral vector.

B08. The pharmaceutical composition of B07, wherein the non-viral vector is selected from plasmid, phage, naked DNA, DNA-RNA lipid complex, liposome, nanoparticle, DNA-polymer complex, mRNA, artificial chromosome, and cosmid.

B09. The pharmaceutical composition of any one of B01 to B08, wherein the pharmaceutical composition further comprises one or more pharmaceutically acceptable additional elements.

B10. The pharmaceutical composition of B09, wherein the pharmaceutically acceptable additional element is independently selected from a carrier, excipient, diluent, and preservative.

B11. The pharmaceutical composition of any one of B01 to B10, wherein the NDST3 protein is a human NDST3 protein.

B12. The pharmaceutical composition of B11, wherein the human NDST3 protein is a wild-type human NDST3 protein or a variant thereof.

B13. The pharmaceutical composition of B11, wherein the human NDST3 protein has the amino acid sequence of SEQ ID NO: 1 or an amino acid sequence having 80% or more and less than 100% sequence identity thereto.

B14. The pharmaceutical composition of B11, wherein the human NDST3 protein has the amino acid sequence of SEQ ID NO: 1.

B15. The pharmaceutical composition of B11, wherein the human NDST3 protein is a wild-type human NDST3 protein or a functional equivalent thereof.

B16. The pharmaceutical composition of B11, wherein the human NDST3 protein is a variant of a wild-type human NDST3 protein, wherein the variant of the wild-type human NDST3 protein comprises one or more conservative substitutions of amino acid residues in the wild-type human NDST3 protein having the amino acid sequence of SEQ ID NO: 1, thereby the human NDST3 protein has an amino acid sequence having 80% or more and less than 100% sequence identity to the amino acid sequence of the wild-type human NDST3 protein.

B17. The pharmaceutical composition of any one of B01 to B10, wherein the nucleic acid encoding the NDST3 protein comprises an NDST3 protein-encoding region, wherein the NDST3 protein-encoding region has a nucleic acid sequence encoding the NDST3 protein.

B18. The pharmaceutical composition of B17, wherein the nucleic acid sequence encoding the NDST3 protein is a DNA sequence, an RNA sequence, or a DNA/RNA hybrid sequence.

B19. The pharmaceutical composition of any one of B17 to B18, wherein the nucleic acid sequence encoding the NDST3 protein has a full-length sequence of an NDST3 gene, an ORF sequence of the NDST3 gene, a CDS of the NDST3 gene, or a cDNA sequence of the NDST3 gene.

B20. The pharmaceutical composition of any one of B17 to B19, wherein the nucleic acid sequence encoding the NDST3 protein has a full-length sequence of a wild-type human NDST3 gene, an ORF sequence of the wild-type human NDST3 gene, a CDS of the wild-type human NDST3 gene, or a cDNA sequence of the wild-type human NDST3 gene.

B21. The pharmaceutical composition of any one of B17 to B19, wherein the nucleic acid sequence encoding the NDST3 protein is the nucleic acid sequence of SEQ ID NO: 7 or a nucleic acid sequence having 80% or more and less than 100% sequence identity thereto.

B22. The pharmaceutical composition of any one of B17 to B19, wherein the nucleic acid sequence encoding the NDST3 protein is the nucleic acid sequence of SEQ ID NO: 7.

B23. The pharmaceutical composition of any one of B17 to B22, wherein the nucleic acid encoding the NDST3 protein further comprises one or more additional elements.

B24. The pharmaceutical composition of B23, wherein each of the one or more additional elements is independently selected from a promoter, enhancer, polyadenylation signal, Kozak consensus sequence, ITR (inverted terminal repeat), LTR (long terminal repeat), terminator, origin of replication, multicloning site (MCS), internal ribosome entry site (IRES), a nuclear localization signal-encoding sequence, and 2A self-cleaving peptides.

B25. The pharmaceutical composition of any one of B17 to B24, wherein the nucleic acid encoding the NDST3 protein further comprises a promoter, and the promoter is operably linked to the NDST3 protein-encoding region.

B26. The pharmaceutical composition of B25, wherein the promoter is an SV40 early promoter, LTR (mouse mammary tumor virus long terminal repeat) promoter, Ad MLP (adenovirus major late) promoter, HSV (herpes simplex virus) promoter, CMV (cytomegalovirus) promoter, RSV (Rous sarcoma virus) promoter, U6 promoter, CBA promoter, PGK promoter, NG2 promoter, NES promoter, GFAP promoter, CaMKII promoter, NSE promoter, SYN1 promoter, or CAG promoter.

B27. A pharmaceutical composition for treating Parkinson's disease, comprising a viral vector encoding an NDST3 protein.

B28. The pharmaceutical composition of B27, wherein the NDST3 protein has the amino acid sequence of SEQ ID NO: 1 or an amino acid sequence having 80% or more and less than 100% sequence identity thereto.

B29. The pharmaceutical composition of B27, wherein the NDST3 protein has the amino acid sequence of SEQ ID NO: 1.

B30. The pharmaceutical composition of any one of B27 to B29, wherein the viral vector is an AAV vector.

B31. The pharmaceutical composition of any one of B27 to B30, wherein the viral vector is an AAV9 vector.

**Exemplary Embodiments of a Method for Treating Parkinson's Disease** C01. A method for treating Parkinson's disease in a subject, comprising administering to the subject the pharmaceutical composition of any one selected from A01 to A29 and B01 to B31.

C02. The method of C01, wherein the subject is a subject having Parkinson's disease.

C03. The method of any one of C01 to C02, wherein the subject is a subject diagnosed with Parkinson's disease.

C04. The method of any one of C01 to C03, wherein the Parkinson's disease of the subject is Parkinson's disease at stage 1, stage 2, stage 3, stage 4, or stage 5.

C05. The method of any one of C01 to C04, wherein the Parkinson's disease of the subject is early-stage, mid-stage, or advanced-stage Parkinson's disease.

C06. The method of any one of C01 to C05, wherein the pharmaceutical composition is administered to the subject via a parenteral route.

C07. The method of any one of C01 to C06, wherein the pharmaceutical composition is administered intravenously, subcutaneously, intramuscularly, intradermally, intraperitoneally, intra-arterially, via a mucosal route, spinally, intrathecally, intranasally, or into the brain.

C08. The method of any one of C01 to C06, wherein the pharmaceutical composition is administered by intracerebroventricular injection.

C09. The method of any one of C01 to C06, wherein the pharmaceutical composition is administered by intrathecal injection.

C10. The method of C09, wherein the pharmaceutical composition is administered into the cisterna magna.

**Experimental Example**

**1. Preparation of an AAV Vector Encoding Ndst3**

Preparation of an AAV Vector Encoding Human NDST3 Protein

The inventors of the present application prepared an AAV vector encoding human NDST3 protein, that is, an AAV vector encoding Ndst3. Information on the CDS (coding sequence) of NDST3 was obtained from human cells. After preparing the AAV vector encoding Ndst3, the inventors treated in vitro and in vivo models in which Parkinson's disease had been induced with the vector and confirmed the therapeutic effect of Ndst3 on Parkinson's disease. As used herein, the AAV vector encoding Ndst3 refers to an AAV vector comprising a nucleic acid sequence encoding human NDST3 protein and constructed so as to enable expression of the human NDST3 protein in cells. Hereinafter, the method for preparing the AAV vector encoding Ndst3 used in the experiments disclosed in the present application is described in detail.

To prepare a vector encoding Ndst3, an AAV 293 cell line (Cell Biolabs; Catalog Number: AAV-100) was used. AAV 293 cells were cultured in a medium containing DMEM (Dulbecco's Modified Eagle Medium; Gibco; Cat. No. 12430054), 10% fetal bovine serum (Gibco; Cat. No. 10100147), 0.1 mM NEAA (MEM™ Non-Essential™ Amino Acids; Gibco; Cat. No. 11140050), 2 mM L-glutamine (Gibco; Cat. No. A2916801), and 1% P/S (Penicillin-Streptomycin; Gibco; Cat. No. 15140122), in a 150 mm$^2$ cell culture dish at 37 °C under 5% $CO_2$ conditions.

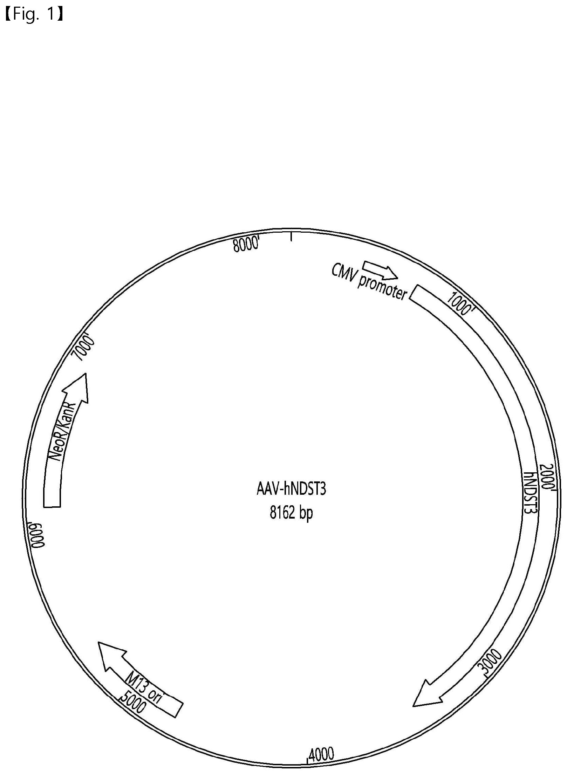

Subsequently, for transfection of a helper plasmid, a Rep-Cap plasmid, and a transfer plasmid containing a nucleic acid encoding the human NDST3 protein, the AAV 293 cells were treated with the helper plasmid (pHelper, 10 μg per dish), the Rep-Cap plasmid (pRC, 10 μg per dish), and the transfer plasmid (10 μg per dish) comprising a nucleic acid sequence encoding the human NDST3 protein, followed by incubation for 48 hours. A schematic diagram of the transfer plasmid comprising the nucleic acid encoding the human NDST3 protein is shown in Fig. 1. The nucleic acid sequence encoding the human NDST3 protein contained in the transfer plasmid has the nucleic acid sequence of SEQ ID NO: 7. The CMV promoter sequence contained in the transfer plasmid has the nucleic acid sequence of SEQ ID NO: 8.

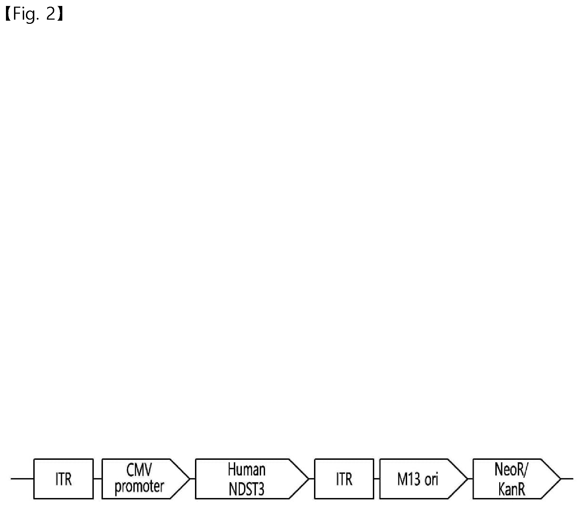

Thereafter, an AAV vector encoding Ndst3 (an AAV9 vector encoding the human NDST3 protein) was obtained from the transfected AAV 293 cell population using an AAV extraction solution and purification kit (Takara; Cat. No. 6679). Here, the human NDST3 protein has the amino acid sequence of SEQ ID NO: 1. A schematic diagram of the nucleic acid contained in the obtained AAV vector encoding Ndst3 is shown in Fig. 2.

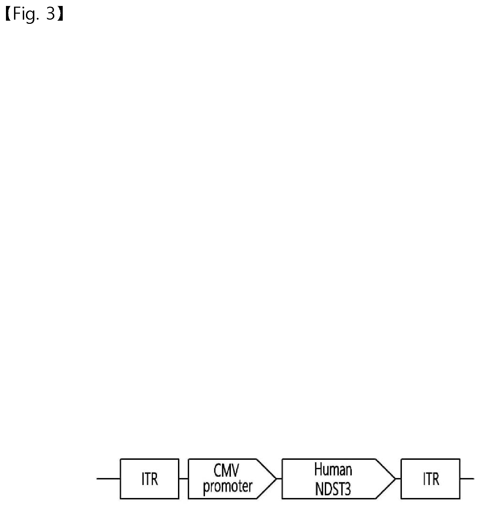

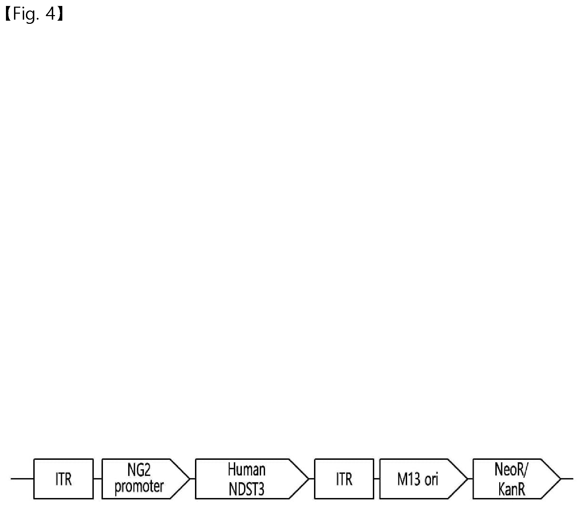

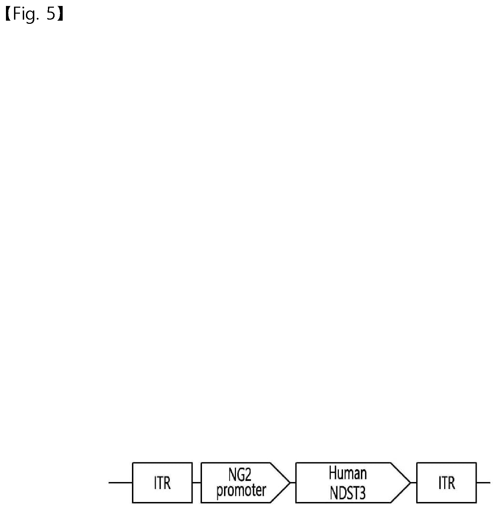

Thereafter, for administration to humans, the AAV vector may comprise a nucleic acid that does not include M13 ori and NeoR/KanR, a schematic diagram of which is shown in Fig. 3. Furthermore, an AAV vector encoding Ndst3 may be prepared in a manner similar to the method described above using an NG2 promoter instead of a CMV promoter, and schematic diagrams of the AAV vectors encoding Ndst3 are shown in Fig. 4 and Fig. 5.

The nucleic acid sequence encoding the human NDST3 protein (SEQ ID NO: 7), the nucleic acid sequence of the CMV promoter (SEQ ID NO: 8), the nucleic acid sequence of M13 ori (SEQ ID NO: 9), the nucleic acid sequence of NeoR/KanR (SEQ ID NO: 10), and the nucleic acid sequence of the NG2 promoter (SEQ ID NO: 11) are disclosed below.

- hNDST3:

atgagttttatcatgaagcttcacagacactttcaaagaacagtcattctgcttgccactttttgt
atggtgagcattattattttctgcttactacctgtacagtggctacaaacaggaaaatgaactctct
gagacggcttcagaagttgactgtggcgacctccaacacctaccatatcaactaatggaagtgaaa
gcaatgaagctttttgatgcctcaaggacagaccccacagtcctagtatttgtagagagccagtac
tcatctcttggtcaagacatcattatgattctagaatcaagtagattccagtatcacattgaaatt
gcccctggaaagggagatctcccagtgcttatagacaaaatgaaaggcaaatacattctcattatt
tatgagaatattttaaagtatataaatatggattcctggaatcgaagccttctagataaatactgt
gtagaatatggtgtgggtgtcattggattccacaaaaactagtgagaagagtgtacagagctttcag
ttaaaaggtttcccttttttccatatatggaaatcttgcagtaaaagattgttgtattaatcctcat
tctccattgattcgtgtgaccaaatcttccaagcttgaaaaaggttctttacctggaactgactgg
acagtttttcagattaatcattcagcctatcaaccagtaatattgccaaagtaaagaccccagaa
aacctttctccttccatctctaaaggtgctttttatgccactattatacatgacctggggcttcat
gatggaattcaaagggttcttttttggcaacaacttgaacttttggctgcacaagctcatcttcata
gatgccatctccttcttatcagggaagaggctgacattgtccttggacaggtacattcttgtggat
attgatgatatatttgtgggaaaagagggaacaagaatgaacaccaatgatgtaaaggccctgctt
gatactcagaatcttttgcgtgcacaaatcacaaattttacattcaacctgggattttcagggaaa
ttttaccatacaggaactgaagaggaagatgaaggagatgactgtctgttggggtctgtggatgag
ttctggtggtttcctcacatgtggagccatatgcagccccacctcttccacaatgagtcatctttg
gtggagcagatgattctcaacaaaaaatttgccttagagcacggcattccaacggacatgggctac
gctgtggcccctcaccattcgggcgtctaccctgtacatgttcagctttatgaggcctggaagaag
gtctggaatattaaaatcaccagcactgaagaatatccacatctgaagccagctagataccggagg
ggttttatccacaaaaacatcatggttctcccaagacaaacctgtgggcttttcactcacaccatt
ttctacaaagaatatccaggggggtcctaaagagctggataagagtatccaaggaggagaacttttc
ttcactgtcgtcctcaaccctatcagcatttttcatgacccatttgtccaactatgggaatgaccga
ctgggattatatacatttgttaatctggccaactttgtgaagagctggaccaacctgcgacttcag
actctgcctccagtacaactggcccacaagtattttgagctgtttcctgatcagaaagaccctctc
tggcagaatccttgcgatgacaaacgccacagagacattggtctaaagaaaaaacttgtgatcgc
ttaccaaaattcttggtaataggaccccagaaaactggtaccactgctttgtatttgttcctggtt
atgcatccttccatccttagtaactcccccagcccaaaaacctttgaggaggtacagttctttaat
agaaataactaccacaggggggattgattggtatatggatttcttcccagtcccatctaatgtcact
accgacttttttgtttgagaagagtgccaattacttccactcagaggaagcccctaaaagagctgct
tctctggttcccaaagccaagattatcaccattctcattgacccttcagaccgagcatactcctgg
taccagcatcagcgatcacatgaagaccctgcagctctgaagtttagcttctacgaagtgatctca

gcagggccccgtgcaccctcggagctcagagccttgcagaagagatgtttggtcccggggtggtat
gccagccacatcgagagatggcttgtttatttcccccccatttcagttgctaattattgatgggcaa
caactaagaactgatcctgctacagtgatggatgaagtacagaagtttctaggagtcttgcctcat
tataattactcagaagctttaacgtttgattctcataaaggtttctggtgtcagttactggaagaa
ggtaaaacaaaatgccttggaaagagcaaaggaagaaaataccctccaatggattctgatagcagg
acatttctgtcaagctactatcgagatcacaacgtggaactctcaaagctgctgcacaaactgggt
cagcctctgccatcctggctgagacaggagctgcagaaagtaaga_tag_

(SEQ ID NO: 7)

- CMV promoter:

Gtgatgcggttttggcagtacatcaatgggcgtggatagcggtttgactcacggggatttccaagtctccaccccat

tgacgtcaatgggagtttgttttggcaccaaaatcaacgggactttccaaaatgtcgtaacaactccgccccattga

cgcaaatgggcggtaggcgtgtacggtgggaggtctatataagcagagct（SEQ ID NO: 8)

- M13 ori:

acgcgccctgtagcggcgcattaagcgcggcgggtgtggtggttacgcgcagcgtgaccgctacac
ttgccagcgccctagcgcccgctcctttcgctttcttcccttcctttctcgccacgttcgccggct
ttccccgtcaagctctaaatcggggggctccctttagggttccgatttagtgctttacggcacctcg
accccaaaaaacttgattagggtgatggttcacgtagtgggccatcgccctgatagacggtttttc
gccctttgacgttggagtccacgttctttaatagtggactcttgttccaaactggaacaacactca
accctatctcggtctattcttttgatttataagggattttgccgatttcggcctattggttaaaaa
atgagctgatttaacaaaaatttaacgcgaattttaacaaaatattaacgcttacaatttaaatat
ttgcttatacaatcttcctgttttttggggctttctgattatcaaccggggt

(SEQ ID NO: 9)

- NeoR/KanR:

ATGATTGAACAAGATGGATTGCACGCAGGTTCTCCGGCCGCTTGGGTGGAGAGGCTATTCGGCTAT
GACTGGGCACAACAGACAATCGGCTGCTCTGATGCCGCCGTGTTCCGGCTGTCAGCGCAGGGGCGC
CCGGTTCTTTTTGTCAAGACCGACCTGTCCGGTGCCCTGAATGAACTGCAAGACGAGGCAGCGCGG
CTATCGTGGCTGGCCACGACGGGCGTTCCTTGCGCAGCTGTGCTCGACGTTGTCACTGAAGCGGGA
AGGGACTGGCTGCTATTGGGCGAAGTGCCGGGGCAGGATCTCCTGTCATCTCACCTTGCTCCTGCC
GAGAAAGTATCCATCATGGCTGATGCAATGCGGCGGCTGCATACGCTTGATCCGGCTACCTGCCCA
TTCGACCACCAAGCGAAACATCGCATCGAGCGAGCACGTACTCGGATGGAAGCCGGTCTTGTCGAT
CAGGATGATCTGGACGAAGAGCATCAGGGGCTCGCGCCAGCCGAACTGTTCGCCAGGCTCAAGGCG
AGCATGCCCGACGGCGAGGATCTCGTCGTGACCCATGGCGATGCCTGCTTGCCGAATATCATGGTG
GAAAATGGCCGCTTTTCTGGATTCATCGACTGTGGCCGGCTGGGTGTGGCGGACCGCTATCAGGAC
ATAGCGTTGGCTACCCGTGATATTGCTGAAGAGCTTGGCGGCGAATGGGCTGACCGCTTCCTCGTG
CTTTACGGTATCGCCGCTCCCGATTCGCAGCGCATCGCCTTCTATCGCCTTCTTGACGAGTTCTTC
TGA

(SEQ ID NO: 10)

- NG2 promoter:

```
TTGAGCTGCACTTTCGTCCTCCATGAAATGGGGGAGGGGATGCTCCTCACCCACCTTGCAAGGTTA
TTTTGAGGCAAATGTCATGGCGGGACTGAGAATTCTTCTGCCCTGCGAGGAAATCCAGACATCTCT
CCCTTACAGACAGGGAGACTGAGGTGAGGCCCTTCCAGGCAGAGAAGGTCACTGTTGCAGCCATGG
GCAGTGCCCCACAGGACCTCGGGTGGTGCCTCTGGAGTCTGGAGAAGTTCCTAGGGGACCTCCGAG
GCAAAGCAGCCCAAAAGCCGCCTGTGAGGGTGGCTGGTGTCTGTCCTTCCTCCTAAGGCTGGAGTG
TGCCTGTGGAGGGGTCTCCTGAACTCCCGCAAAGGCAGAAAGGAGGGAAGTAGGGGCTGGGACAGT
TCATGCCTCCTCCCTGAGGGGGTCTCCCGGGCTCGGCTCTTGGGGCCAGAGTTCAGGGTGTCTGGG
CCTCTCTATGACTTTGTTCTAAGTCTTTAGGGTGGGGCTGGGGTCTGGCCCAGCTGCAAGGGCCCC
CTCACCCCTGCCCCAGAGAGGAACAGCCCCGCACGGGCCCTTTAAGAAGGTTGAGGGTGGGGGCAG
GTGGGGGAGTCCAAGCCTGAAACCCGAGCGGGCGCGCGGGTCTGCGCCTGCCCCGCCCCCGGAGTT
AAGTGCGCGGACACCCGGAGCCGGCCCGCGCCCAGGAGCAGAGCCGCGCTCGCTCCACTCAGCTCC
CAGCTCCCAGGACTCCGCTGGCTCCTCGCAAGTCCT
```

(SEQ ID NO: 11)

## 2. Confirmation of the Therapeutic Effect of Ndst3 on Parkinson's Disease

2.1. Establishment of an in vitro Parkinson's disease model using 6-OHDA and treatment with the AAV vector

Establishment of an in vitro Parkinson's disease model

### (1) Isolation and culture of primary dopaminergic neurons

The skull was removed from E13-15 mouse embryos and the brain was extracted. Thereafter, the meninges were removed from the extracted brain. The midbrain region was collected, and the brain tissue was finely chopped using a sectioning method. The chopped tissue was transferred into a conical tube using DPBS (Dulbecco's Phosphate-Buffered Saline). The tissue was pelleted by centrifugation and the supernatant was removed. To dissociate the tissue, TrypLE (Gibco; Cat. No. 12605010) and 0.5 M ascorbic acid (Sigma; Cat. No. A4403) were added to the chopped tissue, followed by mixing by inversion and incubation at 37 °C for 20 minutes, with inversion every 10 minutes. The resulting tissue was then neutralized with a plating medium composed of Neurobasal medium (Gibco; Cat. No. 21103049), $1\times$ B27 (Gibco; Cat. No. 17504044), 1.2 mg/ml laminin (Corning; Cat. No. 354232), 1% L-glu (Gibco; Cat. No. A2916801), 10% fetal bovine serum (Gibco; Cat. No. 10100147), and 1% penicillin-streptomycin (P/S; Gibco; Cat. No. 15140122). The dissociated tissue was filtered through a 70 $\mu$m cell strainer (Falcon; Cat. No. 352350) and centrifuged at 1200 rpm for 3 minutes to obtain cells from the dissociated tissue. The obtained cells (dopaminergic neurons) were seeded onto a cell culture dish coated with PDL (Poly-D-lysine) together with the plating medium. For culture of the primary dopaminergic neurons, the medium in the dish was replaced the next day with a culture medium composed of Neurobasal medium (Gibco), 1.2 mg/ml laminin (Corning), 0.25% L-glu (Gibco), 1% fetal bovine serum (Gibco), and 1% penicillin-streptomycin (P/S; Gibco).

### (2) Establishment of a Parkinson's disease in vitro model using 6-OHDA treatment

To establish a Parkinson's disease model in primary dopaminergic neurons, 6-OHDA (6-hydroxydopamine hydrochloride; Sigma; Cat. No. H4381), which is commonly used for modeling Parkinson's disease, was treated to generate a 6-OHDA-induced Parkinson's disease in vitro model. Three days after seeding the cells, 100 $\mu$M 6-OHDA was added to the primary dopaminergic neuron culture dish.

Treatment with the AAV vector encoding Ndst3

On the day following the 6-OHDA treatment, the AAV vector encoding Ndst3 (Ndst3, $1 \times 10^8$ GC) obtained in Experimental Example 1 ("1. Preparation of an AAV vector encoding Ndst3") was added to the primary dopaminergic neuron culture dish. Thereafter, for the experiment, both the AAV vector-treated group and the untreated group were cultured. The group treated or injected with the AAV vector encoding Ndst3 may also be referred to as the Ndst3-treated group.

Experimental timeline

The timeline of the experiments performed in relation to the 6-OHDA-induced Parkinson's disease in vitro model is summarized below.

Table 4. Experimental timeline related to the 6-OHDA-induced Parkinson's disease in vitro model.

| Day 0 | Seeding of primary dopaminergic neurons obtained from dissected tissue |
|---|---|
| Day 3 | Treatment of the primary dopaminergic neuron culture with 6-OHDA |
| Day 4 | Treatment of the 6-OHDA-treated primary dopaminergic neuron culture with an AAV vector encoding Ndst3 (Ndst3) |
| Day 14 | Analysis of the Ndst3-encoding AAV vector-treated group and the untreated group (e.g., neuronal marker analysis, electrophysiological analysis, etc.) |

## 2.2. Establishment of a 6-OHDA-induced Parkinson's disease mouse model and administration of an AAV vector encoding Ndst3

Establishment of a 6-OHDA-induced Parkinson's disease mouse model

A 6-OHDA-induced Parkinson's disease mouse model was established. Eight-week-old ICR mice (Daehan Biolink, DBL) were anesthetized by intraperitoneal injection of avertin (125-250 mg/kg). Once the mice were anesthetized, they were fixed on a surgical table, and the surgical site was disinfected using povidone and 70% ethanol and then sufficiently dried. After confirming anesthesia, the scalp was incised and the stereotaxic coordinates were fixed. A total of 2 $\mu$L of 6-OHDA (10 $\mu$g/$\mu$L, dissolved in 0.02% ascorbate/saline) was injected at the coordinates Medial-Lateral (ML) -1.1 mm, Anterior-Posterior (AP) -3.1 mm, and Dorsal-Ventral (DV) -4.4 mm to establish the 6-OHDA-induced Parkinson's disease model.

Administration of the AAV vector encoding Ndst3

An AAV vector encoding Ndst3 was injected into the 6-OHDA-induced Parkinson's disease mouse model. Three to five days after establishment of the 6-OHDA-induced Parkinson's disease model, the AAV vector encoding Ndst3 (1 $\times$ $10^8$ GC), obtained in Experimental Example 1 ("1. Preparation of an AAV vector encoding Ndst3"), was injected at the coordinates Medial-Lateral (ML) -1.1 mm, Anterior-Posterior (AP) -3.1 mm, and Dorsal-Ventral (DV) -4.4 mm.

Summary of Experimental Timeline

The timeline of the experiments performed in relation to the 6-OHDA-induced Parkinson's disease mouse model is summarized below.

Table 5. Experimental timeline related to the 6-OHDA-induced Parkinson's disease mouse model.

| Day 0 | Establishment of a 6-OHDA-induced Parkinson's disease mouse model by injection of 6-OHDA |
|---|---|
| Day 3-5 | Injection of an AAV vector encoding Ndst3 (Ndst3) into the 6-OHDA-induced Parkinson's disease mouse model |
| Day 21-23 | Analysis of the Ndst3-treated group and the untreated group (behavioral analysis) |
| Day 23-25 | Analysis of the Ndst3-treated group and the untreated group (post-sacrifice histological analysis, etc.) |

## 2.3. Establishment of an MPTP-induced Parkinson's disease mouse model and administration of an AAV vector encoding Ndst3

Establishment of an MPTP-induced Parkinson's disease mouse model

An MPTP-induced Parkinson's disease mouse model was established. Eight-week-old C57BL/6 mice (Daehan Biolink, DBL) were intraperitoneally injected with MPTP (30 mg/kg; Sigma; Cat. No. M0896) once daily for seven consecutive days to establish the MPTP-induced Parkinson's disease model.

Administration of the AAV vector encoding Ndst3

An AAV vector encoding Ndst3 was injected into the MPTP-induced Parkinson's disease mouse model. Seven days after completion of the MPTP injections, the AAV vector encoding Ndst3 ($1 \times 10^8$ GC), obtained in Experimental Example 1 ("1. Preparation of an AAV vector encoding Ndst3"), was injected at the coordinates Medial-Lateral (ML) -1.1 mm, Anterior-Posterior (AP) -3.1 mm, and Dorsal-Ventral (DV) -4.4 mm.

Summary of Experimental Timeline

The timeline of the experiments performed in relation to the MPTP-induced Parkinson's disease mouse model is summarized below.

Table 6. Experimental timeline related to the MPTP-induced Parkinson's disease mouse model.

| Day 0-6 | Establishment of an MPTP-induced Parkinson's disease mouse model by daily MPTP injections for 7 days |
|---|---|
| Day 13 | Injection of an AAV vector encoding Ndst3 (Ndst3) into the MPTP-induced Parkinson's disease mouse model |
| Day 27-37 | Analysis of the Ndst3-treated group and the untreated group (behavioral analysis, etc.) |
| Day 37-39 | Analysis of the Ndst3-treated group and the untreated group (post-sacrifice histological analysis, etc.) |

**2.4. Confirmation of the Therapeutic Effect of Ndst3 on Parkinson's Disease - in vitro**

Analysis of neuronal marker (TUJ1, MAP2) expression (1) - Immunofluorescence staining analysis

**(1) Method**

As described above, after treating the primary dopaminergic neuron culture dish with the AAV vector encoding Ndst3 (treatment with Ndst3) (for the treated group), the cells were cultured for 10 days. Thereafter, for fixation of the cells, the cells were washed twice with PBS for 5 minutes each, and 4% paraformaldehyde (Thermo Fisher) was added to the cell culture dish, followed by incubation at room temperature for 10 minutes. The cells were then washed three times with PBS for 5 minutes each. For blocking, 1% BSA + PBST (PBS + 0.1% Tween 20) was added to the dish and incubated at room temperature for 30 minutes. For immunofluorescence staining analysis, a Tuj1 antibody (Abcam; dilution concentration 1:1000) and a Map2 antibody (Cell Signaling; 1:200) were used as primary antibodies. The primary antibodies, anti-Tuj1 antibody and anti-Map2 antibody, were added to 1% BSA + PBST and incubated overnight at 4 °C. After washing three times with PBS for 5 minutes each, a secondary antibody, Alexa Fluor™ 488 antibody (Thermo Fisher; 1:1000), was added to 1% BSA + PBST and incubated for 1 hour at room temperature in the dark. After washing three times with PBS for 5 minutes each, DAPI staining was performed.

**(2) Results**

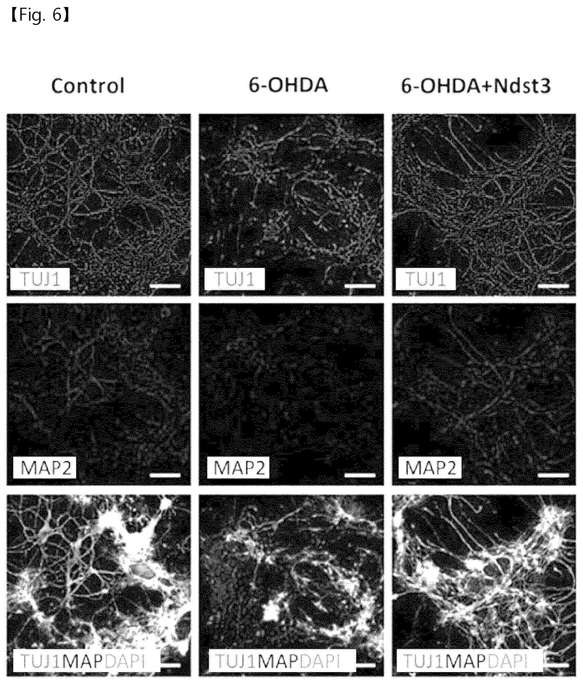

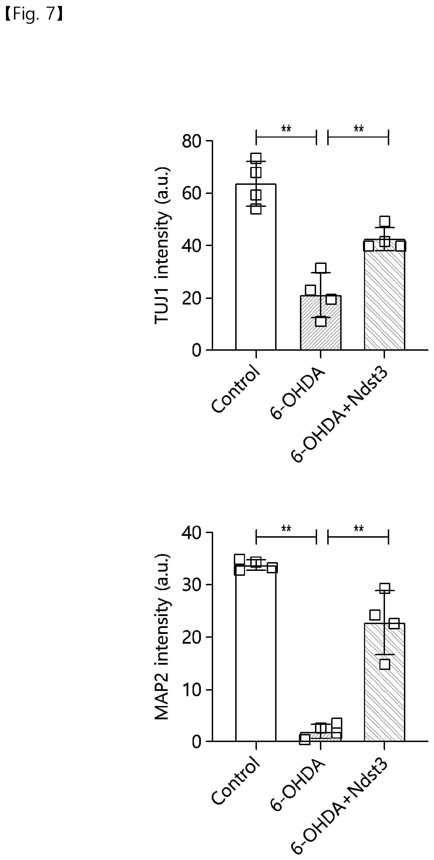

The results confirming the dopaminergic neuronal markers (Tuj1 and Map2) in the control group, the 6-OHDA-treated group (Ndst3-untreated group), and the Ndst3-treated group (6-OHDA and Ndst3-treated group) are shown in Fig. 6 and Fig. 7. Fig. 6 shows images confirming the expression of neuronal markers in each group through immunofluorescence staining. Fig. 7 shows the quantified results of the immunofluorescence staining analysis. According to the analysis results, compared to the 6-OHDA-treated group, the Ndst3-treated group showed increased intensity of the neuronal markers Tuj1 and Map2. The increase in the neuronal markers Tuj1 and Map2 indicates an increase in neuronal cells. The increased levels of Tuj1 and Map2 observed in the NDST3-treated group compared with the 6-OHDA-treated group indicate that neuronal cells increased as a result of NDST3 treatment.

Analysis of dopaminergic neuronal marker (TH) expression (2) - Western blot analysis

**(1) Method**

As described above, after treating the primary dopaminergic neuron culture dish with Ndst3, the cells were cultured for 10 days. The cells were washed twice with PBS (Gibco) for 5 minutes each. Thereafter, a lysis buffer [RIPA buffer (Sigma, R0278) and 1× protease inhibitor cocktail (GenDEPOT, P3100)] was added to the dish, and the cells were collected using a scraper. The collected cells were transferred to an Eppendorf tube and vortexed for 15 seconds, followed by centrifugation at 14,000 rpm for 10 minutes. The supernatant was obtained from the centrifuged sample and mixed with 5× loading dye to prepare a western blot sample. The sample was loaded onto an acrylamide gel and electrophoresis was performed. The proteins separated on the gel were then transferred onto a membrane. The membrane containing the transferred proteins was blocked with 5% skim milk (in TBST). Thereafter, a primary antibody, Anti-TH antibody (1:1000, Millipore), was added in 5% skim milk (in TBST) and incubated overnight at 4 °C. After washing three times with TBST for 10 minutes each, a secondary antibody, HRP (1:1000, Thermo Fisher), was incubated for 1 hour at room temperature. After washing three times with TBST for 10 minutes each, detection was performed using ECL buffer (GenDEPOT, W3652-020).

**(2) Results**

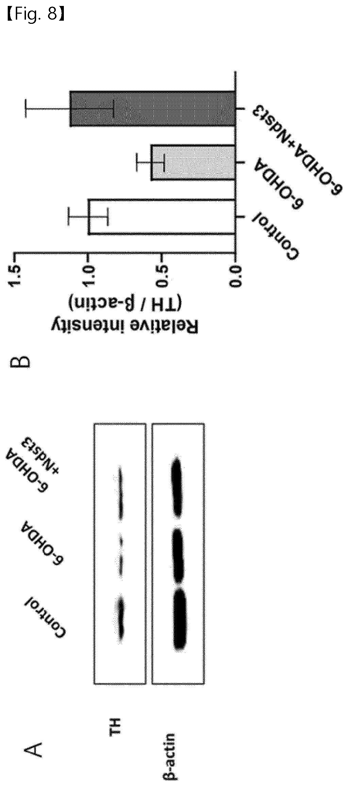

The results confirming the dopaminergic neuronal marker (Tyrosine Hydroxylase; TH) in the control group, the 6-OHDA-treated group (Ndst3-untreated group), and the Ndst3-treated group (6-OHDA and Ndst3-treated group) are shown in Fig. 8. Fig. 8A shows the results of confirming the expression of the neuronal marker TH in the control group (control), the 6-OHDA-treated group (6-OHDA), and the Ndst3-treated group (6-OHDA+Ndst3) by western blot analysis. Fig. 8B is a graph quantifying the results of Fig. 8A. According to the analysis results, the Ndst3-treated group showed a higher expression level of the dopaminergic neuronal marker TH compared to the 6-OHDA-treated group (Fig. 8). The increased expression of the dopaminergic neuronal marker TH in the NDST3-treated group compared with the 6-OHDA-treated group indicates that dopaminergic neurons increased as a result of NDST3 treatment.

Analysis of action potential and resting membrane potential

**(1) Method**

Action potential and resting membrane potential analyses were performed on groups cultured for 10 days after treatment of the primary dopaminergic neuron culture dish with Ndst3. Whole-cell electrophysiological recordings of neurons were measured in artificial cerebrospinal fluid (aCSF; in mM: 124 NaCl, 2.5 KCl, 1.2 $NaH_2PO_4$, 24 $NaHCO_3$, 5 HEPES, 12.5 glucose, 2 $MgSO_4$, and 2 $CaCl_2$, pH 7.4). Electrophysiological recordings were obtained using a Multiclamp 700B amplifier (Molecular Devices, Sunnyvale, CA), DigiDATA (Molecular Devices, Sunnyvale, CA), and pClamp software (version 10.6, Molecular Devices). Patch pipettes with a resistance of 4-8 MΩ were used and were filled with an internal solution (in mM: 120 K-gluconate, 10 KCl, 2 Mg-ATP, 0.5 Na-GTP, 0.5 EGTA, 20 HEPES, and 10 phosphocreatine, pH 7.3). Neuronal action potentials were measured using current steps ranging from 0 pA to 140 pA in increments of 20 pA per step.

**(2) Results**

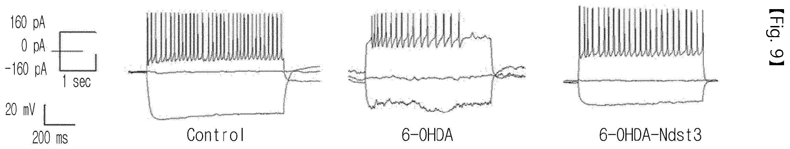

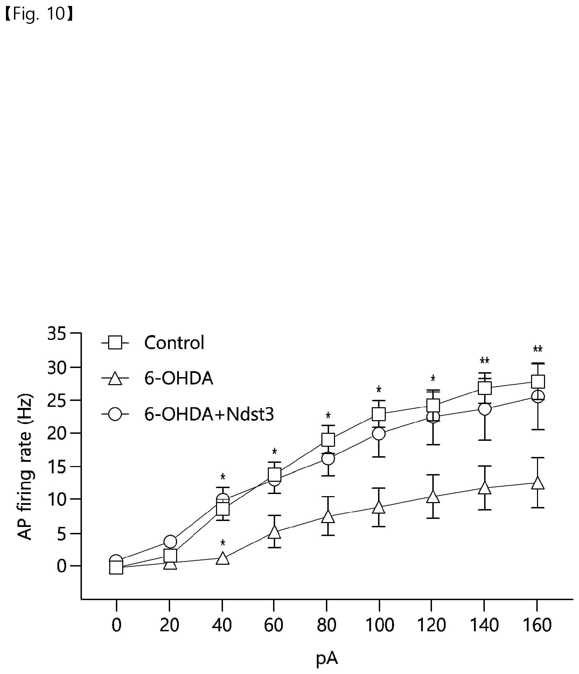

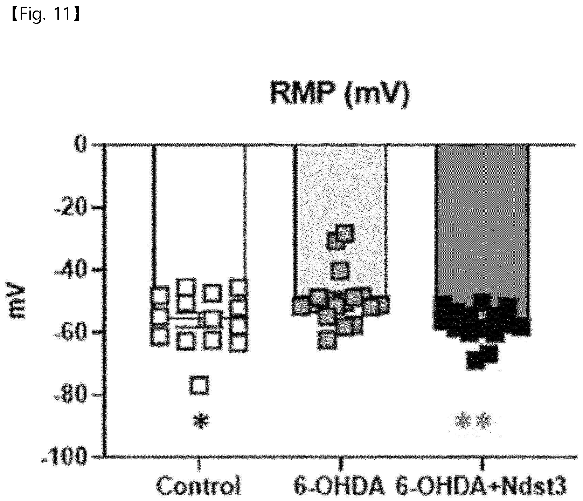

The results of the analysis of action potential and resting membrane potential in the control group, the 6-OHDA-treated group (Ndst3-untreated group), and the Ndst3-treated group (6-OHDA and Ndst3-treated group) are shown in Fig. 9 to Fig. 11. Fig. 9 shows the results of analyzing action potentials (AP; action potential) in each group. Fig. 10 shows the results of analyzing the action potential firing rate (AP firing rate) in each group. Fig. 11 shows the results of analyzing the resting membrane potential in each group. According to the electrophysiological analysis, the Ndst3-treated group exhibited an increased AP firing frequency compared to the 6-OHDA-treated group. Furthermore, the resting membrane potential in the Ndst3-treated group was observed to recover to a level similar to that of the control group. The increased firing rate of action potentials and the recovery of the resting membrane potential in the Ndst3-treated group compared with the 6-OHDA-treated group indicate that neuronal function was restored by Ndst3.

**2.5. Confirmation of the Therapeutic Effect of Ndst3 on Parkinson's Disease - in vivo**

Analysis of dopaminergic neuronal marker (TH) expression in a 6-OHDA-induced mouse model - Immunofluorescence staining analysis

**(1) Method**

Mice in the control group, the 6-OHDA-induced Parkinson's disease group (6-OHDA-treated and Ndst3-untreated), and the Ndst3-treated group (6-OHDA-treated and Ndst3-treated) were sacrificed 23-25 days after 6-OHDA injection. Thereafter, the brains of the mice were extracted and fixed in 4% paraformaldehyde at 4 °C overnight for tissue fixation. The brain regions including the substantia nigra were subjected to coronal sectioning. After sectioning, the tissues were transferred to a dish and washed twice with PBS for 5 minutes each. For permeabilization, PBS containing 0.1% Triton X was added to the container containing the tissues and incubated at room temperature for 10 minutes. For blocking, 1% BSA + PBST (PBS + 0.1% Tween 20) was added to the dish and incubated at room temperature for 30 minutes. For immunofluorescence staining analysis, an anti-TH antibody (Millipore, 1:1000) was used as the primary antibody. The anti-TH antibody was added to 1% BSA + PBST (PBS + 0.1% Tween 20) and incubated overnight at 4 °C. After washing three times with PBS for 5 minutes each, a secondary antibody, Alexa Fluor™ 488 antibody (Thermo Fisher, 1:1000), was added to 1% BSA + PBST (PBS + 0.1% Tween 20) and incubated for 1 hour at room temperature in the dark. After washing three times with PBS for 5 minutes each, DAPI staining was performed.

**(2) Results**

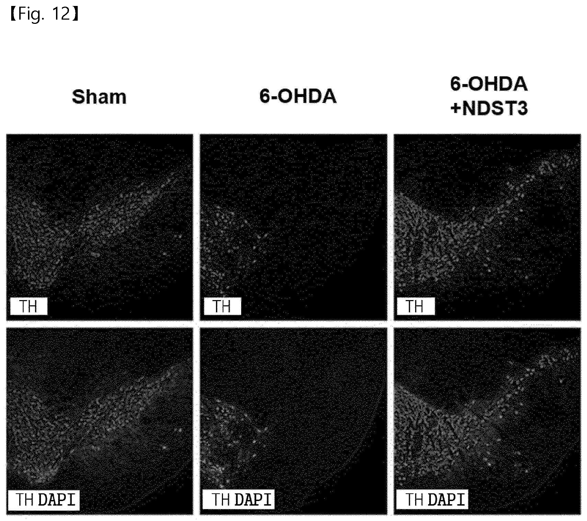

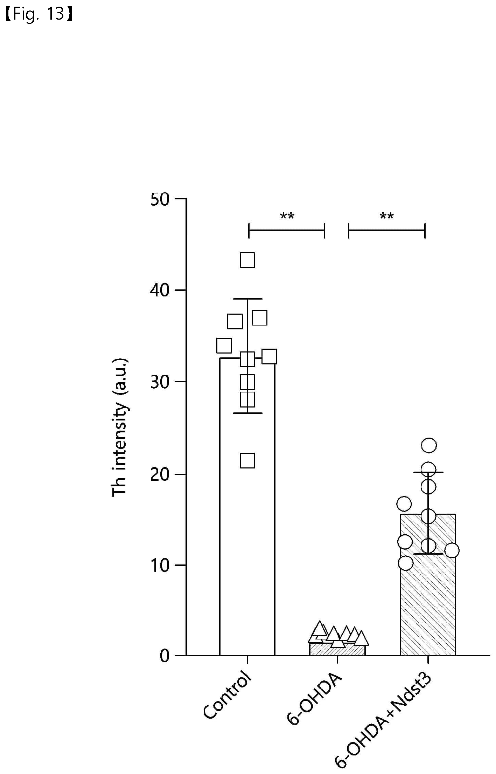

Fig. 12 and Fig. 13 show the results of confirming the expression of the neuronal marker TH by immunofluorescence staining after treatment with Ndst3 in a 6-OHDA-induced Parkinson's disease mouse model. Fig. 12 shows the results of confirming the expression of the neuronal marker (TH) in the control group without treatment with 6-OHDA and Ndst3 (indicated as "Sham" in the figure), the 6-OHDA-induced Parkinson's disease mouse group (6-OHDA-treated group; 6-OHDA-treated and Ndst3-untreated; indicated as "6-OHDA" in the figure), and the Ndst3-treated group (6-OHDA-treated and Ndst3-treated; indicated as "6-OHDA + Ndst3" in the figure). Fig. 13 shows quantified results of the expression of the neuronal marker (TH). As a result of treatment with Ndst3 in the Parkinson's disease mouse model, the expression level of the neuronal marker TH (tyrosine hydroxylase) was confirmed to be increased compared with the Parkinson's disease mouse group that was not treated with Ndst3. The increased expression of the neuronal marker TH in the Ndst3-treated group indicates an increase in dopaminergic neurons.

*Analysis of dopaminergic neuronal markers (TH, DAT) expression in a 6-OHDA-induced mouse model - DAB staining analysis*

**(1) Method**

Mice in the control group, the 6-OHDA-induced Parkinson's disease group (6-OHDA-treated and Ndst3-untreated), and the Ndst3-treated group (6-OHDA-treated and Ndst3-treated) were sacrificed 23-25 days after 6-OHDA injection. Thereafter, the brains of the mice were extracted and fixed in 4% paraformaldehyde at 4 °C overnight for tissue fixation. Brain regions including the striatum or substantia nigra were subjected to coronal sectioning. After sectioning, the tissues were transferred to a dish and washed twice with PBS for 5 minutes each. For permeabilization, PBS containing 0.1% Triton X was added and the tissues were incubated at room temperature for 10 minutes. The tissues were then washed three times with PBS for 5 minutes each. For blocking, 1% BSA + PBST (PBS + 0.1% Tween 20) was added and incubated at room temperature for 30 minutes. As primary antibodies, an anti-TH antibody (Millipore, 1:1000) was used for TH expression analysis, and an anti-DAT antibody (Millipore, 1:1000) was used for DAT expression analysis. The primary antibody (anti-TH antibody or anti-DAT antibody) was added to 1% BSA + PBST and incubated overnight at 4 °C. After washing three times with PBS for 5 minutes each, a secondary antibody, horseradish peroxidase (HRP) (Thermo Fisher, 1:1000), was added to 1% BSA + PBST and incubated at room temperature for 1 hour. Color development was then performed using a DAB Substrate Kit (Abcam).

**(2) Results of TH expression analysis**

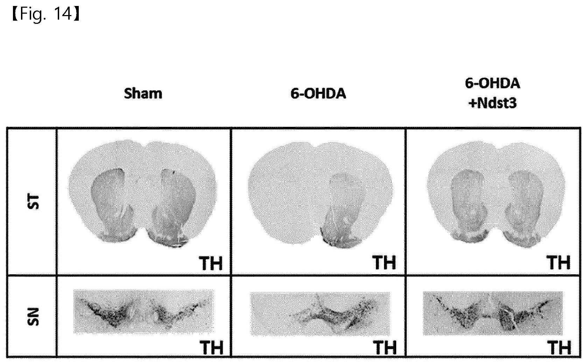

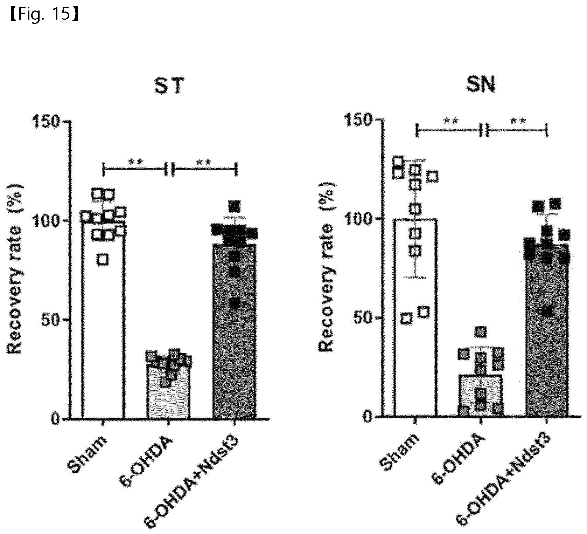

Fig. 14 and Fig. 15 show the results of confirming the expression of the neuronal marker TH in the SN (substantia nigra) and ST (striatum) by immunostaining (DAB staining) after treatment with Ndst3 in the 6-OHDA-induced Parkinson's disease mouse model. Fig. 14 shows the results of confirming the expression of the neuronal marker (TH) in the control group without treatment with 6-OHDA and Ndst3 (healthy control; indicated as "Sham"), the 6-OHDA-induced Parkinson's disease mouse group (6-OHDA-treated and Ndst3-untreated; indicated as "6-OHDA"), and the Ndst3-treated group (6-OHDA-treated and Ndst3-treated; indicated as "6-OHDA + Ndst3"). Fig. 15 shows the quantified results of Fig. 14. As a result of treatment with Ndst3 in the Parkinson's disease mouse model, the expression level of the neuronal marker TH (tyrosine hydroxylase) was confirmed to be increased compared with the Parkinson's disease mouse group that was not treated with Ndst3.

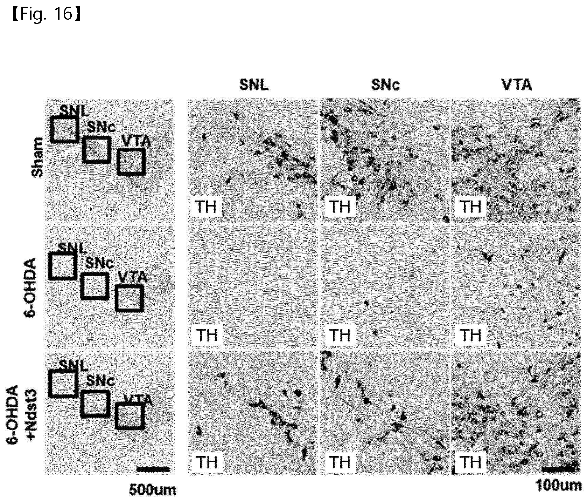

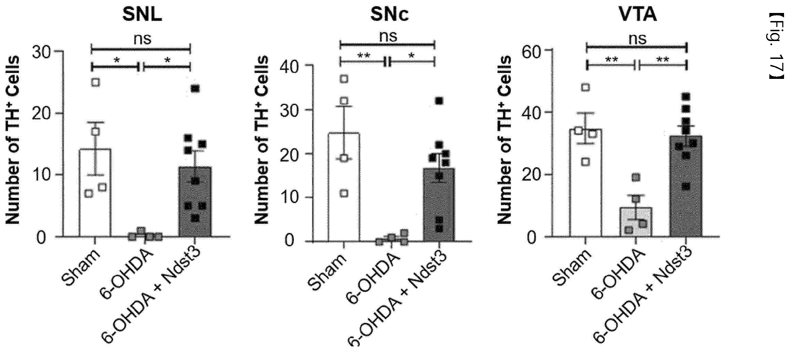

Fig. 16 and Fig. 17 show the results of confirming the expression of the neuronal marker TH in the SNL (lateral substantia nigra), SNc (substantia nigra pars compacta), and VTA (ventral tegmental area) by immunostaining (DAB staining) after treatment with Ndst3 in the 6-OHDA-induced Parkinson's disease mouse model. Fig. 16 shows the results of confirming the expression of the neuronal marker (TH) in each region (SNL, SNc, and VTA) in the control group without treatment with 6-OHDA and Ndst3 (indicated as "Sham"), the 6-OHDA-induced Parkinson's disease mouse group (6-OHDA-treated and Ndst3-untreated; indicated as "6-OHDA"), and the Ndst3-treated group (6-OHDA-treated and Ndst3-treated; indicated as "6-OHDA + Ndst3"). Fig. 17 shows quantified results of Fig. 16. As a result of treatment with Ndst3 in the Parkinson's disease mouse model, the expression of the neuronal marker TH (tyrosine hydroxylase) in each region was confirmed to be increased compared with the Parkinson's disease mouse group that was not treated with Ndst3. The increased expression of the neuronal marker TH in the Ndst3-treated group indicates an increase in dopaminergic neurons.

### (3) Results of DAT Expression Analysis

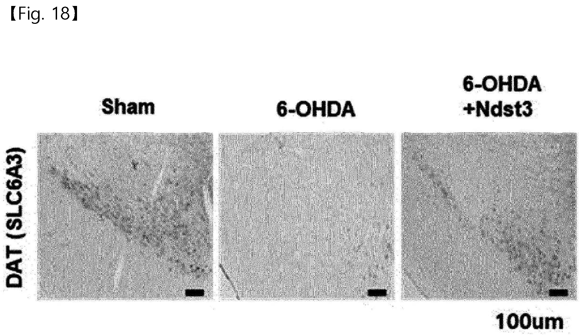

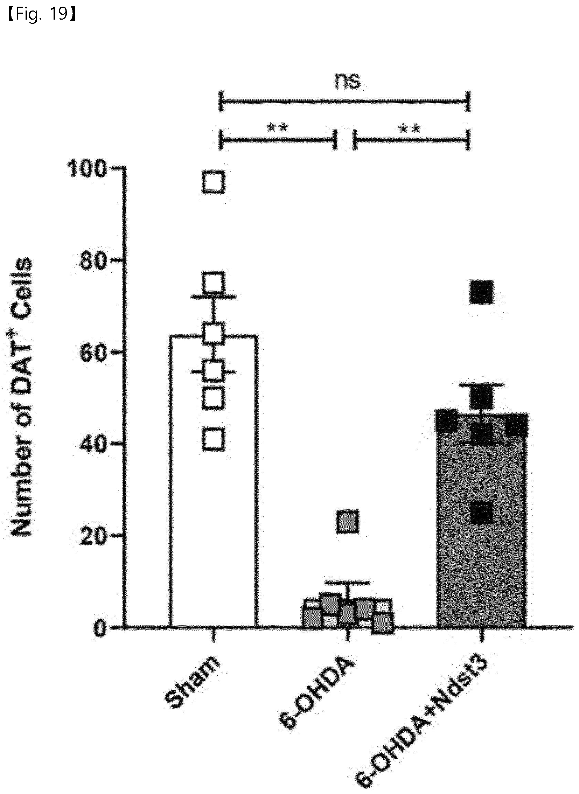

Fig. 18 and Fig. 19 show the results of confirming the expression of the neuronal marker DAT in the SN (substantia nigra) by immunostaining (DAB staining) after treatment with Ndst3 in the 6-OHDA-induced Parkinson's disease mouse model. Fig. 18 shows the results of confirming the expression of the neuronal marker (DAT) in the control group without treatment with 6-OHDA and Ndst3 (indicated as "Sham"), the 6-OHDA-induced Parkinson's disease mouse group (6-OHDA-treated and Ndst3-untreated; indicated as "6-OHDA"), and the Ndst3-treated group (6-OHDA-treated and Ndst3-treated; indicated as "6-OHDA + Ndst3"). Fig. 19 shows quantified results of Fig. 18. As a result of treatment with Ndst3 in the Parkinson's disease mouse model, the expression of the neuronal marker DAT (dopamine transporter) was confirmed to be increased compared with the Parkinson's disease mouse group that was not treated with Ndst3. The increased expression of the neuronal marker DAT in the Ndst3-treated group indicates an increase in dopaminergic neurons.

*Expression Analysis of NDST3 in a 6-OHDA-Induced Mouse Model - qPCR Analysis*

### (1) Method

Mice in the control group, the 6-OHDA-induced Parkinson's disease group (6-OHDA-treated and Ndst3-untreated), and the Ndst3-treated group (6-OHDA-treated and Ndst3-treated) were sacrificed 23-25 days after 6-OHDA injection. Thereafter, a region including the substantia nigra of the mouse brain was collected. RNA was extracted from the tissues according to the protocol using an RNA extraction kit (Qiagen, 74104). The extracted RNA was used for cDNA synthesis using AccuPower® CycleScript™ RT PreMix & Master Mix (Bioneer). qPCR was performed using SYBR Green Realtime PCR Master Mix (Enzynomics). Information on the primers used is disclosed in Table 7 below.

Table 7. Primer Information

| Name | Sequence | Sequence No. |
|------|----------|--------------|
| mGAPDH-F | TGATGGGTGTGAACCACGAG | SEQ ID NO: 12 |
| mGAPDH-R | GGTCATGAGCCCTTCCACAA | SEQ ID NO: 13 |
| mNdst3-F | GGAAACGACCGACTGGGATT | SEQ ID NO: 14 |
| mNdst3-R | TACTTGTGAGCCAGCTGAGC | SEQ ID NO: 15 |

### (2) Results

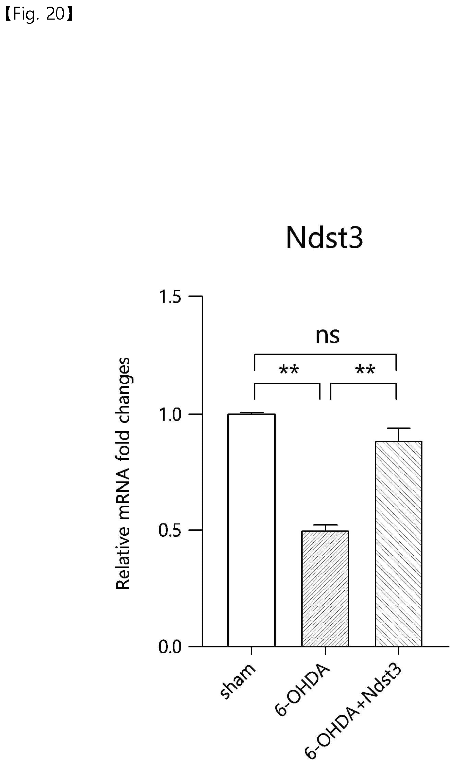

Fig. 20 shows the results of confirming the expression of Ndst3 by quantitative realtime polymerase chain reaction (qRT-PCR) after treatment with Ndst3 in a 6-OHDA-induced Parkinson's disease mouse model. Fig. 20 shows the results of confirming Ndst3 expression in a control group (indicated as "Sham" in the drawing), a 6-OHDA-induced Parkinson's disease mouse model (indicated as "6-OHDA" in the drawing), and an Ndst3-treated group (indicated as "6-OHDA + Ndst3" in the drawing). As a result of treating Ndst3 in the 6-OHDA-induced Parkinson's disease mouse model, it was confirmed that the expression level of Ndst3 was higher than that in the 6-OHDA-treated group (Fig. 20). From these results, it was confirmed that treatment with Ndst3 in the Parkinson's disease-induced mouse model increases the expression of Ndst3 reduced in the Parkinson's disease-induced mouse.

*Expression Analysis of Dopaminergic Neuron Markers (TH, DAT) in an MPTP-Induced Mouse Model - DAB Staining Analysis*

**(1) Method**

Mice in a control group, an MPTP-induced Parkinson's disease group (MPTP-treated and Ndst3-untreated), and an Ndst3-treated group (MPTP-treated and Ndst3-treated) were sacrificed at 37-39 days. Thereafter, the brains of the mice were extracted and fixed overnight at 4 °C in 4% paraformaldehyde for tissue fixation. Regions including the striatum or substantia nigra of the brain were subjected to coronal sectioning. After sectioning, the tissues were transferred to a dish and washed twice with PBS for 5 minutes each. For permeabilization, PBS containing 0.1% Triton X was added and incubated at room temperature for 10 minutes. The tissues were then washed three times with PBS for 5 minutes each. For blocking, 1% BSA + PBST (PBS + 0.1% Tween 20) was added and incubated at room temperature for 30 minutes. As primary antibodies, anti-TH antibody (Millipore, 1:1000) was used for analysis of TH expression, and anti-DAT antibody (Millipore, 1:1000) was used for analysis of DAT expression. The primary antibody (anti-TH antibody or anti-DAT antibody) was added to 1% BSA + PBST and incubated overnight at 4 °C. After washing three times with PBS for 5 minutes each, a secondary antibody, horseradish peroxidase (HRP) (Thermo Fisher, 1:1000), was added to 1% BSA + PBST and incubated for 1 hour at room temperature. Color development was performed using a DAB Substrate Kit (Abcam).

**(2) Results of TH Expression Analysis**

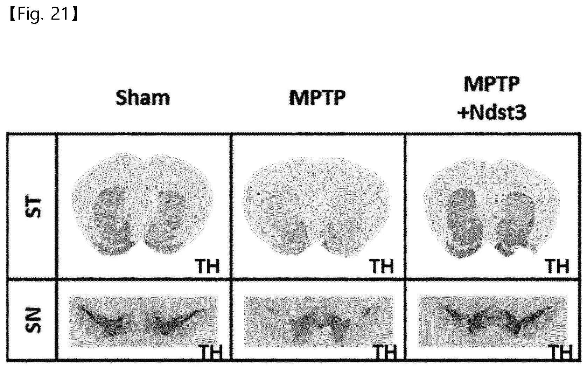

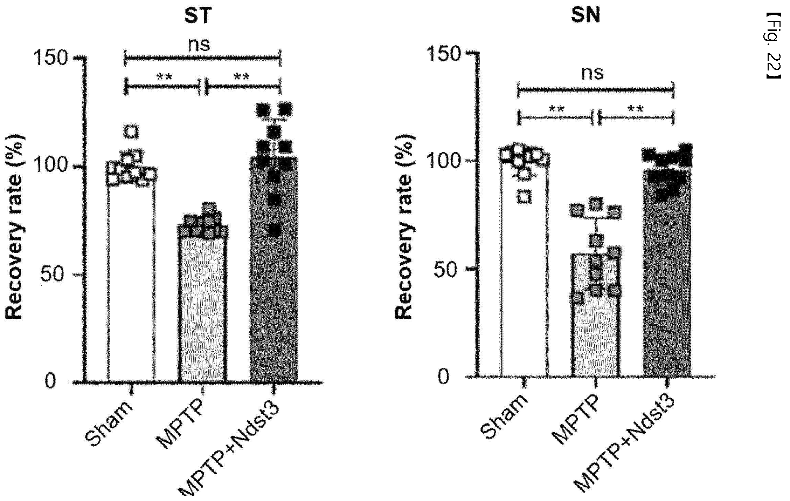

Fig. 21 and Fig. 22 show the results of confirming the expression of the neuronal marker TH in the ST (striatum) and SN (substantia nigra) by immunostaining (DAB staining) after treatment with Ndst3 in an MPTP-induced Parkinson's disease mouse model. Fig. 21 shows the results of confirming the expression of the neuronal marker (TH) in a control group (indicated as "Sham" in the drawing), an MPTP-induced Parkinson's disease mouse group (indicated as "MPTP" in the drawing), and an Ndst3-treated group (indicated as "MPTP + Ndst3" in the drawing). Fig. 22 shows quantified results of Fig. 21. As a result of treatment with Ndst3 in the MPTP-induced Parkinson's disease mouse model, the expression level of the neuronal marker TH was confirmed to be increased compared with the MPTP-treated group. The increased expression of the neuronal marker TH in the Ndst3-treated group indicates an increase in dopaminergic neurons.

(3) Results of DAT Expression Analysis

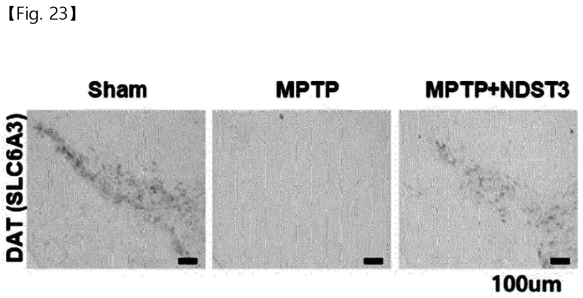

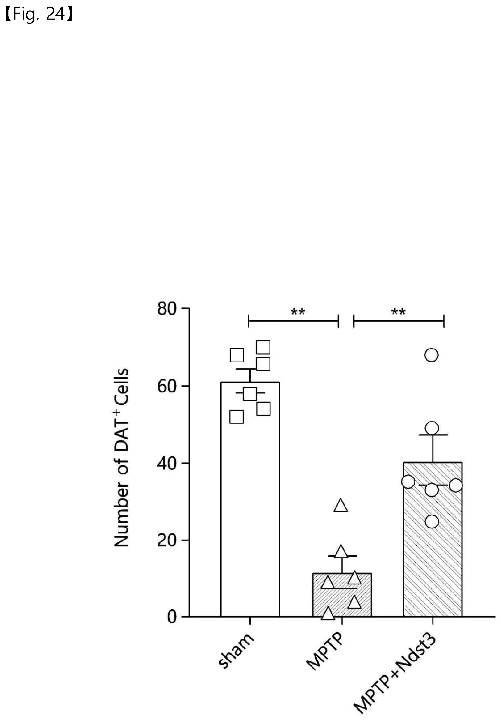

Fig. 23 and Fig. 24 show the results of confirming the expression of the neuronal marker DAT in the SN (substantia nigra) by immunostaining (DAB staining) after treatment with Ndst3 in an MPTP-induced Parkinson's disease mouse model. Fig. 23 shows the results of confirming the expression of the neuronal marker (DAT) in a control group, an MPTP-induced Parkinson's disease mouse group, and an Ndst3-treated group. Fig. 24 shows a graph quantifying the results of Fig. 23. As a result of treatment with Ndst3 in the MPTP-induced Parkinson's disease mouse model, the expression of the neuronal marker DAT was confirmed to be increased compared with the MPTP-treated group. The increased expression of the neuronal marker DAT in the NDST3-treated group indicates an increase in dopaminergic neurons.

*Behavioral Analysis - Rotation Test*

The inventors of the present application performed a rotation test after treating Ndst3 in a 6-OHDA-induced Parkinson's disease mouse model. At least 21 days after the day on which 6-OHDA was administered to the mice, mice in a control group, a 6-OHDA-treated group (6-OHDA-treated and Ndst3-untreated), and an Ndst3-treated group (6-OHDA-treated and Ndst3-treated) were placed in a cylindrical cylinder and observed for 5 minutes. The direction of rotation was quantified to calculate rotation.

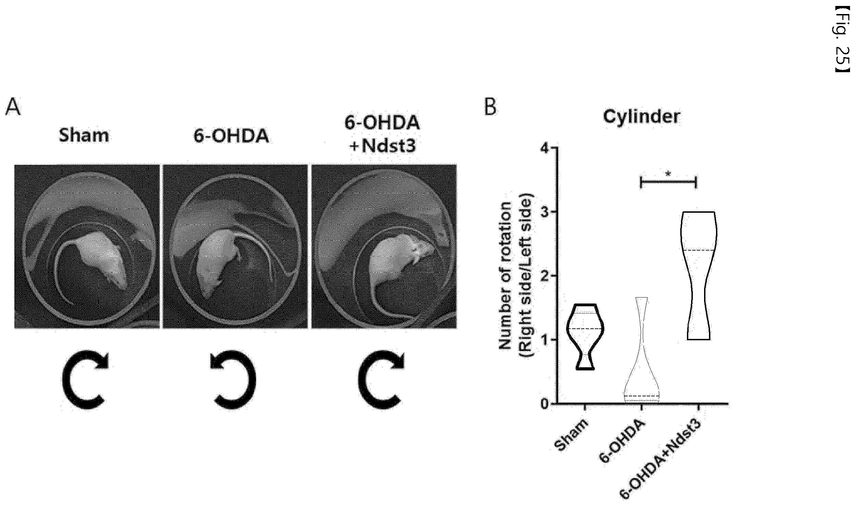

The results of the rotation test are disclosed in Fig. 25. Fig. 25A shows photographs of the rotation test performed in mice of each group. Fig. 25B shows a graph quantifying the results of the rotation test. As a result of the rotation test, it was confirmed that the Ndst3-treated group recovered directional behavior to a level similar to that of the control group.

Behavioral Analysis - Tail Suspension Test

The inventors of the present application performed a tail suspension test after treating Ndst3 in a 6-OHDA-induced Parkinson's disease mouse model. At least 21 days after the day on which 6-OHDA was administered to the mice, mice in a control group, a 6-OHDA-treated group (6-OHDA-treated and Ndst3-untreated), and an Ndst3-treated group (6-OHDA-treated and Ndst3-treated) were set in a tail suspension apparatus and observed for 5 minutes. The direction of movement of the mice was quantified to calculate behavioral activity.

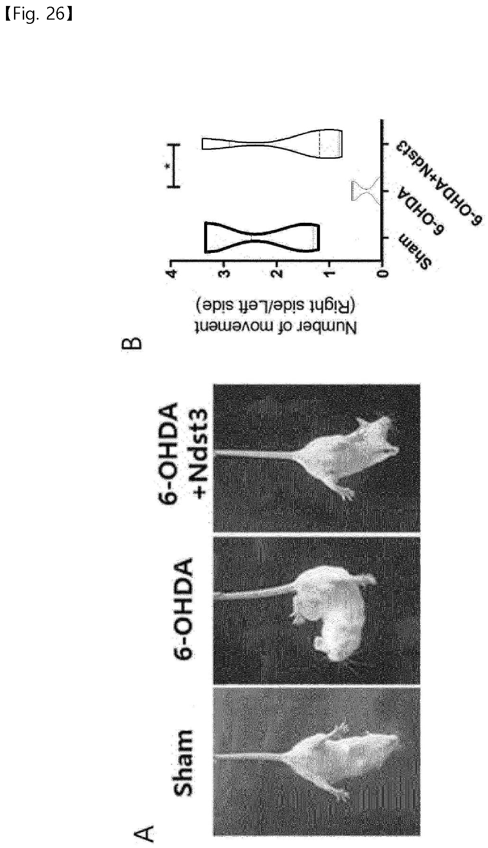

The results of the tail suspension test for the control group, the 6-OHDA-treated group, and the Ndst3-treated group are disclosed in Fig. 26. Fig. 26A shows photographs of the tail suspension test performed in mice of each group. Fig. 26B shows a graph quantifying the results of the tail suspension test. As a result of the tail suspension test, it was confirmed that the Ndst3-treated group recovered behavioral activity to a level similar to that of the control group.

**2.6. Confirmation of Therapeutic Effect of Ndst3 on Parkinson's Disease by Intrathecal Administration - in vivo**

Intrathecal Injection of an AAV Vector Encoding Ndst3 (Administration into the Cisterna Magna)

An AAV vector encoding Ndst3 obtained in "1. Preparation of an AAV vector encoding Ndst3" was injected into the cisterna magna (subarachnoid cerebellomedullary cistern) of the 6-OHDA-induced Parkinson's disease mouse model prepared in "2.2. Establishment of a 6-OHDA-induced Parkinson's disease mouse model and administration of an AAV vector encoding Ndst3". Specifically, 5 days after preparation of the Parkinson's disease model, $1 \times 10^{10}$ GC/head of the AAV vector encoding Ndst3 was injected into the cisterna magna of the mouse.

Behavioral Analysis - Cylinder Test

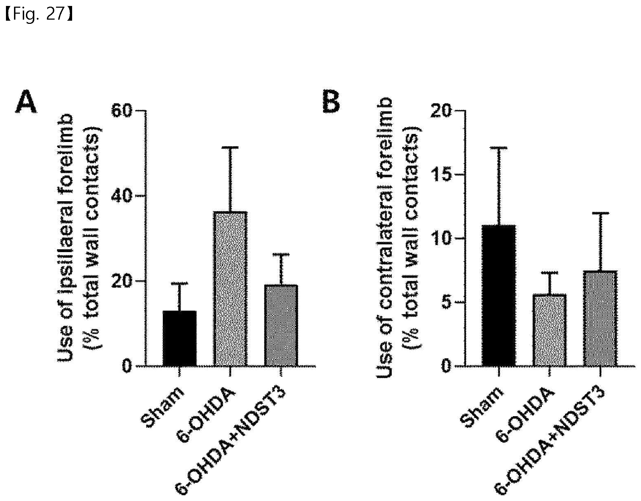

At least 21 days after the day on which 6-OHDA was administered to the mice, mice in a control group, a 6-OHDA-treated group (6-OHDA-treated and Ndst3-untreated), and an Ndst3-treated group (6-OHDA-treated and Ndst3-treated) were placed in a cylinder and observed for 5 minutes. The behavioral activity of the mice was analyzed by measuring the number of uses of the ipsilateral forelimb and the contralateral forelimb on the wall of the cylinder.
The results of the behavioral analysis of the control group, the 6-OHDA-treated group, and the Ndst3-treated group are disclosed in Fig. 27. Fig. 27A shows a graph quantifying the number of uses of the ipsilateral forelimb in each group. Fig. 27B shows a graph quantifying the number of uses of the contralateral forelimb in each group. As a result of the cylinder test, it was confirmed that the Ndst3-treated group showed a recovery in behavioral activity compared with the 6-OHDA-treated group.
The above results demonstrate that Ndst3 is effective in recovering symptoms of Parkinson's disease and in treating Parkinson's disease.

**Claims**

1. A pharmaceutical composition for treating Parkinson's disease, comprising: a vector comprising a nucleic acid encoding an NDST3 protein.

2. The pharmaceutical composition according to claim 1, wherein the vector is a viral vector.

3. The pharmaceutical composition according to claim 2, wherein the viral vector is selected from the group consisting of retrovirus, lentivirus, adenovirus, adeno-associated virus, vaccinia virus, poxvirus, HIV (human immunodeficiency virus), MLV (Murine leukemia virus), ASLV (avian sarcoma/leukosis virus), SNV (spleen necrosis virus), RSV (Rous sarcoma virus), MMTV (mouse mammary tumor virus), herpes simplex virus, episomal vector, and herpes virus.

4. The pharmaceutical composition according to claim 2, wherein the viral vector is an adeno-associated virus (AAV) vector.

5. The pharmaceutical composition according to claim 4, wherein the AAV is selected from the group consisting of AAV1, AAV2, AAV3, AAV4, AAV5, AAV6, AAV7, AAV8, AAV9, AAV10, AAV11, AAV12, AAV13, AAV-DJ, AAV-DJ/8, AAV-Rh10, AAV-retro, AAV-PHP.B, AAV-PHP.eB, and AAV-PHP.S.

6. The pharmaceutical composition according to claim 4, wherein the AAV is AAV9.

7. The pharmaceutical composition according to claim 1, wherein the vector is a non-viral vector.

8. The pharmaceutical composition according to claim 7, wherein the non-viral vector is selected from the group consisting of a plasmid, phage, naked DNA, a DNA-RNA lipid complex, liposome, nanoparticle, DNA-polymer complex, mRNA, artificial chromosome, and cosmid.

9. The pharmaceutical composition according to claim 1, wherein the pharmaceutical composition further comprises one or more pharmaceutically acceptable additional components.

10. The pharmaceutical composition according to claim 9, wherein the pharmaceutically acceptable additional component is independently selected from the group consisting of a carrier, excipient, diluent, and preservative.

11. The pharmaceutical composition according to claim 1, wherein the NDST3 protein is a human NDST3 protein.

12. The pharmaceutical composition according to claim 1, wherein the human NDST3 protein is a wild-type human NDST3 protein or a variant thereof.

13. The pharmaceutical composition according to claim 11, wherein the human NDST3 protein comprises an amino acid sequence of SEQ ID NO: 1 or an amino acid sequence having at least 80% and less than 100% sequence identity thereto.

14. The pharmaceutical composition according to claim 11, wherein the human NDST3 protein comprises the amino acid sequence of SEQ ID NO: 1.

15. The pharmaceutical composition according to claim 11, wherein the human NDST3 protein is a wild-type human NDST3 protein or a functional equivalent thereof.

16. The pharmaceutical composition according to claim 11, wherein the human NDST3 protein is a variant of a wild-type human NDST3 protein, wherein the variant comprises one or more conservative substitutions of amino acid residues in the wild-type human NDST3 protein having the amino acid sequence of SEQ ID NO: 1, thereby the human NDST3 protein comprises an amino acid sequence having at least 80% and less than 100% sequence identity to the amino acid sequence of the wild-type human NDST3 protein.

17. The pharmaceutical composition according to claim 1, wherein the nucleic acid encoding the NDST3 protein comprises an NDST3 protein-encoding region, wherein the NDST3 protein-encoding region has a nucleic acid sequence encoding the NDST3 protein.

18. The pharmaceutical composition according to claim 17, wherein the nucleic acid sequence encoding the NDST3 protein is a DNA sequence, an RNA sequence, or a DNA/RNA hybrid sequence.

19. The pharmaceutical composition according to claim 17, wherein the nucleic acid sequence encoding the NDST3 protein comprises a full-length sequence of an NDST3 gene, an open reading frame (ORF) sequence of the NDST3 gene, a coding sequence (CDS) of the NDST3 gene, or a cDNA sequence of the NDST3 gene.

20. The pharmaceutical composition according to claim 17, wherein the nucleic acid sequence encoding the NDST3 protein comprises a full-length sequence of a wild-type human NDST3 gene, an open reading frame (ORF) sequence of the wild-type human NDST3 gene, a coding sequence (CDS) of the wild-type human NDST3 gene, or a cDNA sequence of the wild-type human NDST3 gene.

21. The pharmaceutical composition according to claim 17, wherein the nucleic acid sequence encoding the NDST3 protein comprises the nucleic acid sequence of SEQ ID NO: 7 or a nucleic acid sequence having at least 80% and less than 100% sequence identity thereto.

22. The pharmaceutical composition according to claim 17, wherein the nucleic acid sequence encoding the NDST3 protein comprises the nucleic acid sequence of SEQ ID NO: 7.

23. The pharmaceutical composition according to claim 17, wherein the nucleic acid encoding the NDST3 protein further comprises one or more additional elements.

24. The pharmaceutical composition according to claim 23, wherein each of the one or more additional elements is independently selected from the group consisting of a promoter, enhancer, polyadenylation signal, Kozak consensus sequence, inverted terminal repeat (ITR), long terminal repeat (LTR), terminator, origin of replication, multicloning site (MCS), internal ribosome entry site (IRES), a nuclear localization signal-encoding sequence, and 2A self-cleaving peptide.

25. The pharmaceutical composition according to claim 17, wherein the nucleic acid encoding the NDST3 protein further comprises a promoter, wherein the promoter is operably linked to the NDST3 protein-encoding region.

26. The pharmaceutical composition according to claim 25, wherein the promoter is an SV40 early promoter, an LTR (mouse mammary tumor virus long terminal repeat) promoter, an Ad MLP (adenovirus major late) promoter, an HSV (herpes simplex virus) promoter, a CMV (cytomegalovirus) promoter, an RSV (Rous sarcoma virus) promoter, a U6 promoter, a CBA promoter, a PGK promoter, an NG2 promoter, an NES promoter, a GFAP promoter, a CaMKII promoter, an NSE promoter, a SYN1 promoter, or a CAG promoter.

27. A pharmaceutical composition for treating Parkinson's disease, comprising:

a viral vector encoding an NDST3 protein,
wherein the viral vector encoding the NDST3 protein comprises a nucleic acid encoding the NDST3 protein.

28. The pharmaceutical composition according to claim 27, wherein the NDST3 protein comprises the amino acid sequence of SEQ ID NO: 1 or an amino acid sequence having at least 80% and less than 100% sequence identity thereto.

29. The pharmaceutical composition according to claim 27, wherein the nucleic acid encoding the NDST3 protein comprises an NDST3 protein-encoding region, wherein the NDST3 protein-encoding region comprises the nucleic acid sequence of SEQ ID NO: 7 or a nucleic acid sequence having at least 80% and less than 100% sequence identity thereto.

30. The pharmaceutical composition according to claim 29, wherein the nucleic acid encoding the NDST3 protein further comprises a promoter, wherein the promoter is operably linked to the NDST3 protein-encoding region.

31. The pharmaceutical composition according to claim 30, wherein the promoter is an SV40 early promoter, an LTR (mouse mammary tumor virus long terminal repeat) promoter, an Ad MLP (adenovirus major late) promoter, an HSV (herpes simplex virus) promoter, a CMV (cytomegalovirus) promoter, an RSV (Rous sarcoma virus) promoter, a U6 promoter, a CBA promoter, a PGK promoter, an NG2 promoter, an NES promoter, a GFAP promoter, a CaMKII promoter, an NSE promoter, a SYN1 promoter, or a CAG promoter.

32. The pharmaceutical composition according to claim 27, wherein the viral vector is an AAV vector.

33. The pharmaceutical composition according to claim 27, wherein the viral vector is an AAV9 vector.

34. A method for treating Parkinson's disease in a subject, comprising: administering to the subject the pharmaceutical composition according to any one of claims 1 to 33.

35. The method according to claim 34, wherein the subject is a subject having Parkinson's disease.

36. The method according to claim 34, wherein the pharmaceutical composition is administered to the subject via a parenteral route.

37. The method according to claim 34, wherein the pharmaceutical composition is administered intravenously, subcutaneously, intramuscularly, intradermally, intraperitoneally, intra-arterially, mucosally, spinally, intrathecally, nasally, or intracerebrally.

【Fig. 1】

【Fig. 2】

【Fig. 3】

【Fig. 4】

【Fig. 5】

【Fig. 6】

【Fig. 7】

【Fig. 8】

【Fig. 9】

【Fig. 10】

【Fig. 11】

【Fig. 12】

【Fig. 13】

【Fig. 14】

【Fig. 15】

【Fig. 16】

【Fig. 17】

【Fig. 18】

【Fig. 19】

【Fig. 20】

Ndst3

【Fig. 21】

【Fig. 22】

【Fig. 23】

【Fig. 24】

【Fig. 25】

【Fig. 26】

【Fig. 27】

# EP 4 782 003 A1

## INTERNATIONAL SEARCH REPORT

| International application No. |
| --- |
| **PCT/KR2024/001916** |

| A. | CLASSIFICATION OF SUBJECT MATTER |
| --- | --- |

**A61K 38/45**(2006.01)i; **A61P 25/16**(2006.01)i; **A61K 38/17**(2006.01)i; **A61P 25/28**(2006.01)i; **A61K 38/50**(2006.01)i; **A61P 25/00**(2006.01)i

According to International Patent Classification (IPC) or to both national classification and IPC

| B. | FIELDS SEARCHED |
| --- | --- |

Minimum documentation searched (classification system followed by classification symbols)

A61K 38/45(2006.01); A61K 35/76(2006.01); C12N 15/113(2010.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 파킨슨 병(Parkinson's disease), 신경퇴행성 질환(neurodegenerative disease), 흑색질(substantia nigra), 도파민성 뉴런(dopaminergic neuron), NDST3(N-Deacetylase and N-sulfotransferase 3), 유전자 치료(gene therapy), 바이러스 벡터(virus vector), 아데노-연관 바이러스 벡터(adeno-associated virus; AAV vector)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| A | TANG, Q. et al. Tubulin deacetylase NDST3 modulates lysosomal acidification: Implications in neurological diseases. Bioessays. 2022, vol. 44, no. 11, article no. 2200110, inner pp. 1-11.<br>See abstract; and inner pages 5-7. | 1-33 |
| A | TANG, Q. et al. NDST3 deacetylates α-tubulin and suppresses V-ATPase assembly and lysosomal acidification. The EMBO Journal. 2021, vol. 40, no. 19, article no. e107204, inner pp. 1-18.<br>See abstract; and inner pages 1-12. | 1-33 |
| A | LENCZ, T. et al. Genome-wide association study implicates NDST3 in schizophrenia and bipolar disorder. Nature Communications. 2013, vol. 4, no. 2739, inner pp. 1-10.<br>See abstract; and inner pages 1-7. | 1-33 |
| A | KR 10-2022-0099944 A (FUNDACION PARA LA INVESTIGACION MEDICA APLICADA et al.) 14 July 2022 (2022-07-14)<br>See abstract; and claims 1-34. | 1-33 |

| ✓ Further documents are listed in the continuation of Box C. | ✓ See patent family annex. |
| --- | --- |

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **14 June 2024** | **17 June 2024** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**EP 4 782 003 A1**

**INTERNATIONAL SEARCH REPORT**

International application No.

**PCT/KR2024/001916**

**C.    DOCUMENTS CONSIDERED TO BE RELEVANT**

| Category* | Citation of document, with indication, where appropriate, of the relevant passages | Relevant to claim No. |
|---|---|---|
| A | KR 10-2020-0078512 A (PREVAIL THERAPEUTICS, INC.) 01 July 2020 (2020-07-01)<br>See abstract; and claims 1-43. | 1-33 |

Form PCT/ISA/210 (second sheet) (July 2022)

**INTERNATIONAL SEARCH REPORT**

International application No.

**PCT/KR2024/001916**

| **Box No. I** | **Nucleotide and/or amino acid sequence(s) (Continuation of item 1.c of the first sheet)** |
|---|---|

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

**INTERNATIONAL SEARCH REPORT**

| International application No. |
|---|
| **PCT/KR2024/001916** |

**Box No. II     Observations where certain claims were found unsearchable (Continuation of item 2 of first sheet)**

This international search report has not been established in respect of certain claims under Article 17(2)(a) for the following reasons:

1. ☑ Claims Nos.: **34-37**
   because they relate to subject matter not required to be searched by this Authority, namely:

   Claims 34-37 pertain to a method for treatment of the human body by surgery or therapy, as well as a diagnostic method (PCT Article 17(2)(a)(i) and PCT Rule 39.1(iv)).

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2022)

| INTERNATIONAL SEARCH REPORT | International application No. |
|---|---|
| Information on patent family members | **PCT/KR2024/001916** |

| Patent document cited in search report | | | Publication date (day/month/year) | Patent family member(s) | | | Publication date (day/month/year) |
|---|---|---|---|---|---|---|---|
| KR | 10-2022-0099944 | A | 14 July 2022 | AR | 119609 | A1 | 29 December 2021 |
| | | | | AU | 2020-328827 | A1 | 03 March 2022 |
| | | | | AU | 2020-328827 | A1 | 18 February 2021 |
| | | | | BR | 11-2022-002615 | A2 | 03 May 2022 |
| | | | | CA | 3149844 | A1 | 18 February 2021 |
| | | | | CL | 2022-000292 | A1 | 21 October 2022 |
| | | | | CN | 114786694 | A | 22 July 2022 |
| | | | | CO | 2022-001192 | A2 | 19 July 2022 |
| | | | | EP | 4013437 | A1 | 22 June 2022 |
| | | | | IL | 290357 | D0 | 01 April 2022 |
| | | | | JP | 2023-500011 | A | 04 January 2023 |
| | | | | TW | 2021-20687 | A | 01 June 2021 |
| | | | | US | 2022-0298528 | A1 | 22 September 2022 |
| | | | | WO | 2021-028299 | A1 | 18 February 2021 |
| KR | 10-2020-0078512 | A | 01 July 2020 | AU | 2018-346105 | A2 | 11 April 2019 |
| | | | | BR | 11-2020-006633 | A2 | 06 October 2020 |
| | | | | BR | 11-2020-006661 | A2 | 13 October 2020 |
| | | | | CA | 3074511 | A1 | 11 April 2019 |
| | | | | CN | 111373024 | A | 03 July 2020 |
| | | | | EP | 3692158 | A1 | 12 August 2020 |
| | | | | JP | 2020-532623 | A | 12 November 2020 |
| | | | | JP | 2020-537544 | A | 24 December 2020 |
| | | | | JP | 2022-068158 | A | 09 May 2022 |
| | | | | JP | 2023-086740 | A | 22 June 2023 |
| | | | | JP | 7254815 | B2 | 10 April 2023 |
| | | | | JP | 7336730 | B2 | 01 September 2023 |
| | | | | JP | 7361037 | B2 | 13 October 2023 |
| | | | | JP | 7389828 | B2 | 30 November 2023 |
| | | | | KR | 10-2020-0074132 | A | 24 June 2020 |
| | | | | KR | 10-2022-0015499 | A | 08 February 2022 |
| | | | | KR | 10-2022-0015500 | A | 08 February 2022 |
| | | | | US | 11802294 | B2 | 31 October 2023 |
| | | | | US | 2023-287358 | A1 | 14 September 2023 |
| | | | | WO | 2019-070894 | A1 | 11 April 2019 |
| | | | | WO | 2019-070923 | A1 | 11 April 2019 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 8980821 B2

- US 13088233 B

### Non-patent literature cited in the description

- **GREENAMYRE, J. T** ; **HASTINGS, T. G**. Parkinson's-divergent causes, convergent mechanisms. *Science*, 2004, vol. 304 (5674), 1120-1122
- **ZIEMSSEN, T** ; **REICHMANN, H**. Non-motor dysfunction in Parkinson's disease. *Parkinsonism & Related Disorders*, 2007, vol. 13 (6), 323-332
- **BOSE, A** ; **BEAL, M. F**. Mitochondrial dysfunction in Parkinson's disease. *Journal of Neurochemistry*, 2016, vol. 139, 216-231
- **GREEN, MICHAEL R** ; **JOSEPH SAMBROOK**. Molecular Cloning: A Laboratory Manual. 2012
- **JAIN, A** ; **JAIN, A** ; **GULBAKE, A** ; **SHILPI, S** ; **HURKAT, P** ; **JAIN, S. K**. Peptide and protein delivery using new drug delivery systems. *Critical Reviews in Therapeutic Drug Carrier Systems*, 2013, vol. 30 (4)
- **BULCHA, J. T** ; **WANG, Y** ; **MA, H** ; **TAI, P. W** ; **GAO, G**. Viral vector platforms within the gene therapy landscape. *Signal Transduction and Targeted Therapy*, 2021, vol. 6 (1), 53
- **LUNDSTROM, K**. Viral vectors in gene therapy. *Diseases*, 2018, vol. 6 (2), 42
- **ASOKAN, A** ; **SCHAFFER, D. V** ; **SAMULSKI, R. J**. The AAV vector toolkit: poised at the clinical crossroads. *Molecular Therapy*, 2012, vol. 20 (4), 699-708
- **LI, C** ; **SAMULSKI, R. J**. Engineering adeno-associated virus vectors for gene therapy. *Nature Reviews Genetics*, 2020, vol. 21 (4), 255-272
- **NAIR, A. B** ; **JACOB, S**. A simple practice guide for dose conversion between animals and human. *Journal of Basic and Clinical Pharmacy*, 2016, vol. 7 (2), 27